(19) Europäisches Patentamt
European Patent Office
Office européen des brevets

(11) EP 1 915 124 B1

# (12) EUROPÄISCHE PATENTSCHRIFT

(45) Veröffentlichungstag und Bekanntmachung des Hinweises auf die Patenterteilung:
**25.04.2012 Patentblatt 2012/17**

(21) Anmeldenummer: **06764299.1**

(22) Anmeldetag: **02.08.2006**

(51) Int Cl.:
***A61K 8/81*** *(2006.01)* ***C08F 220/04*** *(2006.01)*
***C08F 220/10*** *(2006.01)*

(86) Internationale Anmeldenummer:
**PCT/EP2006/064960**

(87) Internationale Veröffentlichungsnummer:
**WO 2007/017440 (15.02.2007 Gazette 2007/07)**

# (54) FESTIGERPOLYMERE AUF BASIS VON POLYESTERACRYLATEN

POLYESTER ACRYLATE-BASED FIXATIVE POLYMERS

POLYMERES FIXATEURS A BASE D'ACRYLATES DE POLYESTER

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **11.08.2005 EP 05107394**

(43) Veröffentlichungstag der Anmeldung:
**30.04.2008 Patentblatt 2008/18**

(73) Patentinhaber: **BASF SE**
**67056 Ludwigshafen (DE)**

(72) Erfinder:
- **WINTER, Gabi**
  **67061 Ludwigshafen (DE)**
- **PIEROBON, Marianna**
  **67063 Ludwigshafen (DE)**
- **LAUBENDER, Matthias**
  **67105 Schifferstadt (DE)**
- **NGUYEN KIM, Son**
  **69502 Hemsbach (DE)**

(74) Vertreter: **Reinhardt, Jürgen et al**
**BASF Personal Care and Nutrition GmbH**
**Postfach 13 01 64**
**40551 Düsseldorf (DE)**

(56) Entgegenhaltungen:
**WO-A-01/91705 WO-A-03/061615**
**WO-A-03/062288 DE-A1- 3 727 044**
**US-A- 3 940 351**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 915 124 B1

Printed by Jouve, 75001 PARIS (FR)

**Beschreibung**

Gegenstand der vorliegenden Erfindung sind kosmetische wässrige Zubereitungen wenigstens einen kosmetisch akzeptablen Träger B), der ausgewählt ist unter

i) wassermischbaren organischen Lösungsmitteln, vorzugsweise $C_2$-$C_4$-Alkanolen, insbesondere Ethanol,
ii) Ölen, Fetten, Wachsen,
iii) von ii) verschiedenen Estern von $C_6$-$C_{30}$-Monocarbonsäuren mit ein-, zwei- oder dreiwertigen Alkoholen,
iv) gesättigten acyclischen und cyclischen Kohlenwasserstoffen,
v) Fettsäuren,
vi) Fettalkoholen,
vii) Treibmitteln (Treibgasen) und
viii) Mischungen davon

und wenigstens ein Polymer A enthalten, welches

a) 40-89,5 Gew.-% wenigstens eines Esters der (Meth)acrylsäure,
b) 10-49 Gew.-% wenigstens einer olefinisch ungesättigten anionogenen oder anionischen Verbindung,
c) 0,5-10 Gew.-% wenigstens einer Verbindung ausgewählt aus
c1) Polyestern, enthaltend wenigstens zwei radikalisch polymerisierbare, olefinisch ungesättigte Doppelbindungen und
c2) Polyethern, enthaltend wenigstens zwei radikalisch polymerisierbare, olefinisch ungesättigte Doppelbindungen
d) sowie gegebenenfalls 0-30 Gew.-% weiterer olefinisch ungesättigter Verbindungen einpolymerisiert enthält mit der Massgabe, daß sich die Mengen der Komponente a) bis d) zu 100Gew.% addieren, wobei
die Polyether c2) Polyetheracrylate sind, erhältlich durch Reaktion von

A) 1 Äquivalent eines 2-bis 6-wertigen oxalkylierten $C_2$-bis $C_{10}$-Alkohols mit
B) 0,05 bis 1 Äquivalent einer 2-bis 4-wertigen $C_2$-bis $C_{10}$-Carbonsäure oder deren Anhydride und
C) 0,1 bis 1,5 Äquivalenten Acrylsäure und/oder Methacrylsäure sowie Umsetzung der überschüssigen Carboxylgruppen mit der äquivalenten Menge einer Epoxidverbindung.

Stand der Technik

Strengere Umweltauflagen und wachsendes ökologisches Bewusstsein fordern zunehmend immer geringere Anteile an flüchtigen organischen Komponenten (englisch: volatile organic compounds, VOC) in kosmetischen Aerosol-Zubereitungen wie beispielsweise Aerosol-Haarsprays.
Der VOC-Gehalt in Haarsprays wird im wesentlichen durch die nicht-wässrigen Lösungsmittel und die Treibmittel bestimmt. Daher wird gegenwärtig verstärkt anstelle von nicht-wässrigen Lösungsmittel auf Wasser als Lösungsmittel zurückgegriffen. Dieser Ersatz der organischen Lösungsmitteln bringt allerdings einige Probleme mit sich.
So sind Formulierungen der aus dem Stand der Technik bekannten filmbildenden Polymere, die entsprechende VOC-Auflagen erfüllen, beispielsweise nicht oder erst nach weiterer Verdünnung sprühbar und somit nur bedingt für die Verwendung in Haarsprays geeignet. Polymerfilme, die aus solchen Zubereitungen entstehen, weisen mitunter nicht die notwendige mechanische Qualität und somit ungenügende Festigungswirkung und schlechten Halt für das Haar auf.

Aufgabe und Lösung

Eine Aufgabe der vorliegenden Erfindung bestand darin, Polymere für kosmetische, insbesondere haarkosmetische Zubereitungen bereitzustellen, die als Pump- oder Aerosolspray in Lösungsmitteln oder Lösungsmittelgemischen mit erhöhtem Wasseranteil gut formulierbar sind, deren Formulierungen in Form von kleinen gleichmäßigen Tröpfchen gut sprühbar sind und während und nach dem Aufbringen möglichst wenig zur Schaumbildung neigen und deren dann gebildete Filme nicht klebrig sind und gute mechanische Eigenschaften aufweisen.
Neben der guten Verträglichkeit mit den üblichen kosmetischen Inhaltsstoffen sollen die auf das Haar aufgetragenen Polymere schnell trocknen und dem Haar gute Festigung und längeren Halt auch bei erhöhter Luftfeuchtigkeit verleihen, gute Auswaschbarkeit aufweisen und sich als optisch klare VOC-55-Aerosole (d.h. mit einem VOC-Anteil von höchstens 55 Gew.-%) formulieren lassen. Weiterhin soll das behandelte Haar gute haptische Eigenschaften wie beispielsweise ein gutes Anfaßgefühl aufweisen.
Diese Aufgaben wurden überraschenderweise gelöst durch die eingangs beschriebenen kosmetischen wässrigen Zubereitungen, die wenigstens einen kosmetisch akzeptablen Träger B) enthalten.

WO 03/062288 und WO 03/061615 beschreiben wässrige Haarfestiger-Zusammensetzungen, die eine effektive Menge eines rheologiemodifizierenden haarfestigenden Assoziativpolymeren aus einem Säuremonomer und einem Assoziativmonomer enthalten. Als hydrophobe Assoziativmonomere sind langkettige Ester der (Meth)acrylsäure bevorzugt. Die Polymere können weitere Monomere und Vernetzer enthalten.

EP-A 0 184 785 beschreibt eine wäßrige Copolymerisat-Dispersion aus 50-60 Gew.-% Ethylacrylat, 30-40 Gew.-% Methacrylsäure, 5-15 Gew.-% Acrylsäure und 0,02-0,04 Gew.-% eines mehrfach ungesättigten copolymerisierbaren Monomeren mit einem Feststoffgehalt von 5 - 30 Gew.-%, die sich zur Verdickung wäßriger Systeme, insbesondere von Wasserstoffperoxid-Zubereitungen eignet, wie sie als Entwickler-Zubereitungen für Oxidationshaarfärbemittel und für Haarbleichmittel verwendet werden. Mehrfach ungesättigte Monomere werden nur in sehr kleinen Mengen eingesetzt.

WO 95/05402 beschreibt haarkosmetische Zubereitungen, die wässrige Copolymerisatdispersionen umfassen, welche erhältlich sind durch Copolymerisation von 40 bis 99 Gew.-% eines oder mehrerer wasserunlöslicher, monoethylenisch ungesättigter Monomeren und 1 bis 60 Gew%. eines oder mehrerer wasserlöslicher, monoethyle nisch ungesättigter Monomeren. Optional können 0 bis 30 Gew.-% eines oder mehrerer mehrfach ethylenisch ungesättigter Monomeren eingesetzt werden.

DE 2 330 957 beschreibt gepfropfte und vernetzte kationische Mischpolymerisate, die erhalten werden durch Mischpolymerisation von a) wenigstens einem kosmetischen Monomer, b) Dimethylaminoäthylmethacrylat, c) Polyethylenglykol und d) einem mehrfach ungesättigten Vernetzungsmittel.

US 3,940,351 beschreibt Polymere aus ethylenisch ungesättigten Carbonsäuren, langkettigen Estern solcher Carbonsäuren und Allylgruppen enthaltenden Polyethern, deren Herstellung in Gegenwart eines Halogenalkans erfolgt. Im Rahmen der vorliegenden Erfindung umfasst der Ausdruck Alkyl geradkettige und verzweigte Alkylgruppen. Geeignete kurzkettige Alkylgruppen sind z.B. geradkettige oder verzweigte $C_1$-$C_{12}$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl etc..

Geeignete längerkettige $C_6$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl (en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc..

Cycloalkyl steht vorzugsweise für $C_5$-$C_8$-Cycloalkyl, wie Cyclopentyl, Cyclohexyl, Cycloheptyl oder Cyclooctyl.

Der Ausdruck Heterocycloalkyl im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR, $COO^-M^+$ und $NE^1E^2$, bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, $-SO_3H$, Sulfonat, $NE^1E^2$, Alkylen-$NE^1E^2$, Nitro, Cyano oder Halogen auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Arylalkyl steht für Gruppen, die sowohl Alkyl- als auch Arylreste enthalten, wobei diese Arylalkyl-Gruppen entweder über den Aryl- oder über den Alkylrest mit der sie tragenden Verbindung verknüpft sind.

Komponente a)

Komponente a) ist ausgewählt aus den Estern der (Meth)acrylsäure.

Komponente a) ist beispielsweise ausgewählt aus der Gruppe bestehend aus Methyl(meth)acrylat, Ethyl(meth) acrylat, n-Propyl(meth)acrylat, i-Propyl(meth)acrylat, n-Butyl(meth)acrylat, tert.-Butyl(meth)acrylat, i-Butyl(meth)acrylat,

sec- Butyl(meth)acrylat, 2-Pentyl(meth)acrylat, 3-Pentyl(meth)acrylat, Isopentyl(meth)acrylat, Neopentyl(meth)acrylat, n-Octyl(meth)acrylat, 1,1, 3,3-Tetramethylbutyl(meth)acrylat, Ethylhexyl(meth)acrylat, n-Nonyl(meth)acrylat, n-Decyl (meth)acrylat, n-Undecyl(meth)acrylat, Tridecyl(meth)acrylat, Myristyl(meth)acrylat, Pentadecyl(meth)acrylat, Palmityl (meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, Phenoxyethyl(meth)acrylat, 4-t-Butylcyclohexylacrylat, Cyclohexyl(meth)acrylat, Ureido(meth)acrylat, Tetrahydrofurfuryl(meth)acrylat und deren Mischungen.

Komponente a) kann auch ausgewählt sein aus Estern der (Meth)acrylsäure mit Alkandiolen. Beispielsweise sind dies 2-Hydroxyethyl(meth)acrylat, 2-Hydroxyethylethacrylat, 2-Hydroxypropyl(meth)acrylat, 3-Hydroxypropyl(meth) acrylat, 3-Hydroxybutyl(meth)acrylat, 4-Hydroxybutyl(meth)acrylat, 6-Hydroxyhexyl(meth)acrylat, 3-Hydroxy-2-ethylhexyl(meth)acrylat, Neopentylglykolmono(meth)acrylat, 1,5-Pentandiolmono(meth)acrylat und 1,6-Hexandiolmono(meth) acrylat.

Bevorzugte (Meth)acrylate sind $C_1$-$C_{10}$-, besonders bevorzugt $C_1$-$C_8$- und insbesondere $C_1$-$C_4$-Alkyl(meth) acrylate. Komponente a) kann auch eine Mischung von Methacrylaten und Acrylaten sein.

Komponente a) ist ganz besonders bevorzugt ausgewählt aus der Gruppe bestehend aus tert.-Butylacrylat, Methylmethacrylat, Ethylmethacrylat, n-Propylmethacrylat, i-Propylmethacrylat, n-Butylmethacrylat, tert.-Butylmethacrylat, i-Butylmethacrylat, sec-Butylmethacrylat und deren Mischungen.

Noch weiter bevorzugt ist Komponente a) ausgewählt aus der Gruppe bestehend aus Methylmethacrylat, Ethylmethacrylat und deren Mischungen.

Am meisten bevorzugt als Komponente a) ist Methylmethacrylat (MMA).

Polymer A enthält 40-89,5, besonders bevorzugt 60-80 und insbesondere 70-80 Gew.-% der Komponente a) einpolymerisiert.

Komponente b)

Komponente b) ist eine olefinisch ungesättigte, radikalisch polymerisierbare anionogene oder anionische Verbindung. Unter einer anionogenen Verbindung wird im Rahmen der vorliegenden Erfindung eine Verbindung verstanden, die durch Deprotonierung mit üblichen, bevorzugt kosmetisch akzeptablen organischen oder anorganischen Basen in die entsprechende anionische Form überführt werden kann.

Komponente b) kann ausgewählt sein aus olefinisch ungesättigten, radikalisch polymerisierbaren Carbon-, Sulfon- oder Phosphonsäuren und deren organischen und anorganischen Salzen.

Beispiele für bevorzugte Sulfonsäuren sind 2-Acrylamido-2-methylpropansulfonsäure (AMPS), Styrolsulfonsäure, Vinylsulfonsäure und deren Salze.

Beispiele für bevorzugte Phosphonsäuren sind Vinylphosphonsäure, 2-Acrylamido-2-methylpropanphosphonsäure, Allylphosphonsäure und deren Salze.

Komponente b) ist bevorzugt ausgewählt aus der Gruppe der olefinisch ungesättigten, radikalisch polymerisierbaren Carbonsäuren und deren organischen und anorganischen Salzen. Bei den Carbonsäuren kann es sich um Monocarbonsäuren, Dicarbonsäuren, Carbonsäureanhydride oder Halbester von Dicarbonsäuren handeln.

Komponente b) ist besonders bevorzugt ausgewählt aus der Gruppe bestehend aus Acrylsäure, Methacrylsäure, Ethacrylsäure, alpha-Chloracrylsäure, Crotonsäure, Maleinsäure, Maleinsäureanhydrid, Itaconsäure, Citraconsäure, Mesaconsäure, Glutaconsäure, Aconitsäure, Fumarsäure, Halbestern von olefinisch ungesättigten Dicarbonsäuren mit 4 bis 10, vorzugsweise 4 bis 6 C-Atomen und deren Salzen.

Komponente b) umfasst oder besteht ganz besonders bevorzugt aus Verbindungen ausgewählt aus der Gruppe bestehend aus Acrylsäure, Methacrylsäure, deren Salzen und deren Mischungen.

Komponente b) umfasst oder besteht ganz besonders bevorzugt aus Verbindungen ausgewählt aus der Gruppe bestehend aus Acrylsäure, Itaconsäure, deren Salzen und deren Mischungen.

Polymer A enthält 10-49 besonders bevorzugt 12-39 und insbesondere 15-29 Gew.-% der Komponente b) einpolymerisiert.

Komponente c)

Komponente c) ist ausgewählt aus

c1) Polyestern, enthaltend wenigstens zwei radikalisch polymerisierbare, olefinisch ungesättigte Doppelbindungen und

c2) Polyethern, enthaltend wenigstens zwei radikalisch polymerisierbare, olefinisch ungesättigte Doppelbindungen.

Polyester und Polyether sind dem Fachmann prinzipiell bekannt. Bevorzugte Polyester c1) und Polyether c2) sind Polyester- und Polyether(meth)acrylate. Als Polyester- und Polyether(meth)acrylate werden im Rahmen dieser Erfindung Verbindungen bezeichnet, die zusätzlich zu den enthaltenen (Meth)acrylat-Estergruppen wenigstens 2 weitere, bevorzugt mehr als 2 weitere Ester- und/oder Ethergruppen enthalten.

Selbstverständlich können die Polyester auch Ether-Struktureinheiten und die Polyether auch Ester-Struktureinheiten enthalten. Zahlreiche geeignete Komponenten c) enthalten gleichzeitig sowohl Ester- als auch Ethergruppen. Komponente c) umfasst selbstverständlich auch beliebige Mischungen von c1) und c2).

Bevorzugte Komponenten c) enthalten als eine der wenigstens zwei radikalisch polymerisierbaren, olefinisch ungesättigten Doppelbindungen eine (Meth)acrylatgruppe der allgemeinen Formel $H_2C=CR-COO-$ wobei R H oder Methyl bedeutet. Weiter bevorzugte Komponenten c) enthalten wenigstens zwei (Meth)acrylatgruppen.

Komponente c1)

Der Begriff Polyester ist dem Fachmann bekannt. Polyester sind Polymere mit Esterbindungen - [-CO-O-]- in der Hauptkette. Komponenten c1) gemäß dieser Erfindung sind beispielsweise Polyester(meth)acrylate, die wenigstens zwei radikalisch polymerisierbare, olefinisch ungesättigte Doppelbindungen pro Molekül enthalten.

Polyester(meth)acrylate sind dem Fachmann prinzipiell bekannt. Sie sind durch verschiedene Methoden herstellbar. Beispielsweise kann (Meth)Acrylsäure direkt als Säurekomponente beim Aufbau der Polyester eingesetzt werden. Daneben besteht die Möglichkeit, Hydroxyalkylester der (Meth)Acrylsäure als Alkoholkomponente direkt beim Aufbau der Polyester einzusetzen. Bevorzugt werden die Polyester(meth)acrylate aber durch (Meth)Acrylierung von Polyestern hergestellt. Beispielsweise können zu nächst hydroxylgruppenhaltige Polyester aufgebaut werden, die dann mit Acryl- oder Methacrylsäure umgesetzt werden. Bevorzugt werden wenigstens 2 der Hydroxylgruppen pro MoleKül der hydroxylgruppenhaltige Polyester mit (Meth)Acrylsäure umgesetzt, so dass pro Molekül des Reaktionsproduktes wenigstens zwei radikalisch polymerisierbare, olefinisch ungesättigte Doppelbindungen enthalten sind.

Es können auch zunächst carboxylgruppenhaltige Polyester aufgebaut werden, die dann mit einem Hydroxyalkylester der Acryl- oder Methacrylsäure umgesetzt werden. Auch hier werden wenigstens zwei der Carboxylgruppen pro Molekül der carboxylgruppenhaltige Polyester mit dem Hydroxyalkylester der (Meth)Acrylsäure umgesetzt, so dass pro Molekül des Reaktionsproduktes wenigstens zwei radikalisch polymerisierbare, olefinisch ungesättigte Doppelbindungen enthalten sind.

Bevorzugt ist, es Gemische von Polyester(meth)acrylaten einzusetzen, die durchschnittlich mehr als 2 radikalisch polymerisierbare, olefinisch ungesättlgte Doppelbindungen pro Molekül Polyester(meth)acrylat enthalten.

Als Komponente c1) geeignete Polyesteracrylate sind beilspielsweise beschrieben in der EP-A 0 279 303, (EP-A 0 279 303, S.5, Z.28-44). Auch die DE 2 853 921 beschreibt geeignete Polyesteracrylate, nämlich solche aus aliphatischen und/oder aromatischen Dicarbonsäuren, wie Bernsteinsäure, Glutarsäure, Adipinsäure, Pimelinsäure, Korksäure, Azelainsäure, Sebacinsäure, Cyclohexandicarbonsäure, Phthalsäure, Isophthalsäure, Terephthalsäure, Maleinsäure, Fumarsäure, Itakonsäure bzw. deren Derivaten und mehrwertigen Alkoholen, wie Ethylenglykol, Polyethylenglykolen, Propylenglykol, Polypropylenglykolen, Butandiol, Hexanoiol, Neopentylglykol, Hydroxypivalinsäureneopentylglykolester, Trimethylolpropan, Glycerin, Pentaerythrit und/ oder Trishydroxyethylisocyanurat sowie $\alpha,\beta$-ethylenisch ungesättigten Monocarbonsäuren, beispielsweise Acrylsäure, Methacrylsäure, Crotonsäure, Zimtsäure und/oder Dicarbonsäurehalbester von Monoalkanolen, wie Malein-, Fumar und Itakonsäurehalbester mit $C_1$-$C_4$-Monoalkoholen, wobei Acrylsäure und Methacrylsäure bevorzugt sind.

Auch die EP-A 0 686 621 beschreibt geeignete Komponenten c1). Dabei handelt es sich um Umsetzungsprodukte der (Meth)acrylsäure mit einer Hydroxyverbindung. Als Hydroxyverbindungen in Betracht kommen Verbindungen mit einer oder mehreren Hydroxygruppen.

Genannt sind Monoalkohole, $C_2$-$C_6$-Alkylendlole, Trimethylolpropan, Glycerin oder Pentaerythrit oder z.B. mit Ethylenoxid oder Propylenoxid alkoxylierte, Hydroxygruppen enthaltende Verbindungen.

Als Hydroxyverbindungen kommen weiterhin hydroxylgruppenhaltige Polyester in Betracht. Derartige hydroxylgruppenhaltige Polyester können z.B. in üblicher Weise durch Veresterung von Dicarbonsäuren oder Polycarbonsäuren mit Diolen oder Polyolen hergestellt werden. Die Ausgangsstoffe für solche hydroxylgruppenhaltige Polyester sind dem Fachmann bekannt. Bevorzugt können als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride, z.B. Maleinsäureanhydrid, oder Dialkylester der genannten Säuren eingesetzt werden. Als Polycarbonsäure kommt z.B. Trimellitsäure in Betracht. Zu den einzusetzenden Polyesterolen zählen auch Polycaprolactondiole und -triole, deren Herstellung dem Fachmann ebenfalls bekannt ist.

Bevorzugte Hydroxyverbindungen sind mindestens 2, insbesondere 2 bis 6 freie Hydroxylgruppen enthaltende, gesättigte Polyester, welche gegebenenfalls auch Ethergruppen enthalten können oder Polyether (als Komponente c2)) mit mindestens 2, insbesondere 2 bis 6 freien Hydroxylgruppen.

Die Komponenten c1), wie beispielsweise Polyester(meth)acrylate besitzen wenigstens 2 radikalisch polymerisierbare Doppelbindungen pro Molekül. Weiterhin bevorzugt ist es, Gemische von Komponenten c), beispielsweise von Polyester(meth)acrylaten einzusetzen, die durchschnittlich mehr als 2 radikalisch polymerisierbare, olefinisch ungesättigte Doppelbindungen pro Molekül Polyester(meth)acrylat enthalten. Solche Gemische ergeben sich beispielsweise durch Mischen von Verbindungen mit jeweils 2 und Verbindungen mit jeweils 3 oder mehr polymerisierbaren Doppelbindungen pro Molekül. Natürlich können in den Gemischen auch Verbindungen enthalten sein, die nur eine oder keine Doppelbindung pro Molekül enthalten. Solche Verbindungen sind dann aber in derartigen Mengen enthalten, dass die durchschnittliche Zahl von polymerisierbaren Doppelbindungen pro Molekül trotzdem mehr als 2 beträgt.

Komponente c2)

Der Begriff der Polyether ist dem Fachmann bekannt. Polyether sind Polymere, deren organ. Wiederholungseinheiten durch Ether-Funktionalitäten (C-O-C) zusammengehalten werden.

Komponenten c2) gemäß dieser Erfindung sind beispielsweise Polyether(meth)acrylate, die wenigstens 2 radikalisch polymerisierbare Doppelbindungen pro Molekül enthalten. Diese sind dem Fachmann ebenfalls prinzipiell bekannt. Sie sind durch verschiedene Methoden herstellbar. Beispielsweise können hydroxylgruppenhaltige Polyether, die mit Acrylsäure und/oder Methacrylsäure zu den Poly ether(meth)acrylaten verestert werden, durch Umsetzung von zwei- und/oder mehrwertigen Alkoholen mit verschiedenen Mengen an Ethylenoxid und/oder Propylenoxid nach gut bekannten Methoden (vgl. z.B. Houben-Weyl, Band XIV, 2, Makromolekulare Stoffe II, (1963)) erhalten werden. Einsetzbar sind auch Polymerisationsprodukte des Tetrahydrofurans oder Butylenoxids.

Auch die DE 2 853 921 beschreibt geeignete Komponenten c2) wie beispielsweise aliphatische oder aromatisch-aliphatische Polyether, welche durch Umsetzung von zwei- und/oder mehrwertigen Alkoholen, mit verschiedenen Mengen an Ethylen-und/oder Propylenoxid erhalten werden und deren freie Hydroxylgruppen ganz oder teilweise mit ethylenisch ungesättigten Alkoholen, beispielsweise Allylalkohol, Methallylalkohol, Crotylalkohol, Zimtalkohol, verethert und/oder mit $\alpha,\beta$-ethylenisch ungesättigten Monocarbonsäuren verestert sind.

Als Komponente c2) geeignete Polyetheracrylate sind beispielsweise beschrieben in der EP-A 0 279 303.

Diese Polyetheracrylate sind erhältlich durch Reaktion von A) 1 Äquivalent eines 2-bis 6-wertigen oxalkylierten $C_2$-bis $C_{10}$-Alkohols mit B) 0,05 bis 1 Äquivalent einer 2-bis 4-wertigen $C_2$-bis $C_{10}$-Carbonsäure oder deren Anhydride und C) 0,1 bis 1,5 Äquivalenten Acrylsäure und/oder Methacrylsäure sowie Umsetzung der überschüssigen Carboxylgruppen mit der äquivalenten Menge einer Epoxidverbindung.

Auch die EP-A 0 686 621 beschreibt geeignete Komponenten c2). Dabei handelt es sich um Umsetzungsprodukte der (Meth)acrylsäure mit einer Hydroxyverbindung. Als Hydroxyverbindungen in Betracht kommen Verbindungen mit einer oder mehreren Hydroxygruppen. Genannt seien z.B. mit Ethylenoxid oder Propylenoxid alkoxylierte, Hydroxygruppen enthaltende Verbindungen.

Bevorzugte Hydroxyverbindungen sind gesättigte Polyether mit mindestens 2, insbesondere 2 bis 6 freien Hydroxylgruppen. Als hydroxylgruppenhaltige Polyether kommen z.B. solche in Frage, welche nach bekannten Verfahren durch Umsetzung von zwei- und/oder mehrwertigen Alkoholen mitverschiedenen Mengen an Ethylenoxid und/oder Propylenoxid erhalten werden können. Bei den Ethylenglykol/Propylenglykol-Mischkondensationsprodukten kann die Umsetzung zweckmäßigerweise so gesteuert werden, daß endständig überwiegend primäre Hydroxylgruppen entstehen. Desgleichen sind auch Polymerisationsprodukte des Tetrahydrofurans oder Butylenoxids, die Hydroxylgruppen enthalten, verwendbar.

Beispiele für Komponente c) sind Polyalkylenglykol(meth)acrylate.

In einer bevorzugten Ausführungsform der Erfindung werden als Komponente c) solche Verbindungen verwendet, deren Molekulargewicht $M_w$ mindestens 200 g/mol, besonders bevorzugt mindestens 400 g/mol, ganz besonders bevorzugt mindestens 500 g/mol und am meisten bevorzugt mehr als 700 g/mol beträgt.

In einer weiteren bevorzugten Ausführungsform der Erfindung werden als Komponente c) Verbindungen c1) und/oder c2) oder Gemische von Verbindungen c1) und/oder c2) verwendet, wobei die durchschnittliche Anzahl von olefinischen, radikalisch polymerisierbaren Doppelbindungen pro Molekül mehr als 2 beträgt. Solche Gemische ergeben sich beispielsweise durch Mischen von Verbindungen mit jeweils 2 und Verbindungen mit jeweils 3 oder mehr polymerisierbaren Doppelbindungen pro Molekül. Natürlich können in den Gemischen auch Verbindungen enthalten sein, die nur eine oder keine Doppelbindung pro Molekül enthalten. Solche Verbindungen sind dann aber in derartigen Mengen enthalten, dass die durchschnittliche Zahl von polymerisierbaren Doppelbindungen pro Molekül trotzdem mehr als 2 beträgt.

Es soll an dieser Stelle betont werden, dass es als Komponente c) geeignete Verbindungen gibt, die beiden Gruppen c1) und c2) zugeordnet werden können, da sie sowohl Ester- als auch Ethergruppen enthalten. Komponente c) sind erfindungsgemäß somit auch wenigstens zwei radikalisch polymerisierbare, olefinisch ungesättigte Doppelbindungen enthaltende Verbindungen, die gleichzeitig sowohl Ether- als auch Esterstrukturen enthalten.

Kommerziell erhältliche Produkte, die als Komponente c) geeignet sind, sind beispielsweise:

Photomer®5010, Photomer®5429, Photomer®5430, Photomer®5432, Photomer®5662, Photomer®5806, Photomer®5930 der Firma Cognis;

die Resin®-Marken der Firma UCB wie z.B. Resin®80, 81, 83, 450, 657, 770, 809, 810, 830, 835, 870, 1657, 1810, 1870, 2047**, 2870;

die CN®-Marken der Firma Sartomer wie z.B. CN293, CN294, CN296, CN292, CN2297A, CN2279, CN2280, CN2470, CN295, CN2300, CN2200, CN2203, CN2282, CN2284, CN2270, CN2271, CN2272, CN2273, CN2276, CN2250, CN2251, CN2252, CN2253, CN2255, CN2256, CN2257, CN2258, CN2259, CN2260, CN2261;

AROPLAZ®4097-WG4-55 der Firma Reichhold;

Syntholux®-PE-Marken der Firma Synthopol als Polyesteracrylate und die Syntholux®-PA-Marken der Firma Synthopol als Polyetheracrylate;

Laromer®-Marken Laromer®PE 55F, Laromer®PE 56F, Laromer®PE 46T, Laromer®9004, Laromer®PE 44F, Laromer®8800, Laromer®LR 8981, Laromer®LR 8992, Laromer®PE 22WN, Laromer®PE 55WN, Laromer®PO 33F, Laromer®LR 8863, Laromer®PO 43F, Laromer®LR 8967, LaromervLR 8982, Laromer®LR 9007 (BASF). Polymer A enthält 0,5-10, bevorzugt 1-8, besonders bevorzugt 1-5 Gew.-% der Komponente c) einpolymerisiert.

Komponente d)

Als Komponente d) kommen alle von den Komponenten a) bis c) verschiedenen, radikalisch polymerisierbaren, ungesättigten Verbindungen in Betracht, die mit den Komponenten a) bis c) copolymerisiert werden können.

Bevorzugte Komponenten d) sind

d1) von d2) verschiedene amidgruppenhaltige Verbindungen,
d2) (Meth)acrylamide,
d3) kationogene Monomere,
d4) kationische Monomere,
d5) Verbindungen mit wenigstens 2 polymerisierbaren Doppelbindungen, die üblicherweise auch als Vernetzer bezeichnet werden und
d6) Mischungen daraus.

Komponente d1)

Die amidgruppenhaltigen Verbindungen d1) sind bevorzugt ausgewählt aus von d2) verschiedenen Verbindungen der allgemeinen Formel VI

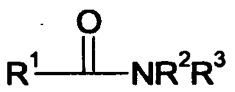

(VI)

wobei $R^1$ für eine Gruppe der Formel $CH_2=CR^4$- mit $R^4$ = H oder $C_1$-$C_4$-Alkyl steht und $R^2$ und $R^3$ unabhängig voneinander für H, Alkyl, Cycloalkyl, Heterocycloalkyl, Aryl oder Hetaryl stehen oder $R^2$ und $R^3$ gemeinsam mit dem Stickstoffatom, an das sie gebunden sind, für einen fünf- bis achtgliedrigen Stickstoff-Heterocyclus stehen oder $R^2$ für eine Gruppe der Formel $CH_2=CR^4$- steht und $R^1$ und $R^3$ unabhängig voneinander für H, Alkyl, Cycloalkyl, Heterocycloalkyl, Aryl oder Hetaryl stehen oder $R^1$ und $R^3$ gemeinsam mit der Amidgruppe, an die sie gebunden sind für ein Lactam mit 5 bis 8 Ringatomen stehen.

Bevorzugt als Komponente d1) sind N-Vinyllactame. Als Komponente d1) eignen sich unsubstituierte N-Vinyllactame und N-Vinyllactamderivate, die z.B. einen oder mehrere $C_1$-$C_6$-Alkylsubstituenten, wie Methyl, Ethyl, n-Propyl, Isopropyl, n-Butyl, sec.-Butyl, tert.-Butyl etc., aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinvl-7-ethyl-2-caprolactam etc. sowie deren Mischungen. Bevorzugte Komponenten d1) sind diejenigen, für die in Formel VI $R^2$ für $CH_2=CH$- und $R^1$ und $R^3$ gemeinsam mit der Amidgruppe, an die sie gebunden sind für ein Lactam mit 5 Ringatomen stehen.

Besonders bevorzugt werden N-Vinylpyrrolidon, N-Vinylcaprolactam, N-Vinylformamid, Acrylamid oder deren

Mischungen eingesetzt, wobei N-Vinylpyrrolidon am meisten bevorzugt ist.

Komponente d2)

Geeignete Komponenten d2) sind die von d3) und d4) verschiedenen Amide der (Meth)crylsäure. Solche Amide sind beispielsweise (Meth)acrylamid, N-Methyl(meth)acrylamid, N-Ethyl(meth)acrylamid, N-n-Propyl-(meth)acrylamid, N-i-Propyl-(meth)acrylamid, N-(n-Butyl)methacrylamid, N-(sek.-Butyl)methacrylamid, N-(tert.-Butyl)methacrylamid, N-(n-Pentyl)(meth)acrylamid, N-(n-Hexyl)(meth)acrylamid, N-(n-Heptyl)(meth)acrylamid, N-(n-Octyl)(meth)acrylamid, N-(tert.-Octyl)(meth)acrylamid N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arrachinyl(meth)acrylamid, N-Behenyl(meth)acrylamid, N-Lignocerenyl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid.

Geeignete Komponenten d2) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Komponenten d3) und d4)

Die Komponenten d3) und d4) sind Monomere, die wenigstens eine kationogene und/oder kationische Gruppe pro Molekül enthalten.

Bevorzugt handelt es sich bei den kationogenen bzw. kationischen Gruppen um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen.

Bevozugt werden die Komponenten d3) und d4) in nicht geladener Form zur Polymerisation eingesetzt. Geeignet ist jedoch auch ein Einsatz in geladener Form.

Geladene kationische Gruppen lassen sich beispielsweise aus den Aminstickstoffatomen durch Protonierung, beispielsweise mit einwertigen oder mehrwertigen Carbonsäuren, wie Milchsäure oder Weinsäure, oder Mineralsäuren, wie Phosphorsäure, Schwefelsäure und Salzsäure erzeugen.

Vorzugsweise sind die Komponenten d3) und d4) ausgewählt ist unter

- Estern α,β-olefinisch ungesättigter Mono- und Dicarbonsäuren mit Aminoalkoholen, welche am Aminstickstoff mono- oder dialkyliert sein können,
- Amiden α,β-olefinisch ungesättigter Mono- und Dicarbonsäuren mit Diaminen, welche mindestens eine primäre oder sekundäre Aminogruppe aufweisen,
- N,N-Diallylamin,
- N,N-Diallyl-N-alkylaminen und deren Derivaten,
- vinyl- und allylsubstituierten Stickstoffheterocyden,
- vinyl- und allylsubstituierten heteroaromatischen Verbindungen und
- Mischungen davon.

Geeignet als Komponenten d3) und d4) sind auch die Ester von α,β-olefinisch ungesättigten Mono- und Dicarbonsäuren mit Aminoalkoholen. Bevorzugte Aminoalkohole sind $C_2$-$C_{12}$-Aminoalkohole, welche am Aminstickstoff $C_1$-$C_8$-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt.

Besonders bevorzugt als Komponenten d3) und d4) sind N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Insbesondere werden als Komponenten d3) und d4) N-(tert.-Butyl)aminoethylacrylat und N-(tert.-Butyl)aminoethylmethacrylat eingesetzt.

Geeignete Komponenten d3) und d4) sind weiterhin die Amide der zuvor genannten α,β-olefinisch ungesättigten Mono- und Dicarbonsäuren mit Diaminen, welche mindestens eine primäre oder sekundäre Aminogruppe aufweisen.

Bevorzugt sind Diamine, die eine tertiäre und eine primäre oder sekundäre Aminogruppe aufweisen. Bevorzugt werden als Komponenten d3) und d4) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl] acrylamid und N-[4-(dimethylamino)cyclohexyl]methacrylamid eingesetzt.

Besonders bevorzugt werden N-[3-(dimethyl-amino)propyl]acrylamid und/oder N-[3-(dimethylamino)propyl] methacrylamid eingesetzt.

Geeignete Komponenten d3) und d4) sind weiterhin N,N-Diallylamine und N,N-Diallyl-N-alkylamine und deren Säureadditionssalze. Alkyl steht dabei vorzugsweise für $C_1$-$C_{24}$-Alkyl. Bevorzugt ist N,N-Diallyl-N-methylamin.

Geeignete Komponenten d3) und d4) sind weiterhin vinyl- und allylsubstituierte Stickstoffheterocyclen, wie N-Vinylimidazol, N-Vinylimidazol-Derivate, z. B. N-Vinyl-2-methylimidazol, vinyl- und allylsubstituierte heteroaromatische Verbindungen, wie 2-und 4-Vinylpyridin, 2- und 4-Allylpyridin, und die Salze davon.

Geeignete Komponenten d3) und d4) sind auch N-Vinylimidazole der allgemeinen Formel VII, worin $R^1$ bis $R^3$ für Wasserstoff, $C_1$-$C_4$-Alkyl oder Phenyl steht

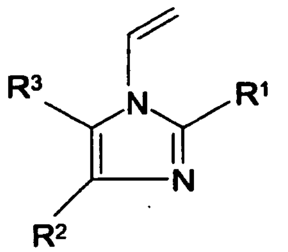

(VII)

Beispiele für Verbindungen der allgemeinen Formel VII sind folgender Tabelle 1 zu entnehmen:

Tabelle 1

| $R^1$ | $R^2$ | $R^3$ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |
| Me = Methyl; Ph = Phenyl | | |

Besonders bevorzugt sind die Komponenten d3) und d4) ausgewählt unter N-(tert.-Butylamino)ethyl(meth)acrylat, N,N-Dimethylaminoethyl(meth)acrylat, N-[3-(dimethylamino)propyl](meth)acrylamid, Vinylimidazol und deren Mischungen.

Enthalten die erfindungsgemäßen Polymere A Komponenten d3) und/oder d4) einpolymerisiert, so enthalten sie wenigstens 0.1 Gew.-%, bevorzugt wenigstens 1 Gew.-%, besonders bevorzugt wenigstens 2 Gew.-% und insbesondere wenigstens 3 Gew.-% und höchstens 30 Gew.-%, bevorzugt höchstens 20 Gew.-%, besonders bevorzugt höchstens 15 Gew.-% und insbesondere höchstens 10 Gew.-% der Komponenten d3) und/oder d4), bezogen auf das Gesamtgewicht eingesetzten Komponenten a) bis d).

Die geladenen kationischen Gruppen lassen sich aus den Aminstickstoffen durch Quatemisierung mit sogenannten Alkylierungsmitteln erzeugen. Beispiele für geeignete Alkylierungsmittel sind $C_1$-$C_4$-Alkylhalogenide oder -sulfate, wie Ethylchlorid, Ethylbromid, Methylchlorid, Methylbromid, Dimethylsulfat und Diethylsulfat. Eine Quatemisierung kann im Allgemeinen sowohl vor als auch nach der Polymerisation erfolgen.

Komponente d5)

Komponente d5) sind Verbindungen mit wenigstens 2 radikalisch polymerisierbaren nichtkonjugierten Doppelbindungen pro Molekül.

Geeignete Komponenten d5) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Komponenten d5) enthalten aber mindestens zwei radikalisch polymerisierbare ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000.

Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden.

Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Bevorzugte mehrwertige Alkohole in diesem Zusammenhang sind auch Di- und Trisaccaride.

Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Komponenten d5) sind die Vinylester oder die Ester einwertiger, ungesättigter Alkohole mit olefinisch ungesättigten $C_3$- bis $C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-of, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Komponenten d5) sind Ester ungesättigter Carbonsäuren mit den oben be-schriebenen mehrwertigen Alkoholen, beispielsweise der Ölsäure, Crotonsäure, Zimtsäure oder 10-Undecensäure.

Geeignet als Komponenten d5) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht kon-jugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Komponenten d5) sind ferner geeignet die Amide von (Meth)Acrylsäure, Itaconsäure und Maleinsäure sowie N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Komponente d5) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Geeignet sind auch Alkylenbisacrylamide wie Methylenbisacrylamid und N,N'-(2,2)butan und 1,1'-bis-(3,3'-vinylbenzimidazolith-2-on)-1,4-butan.

Andere geeignete Komponenten d5) sind beispielsweise Alkylenglykoldi(meth)acrylate wie E-thylenglykoldiacrylat, Ethylenglykoldimethacrylat, Tetraethlyenglykolacrylat, Tetraethy-lenglykoldimethacrylat, Diethylenglykolacrylat, Diethylenglykolmethacrylat, Vinylacrylat, Allylacrylat, Allylmethacrylat, Divinyldioxan, Pentaerythritallylether sowie Gemische dieser Komponenten d5).

Weitere geeignete Komponenten d5) sind Divinyldioxan, Tetraallylsilan oder Tetravinylsilan.

Besonders bevorzugt eingesetzte Komponenten d5) sind beispielsweise Methylenbisacrylamid, Triallylamin und Triallylalkylammoniumsalze, Divinylimidazol, Pentaerythrittriallylether, N,N'-Divinylethylenhamstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Komponente d5) sind Pentaerythrittriallylether, Methylenbisacrylamid, N,N'-Divinylethylenhamstoff, Triallylamin und Triallylmonoalkylammoniumsalze, und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Besonders bevorzugt als Komponente d5) sind Verbindungen mit einem Molekulargewicht $M_w$ < 400 g/mol.

Selbstverständlich können auch Mischungen der vorgenannten Verbindungen eingesetzt werden. Die Komponente d5) ist vorzugsweise im Reaktionsmedium löslich. Ist die Löslichkeit der Komponente d5) im Reaktionsmedium gering, so kann sie in einem Monomeren oder in einer Monomerenmischung gelöst werden oder aber in einem Lösungsmittel gelöst zudosiert werden, das sich mit dem Reaktionsmedium mischt. Besonders bevorzugt sind solche Komponenten d5), die in der Monomermischung löslich sind.

Wird die Komponente d5) zur Herstellung des erfindungsgemäßen Polymers A eingesetzt, so beträgt die eingesetzte Menge wenigstens 0,01, bevorzugt wenigstens 0,05, besonders bevorzugt wenigstens 0,1 und höchstens 5, bevorzugt höchstens 2 und besonders bevorzugt höchstens 1 Gew.-%, bezogen auf die Gesamtmenge der Komponenten a) bis d).

Sollen die erfindungsgemäßen Polymere A eine Komponente d5) einpolymerisiert enthalten, so ist es besonders vorteilhaft, Mischungen der Komponenten c) und d5) einzusetzen. Solche Mischungen sind kommerziell erhältlich und enthalten neben der Komponente c) als sogenannte Reaktivverdünner beispielsweise Tripropylenglykoldiacrylat (TPGDA), Hexandioldiacrylat (HDDA), Dipropylenglykoldiacrylat (DPGDA), Trimethylolpropanformalmonoacrylat (z.B. Laromer®LR 8887), Trimethylolpropantriacrylat (TMPTA), propoxyliertes Glyceryltriacrylat (GPTA), Ethoxyliertes Trimethylolpropantriacrylat (EO3TMPTA), Ethoxyethoxyethylacrylat (EOEOEA), PEG 400-diacrylat (PEG400DA), Isobornylacrylat (IBOA), Propoxyliertes Neopentylglycoldiacrylat (P02NPGDA), 2-Phenoxyethylacrylat (POEA), Butandioldiacrylat (BDDA), Butandiolacrylat (BDMA), Dihydrodicyclopentadienylacrylat (DCPA), Triethylenglycoldivinylether, Ethyldigycolacrylat (EDGA), Laurylacrylat (LA), 4-t-butylcyclohexylacrylat (TBCH).

Als Komponente d) können auch Vinylacetat, Vinylpropionat, Vinylbutyrat, Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon eingesetzt werden.

Die Komponenten d) können auch silikonhaltige Strukturelemente umfassen.

Polymer A enthält 0-30, bevorzugt 0-20, besonders bevorzugt 1-15 und insbesondere 2-10 Gew.-% der Komponente d) einpolymerisiert.

Die in den erfindungsgemäßen Zubereitungen enthaltenen Polymere A enthalten bevorzugt

a) 65-80 Gew.-% der Komponente a),
b) 12-39 Gew.-% der Komponente b),
c) 1-8 Gew.-% der Komponente c) und
d) 0-20 Gew.-% der Komponente d)

einpolymerisiert, mit der Maßgabe, dass sich die Mengen der Komponenten a) bis d) zu 100 Gew.-% addieren.

Die in den erfindungsgemäßen Zubereitungen enthaltenen Polymere A enthalten besonders bevorzugt

a) 70-80 Gew.-% der Komponente a),
b) 15-29 Gew.-% der Komponente b),
c) 1-5 Gew.-% der Komponente c) und
d) 0-20 Gew.-% der Komponente d)

einpolymerisiert enthält, mit der Maßgabe, dass sich die Mengen der Komponenten a) bis d) zu 100 Gew.-% addieren.

Herstellung der erfindungsgemäßen Polymere A

Die Herstellung der erfindungsgemäßen Polymere A kann z.B. durch Lösungs-, Fällungs-, Suspensions- oder Emulsionspolymerisation erfolgen. Derartige Verfahren sind üblich und dem Fachmann bekannt.

Bevorzugt ist die Herstellung durch Lösungspolymerisation. Bevorzugt ist es, die Polymere A durch radikalische Lösungspolymerisation herzustellen.

Bevorzugte Lösungsmittel für die Polymerisation sind alkoholische oder alkoholisch/wässrige Lösungsmittel wie Ethanol oder Isopropanol und Gemische aus Ethanol oder Isopropanol mit Wasser und/oder weiteren Alkoholen wie Methanol, n-Propanol, n-Butanol, sek.-Butanol, tert.-Butanol, n-Hexanol und Cyclohexanol sowie Glykolen wie Ethylenglykol, Propylenglykol und Butylenglykol sowie den Methyl- oder Ethylethern der zweiwertigen Alkohole wie Diethylenglykol, Triethylenglykol, Polyethylenglykolen mit zahlenmittleren Molekulargewichten bis etwa 3000, Glycerin und Dioxan.

Besonders bevorzugt ist die Polymerisation in Alkohol, beispielsweise in Ethanol oder in einem Alkohol/Wasser-Gemisch, beispielsweise in einem Ethanol/Wasser-Gemisch.

Die Polymerisationstemperaturen liegen vorzugsweise in einem Bereich von etwa 30 bis 140°C, besonders bevorzugt 40 bis 120°C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Copolymerisation können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azobis(2-amidinopropan)hydrochlorid (Wako V-50®), 2,2'-Azobis[2-(2-imidazolin-2-yl)propan] (Wako VA-061®), 2,2'-Azobis(2-methyl-butyronitril) (Wako V-59®), Dimethyl 2,2'-azobis(2-methylpropionate) (Wako V-601®), 2,2'-Azobis(2,4-dimethylvaleronitril), 1,1'-Azo-bis-(1-cyclohexancarbonitril), 4,4'-Azobis(4-Cyanovaleriansäure) oder 2-(Carbamoylazo)-isobutyronitril.

Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/ Natriumhydroxymethansulfinat, $H_2O_2/Cu^1$, $H_2O_2$/Ascorbinsäure.

Als Oxidationsmittel für Redoxinitiatorsysteme kommen im wesentlichen die oben genannten Peroxide in Betracht. Als entsprechende Reduktionsmittel können Schwefelverbindungen mit niedriger Oxidationsstufe, wie Alkalisulfite, beispielsweise Kalium- und/oder Natriumsulfit, Alkalihydrogensulfite, beispielsweise Kalium- und/oder Natriumhydrogensulfit, Alkalimetabisulfite, beispielsweise Kalium- und/oder Natriummetabisulfit, Formaldehydsulfoxylate, beispielsweise Kalium- und/oder Natriumformaldehydsulfoxylat, Alkalisalze, speziell Kalium- und/oder Natriumsalze aliphatischer Sulfinsäuren und Alkalimetallhydrogensulfide, wie beispielsweise Kalium- und/oder Natriumhydrogensulfid, Salze mehrwertiger Metalle, wie Eisen-(II)-sulfat, Eisen-(II)-Ammoniumsulfat, Eisen-(II)-phosphat, Endiole, wie Dihydroxymaleinsäure, Benzoin und/oder Ascorbinsäure sowie reduzierende Saccharide, wie Sorbose, Glucose, Fructose und/oder Dihydroxyaceton eingesetzt werden.

Es kann auch vorteilhaft sein, Mischungen von wasserlöslichen und wenig oder nicht wasserlöslichen Initiatoren zu verwenden. Es kann auch vorteilhaft sein, Mischungen von organischen und anorganischen Initiatoren zu verwenden.

Geeignete Initiatoren sind beschrieben in den Kapiteln 20 und 21 von Macromolecules, Vol. 2, 2nd Ed., H. G. Elias, Plenum Press, 1984, New York, worauf hier vollumfänglich Bezug genommen wird. Weiterhin sind geeignete Photoinitiatoren beschrieben in S. P. Pappas, J. Rad. Cur., July 1987, S.6, worauf hier vollumfänglich Bezug genommen wird.

Die Initiatoren werden üblicherweise in Mengen bis zu 10, vorzugsweise 0,02 bis 5 Gew.-%, bezogen auf die zu polymerisierenden Monomeren eingesetzt.

Der K-Wert der Polymeren liegt im Bereich von 15 bis 120, bevorzugt von 25 bis 75 und besonders bevorzugt von 25 bis 55 (Bestimmung nach Fikentscher, Cellulosechemie, Bd. 13, S. 58 bis 64 (1932)). Möglichkeiten, den K-Wert von Polymeren auf einen Wert in einem gewünschten Wertebereich einzustellen, sind dem Fachmann bekannt. Beispielsweise sind dies die Polymerisationstemperatur, die Initiatormenge oder die Verwendung von Kettenübertragungsreagenzien.

Zur Einstellung des Molekulargewichts kann die Polymerisation in Gegenwart wenigstens eines Kettenübertragungsreagens (Reglers) erfolgen. Als Kettenübertragungsreagens können die üblichen, dem Fachmann bekannten Verbindungen, wie z. B. Schwefelverbindungen, z. B. Mercaptoethanol, 2-Ethylhexylthioglycolat, Thioglycolsäure, Alkanthiole, Cystein, Acetylcystein sowie Tribromchlormethan oder andere Verbindungen, die regelnd auf das Molekulargewicht der erhaltenen Polymere wirken, eingesetzt werden.

Die Regler werden üblicherweise in Mengen von 0,1 bis 5 Gew.-%, insbesondere 0,25 bis 2 Gew.-% bezogen auf die zu polymerisierenden Monomere eingesetzt. Üblicherweise werden die Regler zusammen mit den Monomeren der Polymerisation zugesetzt.

Zur Erzielung möglichst reiner Polymere A mit geringem Restmonomergehalt kann sich an die Polymerisation (Hauptpolymerisation) wenigstens ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer WasserdampfDestillation unterzogen werden.

Die Copolymerisation erfolgt nach den üblichen Verfahrenstechniken der Lösungspolymerisation, z.B. nach der sogenannten Batchpolymerisation, bei der man die Monomeren sowie gegebenenfalls Polymerisationsregler und Initiator in einem Lösungsmittel vorlegt und auf die Polymerisationstemperatur erwärmt. Das Reaktionsgemisch wird bevorzugt solange bei der Polymerisationstemperatur gerührt, bis die Umsetzung der Monomeren mehr als 99,9% beträgt. Die Zugabe der Initiatoren kann bei diesen Verfahren gegebenenfalls auch erst nach Erreichen der Polymerisationstemperatur erfolgen.

Weitere Verfahrensvarianten sind Zulaufmethoden, die bevorzugt angewendet werden. Dabei werden einzelne oder alle Reaktionsteilnehmer ganz oder teilweise, absatzweise oder kontinuierlich, gemeinsam oder in getrennten Zuläufen zu einer Reaktionsmischung gegeben. So kann man beispielsweise zu einem Gemisch der Monomeren und eines Lösungsmittels bei der Polymerisationstemperatur innerhalb einer gegebenen Zeit gegebenenfalls eine Lösung des Polymerisationsreglers und eine Initiatorlösung kontinuierlich oder absatzweise zugeben. Es.ist jedoch auch möglich, eine Mischung aus Initiator und gegebenenfalls Regler der auf Polymerisationstemperatur erwärmten Vorlage zuzudosieren. Eine andere Variante besteht darin, den Initiator unterhalb oder bei der Polymerisationstemperatur in die Vorlage zu geben und, falls ein Regler verwendet werden soll, nur den Regler oder eine Lösung des Reglers nach Erreichen der Polymerisationstemperatur innerhalb eines vorgegebenen Zeitraums dem Reaktionsgemisch zuzuführen.

Das bei der Herstellung der Polymere eingesetzte organische Lösungsmittel kann durch übliche, dem Fachmann bekannte Verfahren, z. B. durch Destillation bei vermindertem Druck, entfernt werden. Die bei der Polymerisation entstandenen Mischungen können im Anschluß an den Polymerisationsprozeß einer physikalischen oder chemischen Nachbehandlung unterworfen werden. Solche Verfahren sind beispielsweise die bekannten Verfahren zur Restmonomerenreduzierung wie z.B. die Nachbehandlung durch Zusatz von Polymerisationsinitiatoren oder Mischungen mehrerer Polymerisationsinitiatoren bei geeigneten Temperaturen oder Erhitzen der Polymerisationslösung auf Temperaturen oberhalb der Polymerisationstemperatur, eine Nachbehandlung der Polymerlösung mittels Wasserdampf oder Strippen mit Stickstoff oder Behandeln der Reaktionsmischung mit oxidierenden oder reduzierenden Reagenzien, Adsorptionsverfahren wie die Adsorption von Verunreinigung an ausgewählten Medien wie z.B. Aktivkohle oder eine Ultrafiltration. Es können sich auch die bekannten Aufarbeitungsschritte anschließen, beispielsweise geeignete Trockenverfahren wie Sprüh-, Gefrier- oder Walzentrocknung oder an die Trocknung anschließende Agglomerationsverfahren. Die nach dem erfindungsgemäßen Verfahren erhaltenen restmonomerenarmen Mischungen können auch direkt in den Handel gebracht werden.

Pulverförmige Polymere haben den Vorteil einer besseren Lagerfähigkeit, einer einfacheren Transportmöglichkeit und zeigen in der Regel eine geringere Neigung für Keimbefall.

Neutralisation

Die erfindungsgemäßen Polymere A können teilweise oder vollständig neutralisiert werden. Insbesondere zur Verwendung der Polymere in haarkosmetischen Zubereitungen ist eine teilweise oder vollständige Neutralisation vorteilhaft. In bevorzugten Ausführungsformen sind die Polymere beispielsweise zu mindestens 10, bevorzugt zu mindestens 30, weiter bevorzugt zu mindestens 40, besonders bevorzugt zu mindestens 50, ganz besonders bevorzugt zu mindestens 70 und insbesondere zu mindestens 95 % neutralisiert.

In einer ganz besonders bevorzugten Ausführungsform sind die Polymere zu mindestens 99% neutralisiert. Am meisten bevorzugt ist die Neutralisation zu mindestens 100%.

Die Neutralisation kann während oder nach der Polymerisation erfolgen.

Es ist weiterhin vorteilhaft, wenn das Neutralisationsmittel in mehr als äquivalenter Menge zugesetzt wird, wobei unter äquivalenter Menge die Menge verstanden wird, die mindestens benötigt wird, um alle neutralisierbaren Gruppen der Polymere zu neutralisieren.

Die Neutralisation kann beispielsweise erfolgen mit

- einem Mono-, Di- oder Trialkanolamin mit 2 bis 5 Kohlenstoffatomen im Alkanolrest, der gegebenenfalls in veretherter

Form vorliegt, beispielsweise Mono-, Di- und Triethanolamin, Mono-, Di- und Tri-n-propanolamin, Mono-, Di- und Trii-sopropanolamin, 2-Amino-2-methylpropanol und Di(2-methoxyethyl)amin,

- einem Alkandiolamin mit 2 bis 5 Kohlenstoffatomen, beispielsweise 2-Amino-2-methylpropan-1,3-diol und 2-Amino-2-ethylpropan-1,3-diol, oder

- einem primären, sekundären oder tertiären Alkylamin mit insgesamt 5 bis 10 Kohlenstoffatomen, beispielsweise N, N-Diethylpropylamin oder 3-Diethylamino-1-propylamin.

Als Alkalimetallhydroxide eignen sich zur Neutralisation vor allem Natrium-, oder Kalium- sowie Ammoniumhydroxid.

Häufig werden gute Neutralisationsergebnisse mit 2-Amino-2-methylpropanol, Trii-sopropanolamin, 2-Amino-2-ethylpropan-1,3-diol, N,N-Dimethylaminoethanol oder 3-Diethylamino-1-propylamin erhalten.

In einer bevorzugten Ausführungsform der Erfindung werden zur Neutralisation Hydroxygruppen enthaltende Amine aus der Gruppe bestehend aus N,N-Dimethylethanolamin, N-Methyldiethanolamin, Triethanolamin, 2-Amino-2-Methyl-propanol und Mischungen daraus ausgewählt.

Dabei können sekundäre oder tertiäre Aminogruppen tragende Alkanolamine vorteilhafte Wirkungen zeigen.

Zur Neutralisation der Polymere in den erfindungsgemäßen Zubereitungen und Mitteln sind inbesondere Aminogruppen enthaltende Silikonpolymere geeignet. Geeignete Aminogruppen enthaltende Silikonpolymere sind beispielsweise die Silikon-Aminopolyalkylenoxid-Blockcopolymere der WO 97/32917, die Produkte Silsoft®A-843 (Dimethicone Bisamino Hydroxypropyl Copolyol) und Silsoft®A-858 (Trimethylsilyl Amodimethicone Copolymer) (beide Fa. Witco). Weiterhin sind auch die Neutralisationspolymere der EP-A 1 035 144 und insbesondere die silikonhaltigen Neutralisationspolymere gemäß Anspruch 12 der EP-A 1 035 144 geeignet.

Zur Erhöhung der Lagerstabilität der Polymerlösungen, ist es vorteilhaft, die Polymere im Anschluss an die Herstellung teilweise zu neutralisieren. Besonders vorteilhaft ist eine Neutralisation im unmittelbaren Anschluss an die Polymerisation im Bereich von 10 bis 20 Mol-%, bezogen auf die Gesamtmenge an Säuregruppen.

**Kosmetische Zubereitungen**

Die zuvor beschriebenen Polymere A eignen sich hervorragend zur Herstellung kosmetischer, insbesondere haarkosmetischer Zubereitungen. Sie dienen dabei z.B. als polymere Filmbildner. Sie sind universell in den verschiedensten kosmetischen, bevorzugt haarkosmetischen Zubereitungen einsetzbar und einformulierbar und mit den üblichen Komponenten verträglich.

Die Polymere A eignen sich vorteilhaft zur Erzeugung elastischer Frisuren bei gleichzeitig starker Festigung (auch bei hoher Luftfeuchtigkeit). Die erfindungsgemäßen Polymere A zeichnen sich durch gute Treibgasverträglichkeit, gute Löslichkeit in wässrig/alkoholischen Lösungsmittelgemischen, insbesondere durch die Eignung für einen Einsatz als optisch klare Low-VOC-Formulierungen und durch gute Auswaschbarkeit und Auskämmbarkeit ohne Flaking-Effekt aus. Zudem verbessern sie mit ihnen behandeltes Haar in seinen sensorisch erfassbaren Eigenschaften wie Griff, Volumen, Handhabbarkeit usw.. Haarsprayformulierungen auf Basis der erfindungsgemäßen Polymere A zeichnen sich durch gute Sprühbarkeit und gute rheologische Eigenschaften und äußerst geringe Klebrigkeit der resultierenden Filme aus. Die die Polymer A enthaltenden erfindungsgemäßen kosmetischen, bevorzugt haarkosmetischen Zubereitungen neigen nach dem Aufbringen nicht zur Schaumbildung. Neben der guten Verträglichkeit mit den üblichen kosmetischen Inhaltsstoffen trocknen die aufgetragenen Polymere A schnell.

Kosmetisch akzeptabler Träger B)

Bevorzugt handelt es sich bei den erfindungsgemäßen kosmetischen Zubereitungen um wässrige Zubereitungen, die wenigstens 10, bevorzugt wenigstens 20 und besonders bevorzugt wenigstens 30 Gew.-% Wasser enthatten. Die erfindungsgemäßen kosmetischen Zubereitungen weisen neben Wasser und den Polymeren A weiterhin wenigstens einen kosmetisch akzeptablen Träger B) auf, der ausgewählt ist unter

i) wassermischbaren organischen Lösungsmitteln, vorzugsweise $C_2$-$C_4$-Alkanolen, insbesondere Ethanol,
ii) Ölen, Fetten, Wachsen,
iii) von iii) verschiedenen Estern von $C_6$-$C_{30}$-Monocarbonsäuren mit ein-, zwei- oder dreiwertigen Alkoholen,
iv) gesättigten acyclischen und cyclischen Kohlenwasserstoffen,
v) Fettsäuren,
vi) Fettalkoholen,
vii) Treibmitteln (Treibgasen)

und Mischungen davon.

Geeignete Träger B und weitere, vorteilhaft zu verwendende Wirk- und Zusatzstoffe sind im Folgenden detailliert beschrieben. Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird. Die erfindungsgemäßen Zubereitungen können z.B. eine Öl- bzw. Fettkomponente B) aufweisen, die ausgewählt ist unter:Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestem; Vaselin; Estern, bevorzugt Estern von Fettsäuren, wie z. B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, isoPropylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkylladaten, etc. und Mischungen davon.

Geeignete Siliconöle B) sind z. B., lineare Polydimethylsiloxane, Poly(methylphenyl-siloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc..

Bei den erfindungsgemäßen kosmetischen Zubereitungen kann es sich um hautkosmetische, haarkosmetische, dermatologische, hygienische oder pharmazeutische Mittel handeln. Aufgrund ihrer filmbildenden und flexiblen Eigenschaften eignen sich die zuvor beschriebenen Polymere A insbesondere als Zusatzstoffe für Haar- und Hautkosmetika.

Vorzugsweise liegen die erfindungsgemäßen Zubereitungen, die die Polymere A enthalten, in Form eines Sprays, Gels, Schaums, einer Salbe, Creme, Emulsion, Suspension, Lotion, Milch oder Paste vor. Gewünschtenfalls können auch Liposomen oder Mikrosphären eingesetzt werden.

Vorzugsweise enthalten die erfindungsgemäßen kosmetischen Mittel wenigstens ein wie vorstehend definiertes Polymer A, wenigstens einen wie vorstehend definierten Träger B und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, Verdickern, Haarpolymeren, Haar und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettem, Collagen, Eiweisshydrolysaten, Lipiden, Antioxidantien, Entschäumern, Antistatika, Emollienzien und Weichmachern.

Die erfindungsgemäßen Zubereitungen besitzen bevorzugt in pH-Wert von 2,0 bis 9,3. Besonders bevorzugt ist der pH-Bereich zwischen 4 und 8. Als zusätzliche Co-Solventien können organische Lösungsmittel oder ein Gemisch aus Lösungsmitteln mit einem Siedepunkt unter 400°C in einer Menge von 0,1 bis 15 Gew.%, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.%.

In einer bevorzugten Ausführungsform der Erfindung weisen die erfindungsgemäßen Zubereitungen einen Anteil an flüchtigen organischen Komponenten von höchstens 80 Gew.-%, bevorzugt höchstens 55 Gew.-% und insbesondere höchstens 35 Gew.-% auf. Ein bevorzugter Gegenstand sind somit kosmetische, bevorzugt haarkosmetische Zubereitungen, die dem low-VOC-Standard, also VOC-80- bzw. VOC-55-Standard entsprechen.

Bevorzugt ist die Verwendung der Polymere A insbesondere in Haarsprayzubereitungen, welche die folgenden Bestandteile enthalten:

- teilweise oder vollständig neutralisiertes erfindungsgemäßes Polymer A;

- Wasser;

- kosmetisch übliches organisches Lösungsmittel wie beispielsweise Ethanol, Isopropanol und Dimethoxymethan, daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische;

- kosmetisch übliches Treibmittel wie beispielsweise n-Propan, iso-Propan, n-Butan, i-Butan, 2,2-Dimethylbutan, n-Pentan, Isopentan, Dimethylether, Difluorethan, Fluortrichlormethan, Dichlordifluormethan oder Dichlortetrafluorethan, HFC-152A (1,1-Difluorethan), HFC-134a (1,1,2,2-Tetrafluorethan), $N_2$, $N_2O$ und CO oder deren Gemische.

Zur Neutralisation der erfindungsgemäßen Polymere A und damit der Komponente b) und zur Einstellung des pH-Werts der kosmetischen, bevorzugt haarkosmetischen Zubereitungen werden vorteilhaft Alkanolamine eingesetzt. Beispiele (INCI) sind Aminomethylpropanol, Diethanolamine, Diisopropanolamine, Ethanolamine, Methylethanolamine, N-Lauryl Diethanolamine, Triethanolamine, Triisoproanolamine, usw.. Es können sowohl primäre Aminogruppen tragende als auch sekundäre Aminogruppen tragende Alkanolamine verwendet werden.

Außerdem können Alkalihydroxide (z.B. NaOH, bevorzugt KOH) und andere Basen zur Neutralisation verwendet werden (z.B. Histidin, Arginin, Lysin oder Ethylenediamine, Diethylentriamin, Melamin, Benzoguanamin). Alle angegebenen Basen können allein oder als Gemisch mit anderen Basen zur Neutralisation säurehaltiger kosmetischer Produkte eingesetzt werden.

Ein Gegenstand der vorliegenden Erfindung sind demnach wässrige kosmetische, bevorzugt haarkosmetische Zubereitungen die neben dem wenigstens einen Polymer A und dem Träger B mindestens noch einen Wirk- oder Zusatzstoff ausgewählt aus der Gruppe bestehend aus viskositätsmodifw-ierenden Stoffen, haarpflegenden Stoffen, haarfestigenden Stoffen, Silikonverbindungen, Lichtschutzstoffen, Fetten, Ölen, Wachsen, Konservierungsmitteln, Pigmenten, löslichen Farbstoffen, partikelförmigen Stoffen, und Tensiden enthält.

Erfindungsgemäße haarkosmetische Formulierungen enthalten in einer bevorzugten Ausführungsform

i) 0,05 bis 20 Gew.-% wenigstens eines Polymers A,
ii) 20 bis 99,95 Gew.-% Wasser und/oder Alkohol,
iii) 0 bis 50 Gew.-% wenigstens eines Treibgases,
iv) 0 bis 5 Gew.-% wenigstens eines Emulgators,
v) 0 bis 3 Gew.-% wenigstens eines Verdickers, sowie
vi) bis zu 25 Gew.-% weitere Bestandteile.

Unter Alkohol sind alle vorgenannten, in der Kosmetik üblichen Alkohole zu verstehen, z. B. Ethanol, Isopropanol, n-Propanol.

Treibmittels (Treibgase)

Als Treibmittel (Treibgase) kommen von den genannten Verbindungen vor allem die Kohlenwasserstoffe, insbesondere Propan, n-Butan, n-Pentan und Gemische hieraus sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Die erfindungsgemäßen kosmetischen, bevorzugt haarkosmetischen Zubereitungen eignen sich auch besonders für Pumpsprayzubereitungen ohne den Zusatz von Treibmitteln oder auch für Aerosolsprays mit üblichen Druckgasen wie Stickstoff, Druckluft oder Kohlendioxid als Treibmittel.

Eine wasserhaltige Standard-Aerosol-Sprayformulierung umfasst beispielsweise folgende Bestandteile:

■ zu 100 % mit 2-Amino-2-methylpropanol neutralisiertes erfindungsgemäßes Polymer
■ Alkohol
■ Wasser
■ Dimethylether und/oder Propan/ n-Butan und/oder Propan/iso-Butan,

Dabei beträgt die Gesamtmenge der flüchtigen organischen Komponenten bevorzugt höchstens 80, besonders bevorzugt höchstens 55 Gew.-% der Zubereitung.

Vorzugsweise enthalten die erfindungsgemäßen kosmetischen, bevorzugt haarkosmetischen Zubereitungen wenigstens ein erfindungsgemäßes Polymer A, wenigstens einen wie vorstehend definierten kosmetisch akzeptablen Träger B) und wenigstens einen weiteren, davon verschiedenen Wirk- oder Zusatzstoff, der ausgewählt ist unter kosmetisch aktiven Wirkstoffen, Emulgatoren, Tensiden, Konservierungsmitteln, Parfümölen, Verdickern, Haarpolymeren,

Haarconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollentien, Lanolin-Komponenten, Proteinhydrolysaten und Weichmachern.

Weitere Polymere

Zur gezielten Einstellung der Eigenschaften von kosmetischen, bevorzugt haarkosmetischen Zubereitungen kann es von Vorteil sein, die erfindungsgemäßen Polymere in Mischung mit weiteren (haar)kosmetisch üblichen Polymeren einzusetzen.

In einer weiteren bevorzugten Ausführungsform enthält das erfindungsgemäße Mittel 0,01 bis 15 Gew.%, vorzugsweise 0,5 bis 10 Gew.% mindestens eines synthetischen oder natürlichen nichtionischen, bevorzugt eines filmbildenden Polymers. Unter natürlichen Polymeren werden auch chemisch modifizierte Polymere natürlichen Ursprungs verstanden. Unter filmbildenden Polymeren werden solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden.

Als solche weiteren üblichen Polymere eignen sich dazu beispielsweise anionische, kationische, amphotere, zwitterionische und neutrale Polymere.

Beispiele für solche weiteren Polymere sind

- Copolymere aus Ethylacrylat und Methacrylsäure
- Copolymere aus N-tert.-Butylacrylamid, Ethylacrylat und Acrylsäure
- Polyvinylpyrrolidone
- Polyvinylcaprolactame
- Polyurethane
- Copolymere aus Acrylsäure, Methylmethacrylat, Octylacrylamid, Butylaminoethylmethylacrylat und Hydroxypropylmethacrylat,
- Copolymere aus Vinylacetat und Crotonsäure und/oder (Vinyl)-Neodecanoat,
- Copolymere aus Vinylacetat und/oder Vinylpropionat und N-Vinylpyrrolidon,
- carboxyfunktionelle Copolymere aus Vinylpyrrolidon, t-Butylacrylat, Methacrylsäure,
- Copolymere aus tert.-Butylacrylat, Methacrylsäure und Dimethicone Copolyol. Überraschenderweise wurde gefunden, dass kosmetische und bevorzugt haarkosmetische Zubereitungen, welche die Polymere A in Kombination mit weiteren Polymeren enthalten, unerwartete Eigenschaften aufweisen. Die erfindungsgemäßen kosmetischen und bevorzugt haarkosmetischen Zubereitungen sind insbesondere hinsichtlich der Gesamtheit ihrer kosmetischen Eigenschaften den Zubereitungen aus dem Stand der Technik überlegen.

Copolymere aus Ethylacrylat und Methacrylsäure (INCI Bezeichnung: Acrylates Copolymer), sind beispielsweise als Handelsprodukte Luviflex®Soft (BASF) erhältlich.

Copolymere aus N-tert.-Butylacrylamid, Ethylacrylat und Acrylsäure (INCI Bezeichnung: Acrylates/Acrylamide Copolymer) sind beispielsweise als Handelsprodukte Ultrahold Strenge Ultrahold 8® (BASF) erhältlich.

Polyvinylpyrrolidone (INCI Bezeichnung: PVP) sind beispielsweise unter den Handelsnamen Luviskol®K, Luviskol®K30 (BASF) und PVP K® (ISP) erhältlich.

Polyvinylcaprolactame (INCI: Polyvinylcaprolactame) sind beispielsweise unter dem Handelsnamen Luviskol Plus® (BASF) erhältlich.

Polyurethane (INCI: Polyurethane -1) sind beispielsweise unter dem Handelsnamen Luviset®PUR erhältlich.

Copolymere aus Acrylsäure, Methylmethacrylat, Octylacrylamid, Butytaminoethylmethylacrylat, Hydroxypropylmethacrylat (INCI: Octylacrylamide/ Acrylates/ Butylaminoethyl Methacrylate Copolymer) sind beispielsweise unter den Handelsnamen Amphomer®28-4910 und Amphomer®LV-71 (National Starch) bekannt.

Copolymere aus Vinylacetat und Crotonsäure (INCI: VA/Crotonate/Copolymer) sind beispielsweise unter den

Handelsnamen Luviset®CA 66 (BASF), Resyn®28-1310 (National Starch), Gafset® (GAF) oder Aristoflex®A (Celanese) erhältlich.

Copolymere aus Vinylacetat, Crotonsäure und (Vinyl)neodecanoate (INCI: VA/Crotonates/Neodecanoate Copolymer) sind beispielsweise unter den Handelsnamen Resyn®28-2930 (National Starch) und Luviset®CAN (BASF) erhältlich.

Copolymere aus Vinylacetat und N-Vinylpyrrolidon (INCI: PVP/VA) sind beispielsweise unter den Handelsnamen Luviskol VA® (BASF) und PVP/VA (ISP) erhältlich.

Carboxyfunktionelle Copolymere aus Vinylpyrrolidon, t-Butylacrylat, Methacrylsäure sind beipielsweise unter dem Handelsnamen Luviskol®VBM (BASF) erhältlich.

Copolymere aus tert.-Butylacrylat, Methacrylsäure und Dimethicone Copolyol sind beipielsweise unter dem Handelsnamen Luviflex®Silk (BASF) erhältlich.

Geeignete anionische Polymere sind von den Polymeren A verschiedene Homo- und Copolymerisate von Acrylsäure und Methacrylsäure oder deren Salzen, Copolymere von Acrylsäure und Acrylamid und deren Salzen, Natriumsalze von Polyhydroxycarbonsäuren, Copolymere von Acrylsäure und Methacrylsäure mit beispielsweise hydrophoben Monomeren, z.B. $C_4$-$C_{30}$-Alkylester der (Meth)acrylsäure, $C_4$-$C_{30}$-Alkylvinylester, $C_4$-$C_{30}$-Alkylvinylether und Hyaluronsäure so wie weitere unter den Handelsnamen Amerhold®DR-25, Ultrahold®, Luviset®P.U.R., Acronal®, Acudyne®, Lovocryl®, Versatyl®, Amphomer® (28-4910, LV-71), Placise®L53, Gantrez®ES 425, Advantage Plus®, Omnirez®2000, Resyn®28-1310, Resyn®28-2930, Balance®(0/55), Acudyne®255, Aristoflex®A oder Eastman AQ® bekannte Polymere.

Weiterhin umfasst die Gruppe der geeigneten Polymere beispielhaft Balance®CR (National Starch), Balance®47 (National Starch; Octylaaylamid/Acrylate/Butylaminoethylmethacrylate-Copolymer), Aquaflex®FX 64 (ISP; Isobutylen/Ethylmaleimicu Hydroxyethylmaleimid-Copolymer), Aquaflex®SF-40 (ISP / National Starch; VP/Vinyl Caprolactam/DMAPA Acrylate Copolymer), Allianz®LT-120 (ISP / Rohm & Haas; Acrylat/C1-2 Succinat/Hydroxyacrylat Copolymer), Aquarez® HS (Eastman; Polyester-1).

Geeignet sind auch die Polymere unter den Handelsnamen Diaformer®Z-400 (Clariant; Methacryloylethylbetain/Methacrylat-Copotymer), Diaformer®Z-711 (Clariant; Methacryloylethyl N-oxidlMethacrylat-Copolymer), Diaformer®Z-712 (Clariant; Methacryloylethyl N-oxide/Methacrylat-Copolymer), Omnirez®2000 (ISP; Monoethylester von Poly (Methylvinyletherl Maleinsäure in Ethanol), Amphomer®HC (National Starch; Acrylat/ Octylacrylamid-Copolymer), Amphömer®28-4910 (National Starch; Octylacrylamid/Acrylat/ Butylaminoethylmethacrylat-Copolymer), Advantage®HC 37 (ISP; Terpolymer aus Vinylcaprolactam/Vinylpyrrolidon/Dimethylaminoethylmethacrylat), Advantage®LC55 und LC80 oder LC A und LC E, Advantage®Plus (ISP; VA/Butyl Maleate/Isobornyl Acrylate Copolymer), Aculyne®258 (Rohm & Haas; Acrylat/ Hydroxyesteracrylat-Copolymer), Luviset®P.U.R. (BASF, Polyurethane-1), Eastman®AQ 48 (Eastman), Styleze®CC-10 (ISP; VP/DMAPA Acrylates Copolymer), Styleze® 2000 (ISP; VP/Acrylates/Lauryl Methaaylate Copolymer), DynamX® (National Starch; Polyurethane-14 AMP-Acrylates Copolymer), Resyn®XP (National Starch; Acrylates/ Octylacrylamide Copolymer), Fixomer® A-30 (Orideo Nalco; Polymethacrylsäure (und) Acrylamidomethylpropansulfonsäure), Fixate® G-100 (Noveon; AMP-Acrylates/Allyl Methacrylate Copolymer).

Geeignete Polymere sind auch Copolymere aus (Meth)acrylsäure und Polyetheracrylaten, wobei die Polyetherkette mit einem $C_8$-$C_{30}$-Alkylrest terminiert ist. Dazu zählen z.B. Acrylat/Beheneth-25-methacrylat-Copolymere, die unter der Bezeichnung Aculyn® (Rohm + Haas) erhältlich sind. Besonders geeignete Polymere sind weiterhin Copolymere aus t-Butylacrylat, Ethylacrylat, Methacrylsäure (z.B. Luvimer®100P, Luvimer®Pro55) und Copolymere aus Ethylacrylat und Methacrylsäure (z.B. Luvimer®MAE).

Geeignet sind auch vernetzte Polymere der Acrylsäure, wie sie unter der INCI-Bezeichnung Carbomer erhältlich sind. Derartige vernetzte Homopolymere der Acrylsäure sind kommerziell beispielsweise als Carbopol® (Noveon) erhältlich. Bevorzugt sind auch hydrophob modifizierte vernetzte Polyacrylat-Polymere, wie Carbopol®Ultrez 21 (Noveon). Solche weiteren Polymere können auch zur Rheologiemodifizierung der Zubereitungen, also als Verdicker eingesetzt werden.

Als zusätzliche Polymere weiterhin geeignet sind wasserlösliche oder wasserdisper gierbare Polyester, Polyharnstoffe, Polyurethane, Polyurethanharnstoffe, gegebenenfalls mit Alkoholen umgesetzte Maleinsäureanhydridcopolymere oder anionische Polysiloxane.

Weiterhin zur Verwendung gemeinsam mit den Polymeren A geeignete Polymere sind z.B. auch kationische und kationogene Polymere. Dazu zählen beispielsweise

- Copolymere aus N-Vinylpyrrolidon/N-Vinylimidazoliumsalzen (erhältlich beispielsweise unter den Handelsnamen Luviquat®FC, Luviquat®HM, Luviquat®MS, Luviquat®Care, Luviquat® UltraCare (BASF),
- Copolymere aus Vinylpyrrolidon, Methacrylamid, Vinylimidazol (Luviset®Clear)
- Copolymere aus N-Vinylcaprolactam/N-VinylpyrrolidoNN-Vinylimidazoliumsalzen (erhältlich beispielsweise unter dem Handelsnamen Luviquat®Hold),
- Copolymere aus N-Vinylpyrrolidon/Dimethylaminoethylmethaaylat, quaternisiert mit Diethylsulfat (erhältlich beispielsweise unter dem Handelsnamen Luviquat®PQ11),

- kationische Cellulosederivate (Polyquaternium-4 und -10),
- Acylamidcopolymere (Polyquatemium-7),
- Guar-hydroxypropyltrimethylammoniumchlorid (INCI: Hydroxypropyl Guar Hydroxypropyltrimonium Chloride),
- Polyethylenimine und deren Salze,
- Polyvinylamine und deren Salze,
- Polymere auf Basis von Dimethyldiallylammoniumchlorid (Merquat®),
- Polymere, die durch Reaktion von Polyvinylpyrrolidon mit quatemären Ammoniumverbindungen entstehen (Gafquat®),
- Hydroxyethylcellulose mit kationischen Gruppen (Polymer®JR) und
- kationische Polymere auf pflanzlicher Basis, z.B. Guarpolymere, wie die Jaguar®-Marken der Fa. Rhodia.

Als weitere Haarkosmetik-Polymere sind auch neutrale Polymere geeignet wie

- Polyvinylpyrrolidone,
- Copolymere aus N-Vinylpyrrolidon und Vinylacetat und/oder Vinylpropionat,
- Polysiloxane,
- Polyvinylcaprolactame und
- Copolymere mit N-Vinylpyrrolidon,
- Cellulosederivate,
- Polyasparaginsäuresalze und Derivate,
- Polyamide, z.B. auf Basis von Itaconsäure und aliphatischen Diaminen, wie in der DE-A-43 33 238 beschrieben,

Zu den vorgenannten Polymerarten gehören die unter den Handelsnamen bekannten Luviskol® (K, VA, Plus), PVP K, PVPNA, Advantage®HC, Luviflex®Swing, Kollicoat®IR, $H_2$OLD®EP-1.

Außerdem geeignet als weitere Polymere sind auch Biopolymere, d.h. Polymere, die aus natürlich nachwachsenden Rohstoffen gewonnen werden und aus natürlichen Monomerbausteinen aufgebaut sind, z.B. Cellulosederivate, Chitin-, Chitosan-, DNA-, Hyaluronsäure- und RNA-Derivate.

Geeignete Mischungspartner für die erfindungsgemäßen Polymere sind auch zwitterionische Polymere, wie sie beispielsweise in den deutschen Patentanmeldungen DE 39 29 973, DE 21 50 557, DE 28 17 369 und DE 37 08 451 offenbart sind sowie Methacroylethylbetain/Methacrylat-Copolymere, die unter der Bezeichnung Amersette® (Amerchol) im Handel erhältlich sind oder Copolymere aus Hydroxyethylmethacrylat, Methylmethacrylat, N,N-Dimethylaminoethylmethaaylat und Acrylsäure (Jordapon®). Weitere geeignete Polymere sind auch betainische Polymere wie Yukaformer (R205, SM) und Diaformer.

Als Mischungspartner geeignete Polymere sind auch nichtionische, siloxanhaltige, wasserlösliche oder -dispergierbare Polymere, z.B. Polyethersiloxane, wie Tegopren® (Goldschmidt) oder Belsil® (Wacker).

Kosmetisch und/oder dermatologisch aktive Wirkstoffe

Geeignete kosmetisch und/oder dermatologisch aktive Wirkstoffe sind z. B. färbende Wirkstoffe, Haut- und Haarpigmentierungsrnittel, Tönungsmittel, Bräunungsmittel, Bleichmittel, Keratin-härtende Stoffe, antimikrobielle Wirkstoffe, Lichtfiltervvirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Bevorzugte kosmetische Pflege- und Wirkstoffe sind AHA-Säuren, Fruchtsäuren, Ceramide, Phytantriol, Collagen, Vitamine und Provitamine, beispielsweise Vitamin A, E und C, Retinol, Bisabolol und Panthenol. Besonders bevorzugt als kosmetischer Pflegestoff in den erfindungsgemäßen Zubereitungen ist Panthenol, das beispielsweise als D-Panthenol®USP, D-Panthenol®50 P, D-Panthenol®75 W, D,L-Panthenol®50 W kommerziell erhältlich ist.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt.

Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc..

Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc.. Derartige desodorierend wirkende Stoffe sind z. B. Zinkricinoleat, Triclosan, Undecylensäurealkylolamide, Citronensäuretriethylester, Chlorhexidin etc.. Die erfindungsmäßen Zubereitungen enthalten bevorzugt 0,01 bis 5, besonders bevorzugt 0,05 bis 1 Gew.% mindestens eines Konservierungsmittels. Geeignete weitere Konservierungsmittel sind die im International Cosmetic Ingredient Dictionary and Handbook, 9. Auflage mit der Funktion "Preservatives" aufgeführten Stoffe, z.B. Phenoxyethanol, Benzylparaben, Butylparaben, Ethylparaben, Isobutylparaben, Isopropylparaben, Methylparaben, Propylparaben, Iodopropinylbutylcarbamat, Methyldibromoglutaronitril, DMDM Hydantoin.

UV-Filtersubstanzen

Die erfindungsgemäßen Zubereitungen können in einer Ausführungsform öllösliche und/oder wasserlösliche UVA- und/oder UVB-Filter enthalten.

Die Gesamtmenge der Filtersubstanzen beträgt vorzugsweise 0,01 bis 10 Gew.% oder von 0,1 bis 5 Gew.%, besonders bevorzugt von 0,2 bis 2 Gew.%, bezogen auf das Gesamtgewicht der Zubereitungen.

Der größte Teil der Lichtschutzmittel in den zum Schutz der menschlichen Epidermis dienenden Zubereitungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Die UVB-Filter können öllöslich oder wasserlöslich sein. Vorteilhafte UVB-Filtersubstanzen sind z.B.:

i) Benzimidazolsulfonsäure-Derivate wie z.B. 2-Phenylbenzimidazol-5-sulfonsäure und deren Salze
ii) Benzotriazol-Derivate wie z.B. 2,2'-Methylen-bis-(6-(2H-benzotriazol-2-yl)-4-(1,1,3,3-tetramethylbutyl)-phenol)
iii) 4-Aminobenzoesäure-Derivate, vorzugsweise 4-(Dimethytamino)-benzoesäure(2-ethylhexyl)ester, 4-(Dimethy1amino)benzoesäureamylester;
iv) Ester der Benzalmalonsäure, vorzugsweise 4-Methoxybenzalmalonsäuredi(2-ethylhexyl)ester;
v) Ester der Zimtsäure, vorzugsweise 4-Methoxyzimtsäure(2-ethylhexyl)ester, 4-Methoxyzimtsäureisopentylester;
vi) Derivate des Benzophenons, vorzugsweise 2-Hydroxy-4-methoxybenzophenon, 2-Hydroxy-4-methoxy-4'-methyl benzophenon, 2,2'-Dihydroxy-4-methoxybenzophenon;
vii) Methylidencampherderivate, vorzugsweise 4-Methylbenzylidencampher, Benzyl idencampher;
viii) Triazinderivate, vorzugsweise 4,4',4"-(1,3,5-Triazin-2,4,6-triylimino)-trisbenzoesäure-tris-(2-ethylhexylester) [INCI: Diethylhexyl Butamido Triazine, UVA-Sorb® HEB (Sigma 3V)] und 2,4,6-Tris-[anilino-(p-carbo-2'-ethyl-1'-hexyloxy)]-1 ,3,5-triazin [INCI: Octyl Triazone, Uvinul®T 150 (BASF)].

Vorteilhaft zu verwendende wasserlösliche UVB-Filtersubstanzen sind z.B. Sulfonsäure-Derivate des 3-Benzylidencamphers, wie z.B. 4-(2-Oxo-3-bornylidenmethyl) benzolsulfonsäure, 2-Methyl-5-(2-oxo-3-bornylidenmethyl)sulfonsäure und deren Salze.

Vorteilhaft zu verwendende UVA-Filter sind z.B.:

- 1,4-Phenylendimethincamphersulfonsäurederivate wie z.B. 3,3'-( 1,4-phenylendimethin)-bis-(7,7-dimethyl-2-oxobicyclo[2.2.1]-heptan-1-methamsulfonsäure und ihre Salze
- 1,3,5-Triazinderivate wie 2,4-Bis{[(2-ethylhexyloxy)-2-hydroxy)-phenyl}-6-(4-methoxyp henyl)-1,3,5)-triazin (z.B. Tinosorb®S (Ciba))
- Dibenzoylmethanderivate, vorzugsweise 4-Isopropyldibenzoylmethan, 4-(tert.-Butyl)-4'-methoxydibenzoylmethan
- Benzoxazol-Derivate, beispielsweise das 2,4-Bis-[4-[5-(1,1-dimethylpropyl)benzoxazol-2-yl]phenylimino]-6-[(2-ethylexyl)imino]-1,3,5- triazin (CAS Nr. 288254-1 6-0, Uvasorb®K2A (3V Sigma))
- Hydroxybenzophenone, beispielsweise der 2-(4'-Diethylamino-2'-hydoxybenzoyl)-benzoesäurehexylester (auch: Aminobenzophenon) (Uvinul®A Plus (BASF))

Ferner kann erfndungsgemäß gegebenenfalls von Vorteil sein, Zubereitungen mit weiteren UVA- und/oder UVB-Filtern zu versehen, beispielsweise bestimmten Salicylsäurederivaten wie 4-Isopropylbenzylsalicylat, 2-Ethylhexylsalicylat, Octylsalicylat, Homomenthylsalicylat. Die Gesamtmenge an Salicylsäurederivaten in den kosmetischen Zubereitungen wird vorteilhaft aus dem Bereich von 0,1-15,0, bevorzugt 0,3-10,0 Gew.-% gewählt, bezogen auf das Gesamtgewicht der Zubereitungen. Ein weiterer erfindungsgemäß vorteilhaft zu verwendender Lichtschutzfilter ist Ethylhexyl-2-cyano-3,3-diphenylacrylat (Octocrylen, Uvinul®N 539 (BASF)).

Die folgende Tabelle stellt beispielhaft einige für die Verwendung in den erfindungsgemäßen Zubereitungen geeignete Lichtschutzfilter zusammen:

| Nr. | Stoff | CAS-Nr. |
|---|---|---|
| 1 | 4-Aminobenzoesäme | 150-13-0 |

(fortgesetzt)

| Nr. | Stoff | CAS-Nr. |
|---|---|---|
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4--methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenboman-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4- |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethy)-1-[(trimethylsilyl)oxy] disiloxanyl]propyl]phenol | 155633-54-8 |

(fortgesetzt)

| Nr. | Stoff | CAS-Nr. |
|---|---|---|
| 38 | 1,1-[(2,2'-Dimethylpropoxy)carbonyl]-4,4-diphenyl-1,3-butadien | 363602-15-7 |

Geeignete UV-Lichtschutzfilter mit der CAS-Nr. 113010-52-9 sind beispielsweise unter der Bezeichnung Uvinul®P 25 kommerziell erhältlich.

Auch polymere oder polymergebundene Filtersubstanzen können erfindungsgemäß verwendet werden.

Metalloxide wie Titandioxid oder Zinkoxid können ebenfalls vorteilhaft zum Schutz vor schädlicher Sonnenstrahlung eingesetzt werden. Ihre Wirkung beruht im wesentlichen auf Reflexion, Streuung und Absorption der schädigenden UV-Strahlung und hängt wesentlich von der Primärpartikelgröße der Metalloxide ab. Die erfindungsgemäßen kosmetischen Zubereitungen können vorteilhafterweise außerdem anorganische Pigmente auf Basis von Metalloxiden und/oder anderen in Wasser schwerlöslichen oder unlöslichen Metallverbindungen, ausgewählt aus der Gruppe der Oxide des Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden enthalten. Besonders bevorzugt handelt es sich um Pigmente auf der Basis ZnO.

Die anorganischen Pigmente können dabei in gecoateter Form vorliegen, d.h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.

Zur Verwendung in den erfindungsgemäßen Zubereitungen geeignete Lichtschutzmittel sind die in der EP-A 1 084 696 in den Absätzen [0036] bis [0053] genannten Verbindungen, worauf an dieser Stelle vollumfänglich Bezug genommen wird. Für die erfindungsgemäße Verwendung geeignet sind alle UV-Lichtschutzfilter, die in Anlage 7 (zu § 3b) der deutschen Kosmetik-Verordnung unter "Ultraviolett-Filter für kosmetische Mittel" genannt sind.

Die Aufzählung der genannten UV-Lichtschutz-Filter, die in den erfindungsgemäßen Zubereitungen verwendet werden können, ist nicht abschließend.

Verdickungsmittel

Geeignete Verdickungsmittel sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird.

Konsistenzregulatoren ermöglichen die Einstellung der gewünschten Viskosität von beispielsweise Shampoos. Verdicker, die durch eine Vergrößerung der Tensidmicellen bzw. durch eine Quellung der Wasserphase viskositätsaufbauend wirken, entstammen chemischsehr unterschiedlichen Stoffklassen.

Geeignete Verdickungsmittel für die erfindungsgemäßen Zubereitungen sind vernetzte Polyacrylsäuren und deren Derivate, Polysaccharide wie Xanthan-gum, Guar-Guar, Agar-Agar, Alginate oder Tylosen, Cellulosederivate, z. B. Carboxymethylcellulose oder Hydroxycarboxymethylcellulose, ferner höhermolekulare Polyethylenglycolmono- und -diester von Fettsäuren, Fettalkohole, Monoglyceride und Fettsäuren, Polyvinylalkohol und Polyvinylpyrrolidon.

Geeignete Verdicker sind weiterhin Polyacrylate wie Carbopol® (Noveon), Ultrez® (Noveon), Luvigel® EM (BASF), Capigel®98 (Seppic), Synthalene® (Sigma), die Aculyn® Marken der Fa. Rohm und Haas wie Aculyn® 22 (Copolymerisat aus Acrylaten und Methacrylsäureethoxylaten mit Stearylrest (20 EO-Einheiten)) und Aculyn® 28 (Copolymerisat aus Acrylaten und Methacrylsäureethoxilaten mit Behenylrest (25 EO-Einheiten)).

Geeignete Verdickungsmittel sind weiterhin beispielsweise Aerosil-Typen (hydrophile Kieselsäuren), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit einge-engter Homologenverteilung oder Alkyloligoglucoside sowie Elektrolyte wie Kochsalz und Ammoniumchlorid.

Besonders bevorzugte Verdickungsmittel zur Herstellung von Gelen sind Ultrez®21, Aculyn®28, Luvigel® EM und Capigel®98.

Insbesondere bei höher konzentrierten Shampoo-Formulierungen können zur Regulierung der Konsistenz auch Stoffe zugesetzt werden, die die Viskosität der Formulierungverringern, wie z. B. Propylenglykol oder Glycerin. Diese Stoffe beeinflussen die Produkteigenschaften nur wenig.

Gelbildner

Wird für die erfindungsgemäßen Zubereitungen der Einsatz von Gelbildnern gewünscht, so können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquatemium-32

(and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquatemium-37 (and) Paraffinum Liquidum (and) PPG-1 Trideceth-6, Polyquatemium 37 (and) Propylene Glycole Dicaprate Dicaprylate (and) PPG-1 Trideceth-6, Polyquaternium-7, Polyquaternium-44.

Emulgatoren

Als Emulgatoren kommen beispielsweise nichtionogene Tenside aus mindestens einer der folgenden Gruppen in Frage:

- o Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- o C12/18-Fettsäuremono- und -diester von Anlagerungsprodukten von 1 bis 30 Mol Ethylenoxid an Glycerin;
- o Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- o Alkylmono- und -oligoglycoside mit 8 bis 22 Kohlenstoffatomen im Alkylrest und deren ethoxylierte Analoga;
- o Anlagerungsprodukte von 15 bis 60 Mol Ethylenoxid an Ricinusöl und/oder gehärtetes Ricinusöl;
- o Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
- o Anlagerungsprodukte von 2 bis 15 Mol Ethylenoxid an Ricinusöl und/oder gehärtetes Ricinusöl;
- o Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
- o Mono-, Di- und Trialkylphosphate sowie Mono-, Di- und/oder Tri-PEGalkylphosphate und deren Salze;
- o Wollwachsalkohole;
- o Polysiloxan-Polyalkyl-Polyether-Copolymere bzw. entsprechende Derivate;
- o Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglycose und Polyolen, vorzugsweise Glycerin oder Polyglycerin sowie
- o Polyalkylenglykole.

Die Anlagenrngsprodukte von Ethylenoxid und/oder von Propylenoxid an Fettalkohole, Fettsäuren, Alkylphenole, Glycerinmono- und -diester sowie Sorbitanmono- und - diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12}$ bis $C_{18}$-Fettsäuremono- und -diester von Anlagerungsprodukten von Ethylenoxid an Glycerin sind aus DE-PS 2024051 als Rückfettungsmittel für kosmetische Zubereitungen bekannt. $C_8$ bis $C_{18}$-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidesters gilt, daß sowohl Monoglycoside, bei denen ein cydischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside

werden solche oberflächenaktiven Verbindungen bezeichnet, die im Molekül mindestens eine quartäre Ammoniumgruppe und mindestens eine Carboxylat- und/oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxy-methylglycinat.

Besonders bevorzugt ist das unter der CTFA-Bezeichnung Cocamidoprdpyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_8$ bis $C_{18}$-Alkyl- oder -Acylgruppe im Molekül mindestens eine freie Aminogruppe und mindestens eine -COOH- und/oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäüren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12}$ bis $C_{18}$-Acylsarcosin.

Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Antioxidantien

Ein zusätzlicher Gehalt der Zubereitungen an Antioxidantien kann von Vorteil sein. Erfindungsgemäß können als Antioxidantien alle für kosmetische Anwendungen geeigneten oder gebräuchlichen Antioxidantien verwendet werden. Vorteilhaft werden die Antioxidantien gewählt aus der Gruppe bestehend aus Aminosäuren (z.B. Glycin, Histidin, Tyrosin, Tryptophan) und deren Derivaten, Imidazolen (z.B. Urocaninsäure) und deren Derivaten, Peptiden wie D,L-Camosin, D-Camosin, L-Camosin und deren Derivaten (z.B. Anserin), Carotinoide, Carotinen (z.B. α-Carotin, β-Carotin, γ-Lycopin) und deren Derivaten, Chlorogensäure und deren Derivaten, Liponsäure und deren Derivaten (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Coniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist es vorteilhaft, diese in Konzentrationen von 0,001 bis 10 Gew.-%, bezogen auf das Gesamtgewicht der Zubereitung, bereitzustellen.

Sofern Vitamin A, bzw. Vitamin-A-Derivate, bzw. Carotine bzw. deren Derivate das oder die Antioxidantien darstellen, ist vorteilhaft, diese in Konzentrationen von 0,001 bis 10 Gew.-%, bezogen auf das Gesamtgewicht der Zubereitung, bereitzustellen.

Parfümöle

Die kosmetischen, bevorzugt haarkosmetischen Zubereitungen können Parfümöle enthalten. Als Parfümöle seien beispielsweise Gemische aus natürlichen und synthetischen Riechstoffen genannt. Natürliche Riechstoffe sind Extrakte von Blüten (Lilie, Lavendel, Rose, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln

(Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, 4-tert.-Butylcydohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonat, zu den Ketonen z.B. die Jonone, cc-Isomethylionen und Methylcedrylketon, zu den Alkoholen Anethof, Citronellol, Eugenol, Isoeugenol, Geraniol, Linalool, Phenylethylalkohol und Terioneol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzeöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen eingesetzt.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Silikonverbindungen

In einer Ausführungsform enthalten die erfindungsgemäßen Zubereitungen als haarpflegenden Zusatzstoff mindestens eine Silikonverbindung in einer Menge von vorzugsweise 0,01 bis 15 Gew.%, besonders bevorzugt von 0,1 bis 5 Gew.%. Die Silikonverbindungen umfassen flüchtige und nicht-flüchtige Silikone und in dem Mittel lösliche und unlösliche Silikone. Bei einer Ausführungsform handelt es sich um hochmolekulare Silikone mit einer Viskosität von 1.000 bis 2.000.000 cSt bei 25°C, vorzugsweise 10.000 bis 1.800.000 oder 100.000 bis 1.500.000. Die Silikonverbindungen umfassen Polyalkyl- und Polyarylsiloxane, insbesondere mit Methyl-, Ethyl-, Propyl-, Phenyl-, Methylphenyl- und Phenylmethylgruppen. Bevorzugt sind Polydimethylsiloxane, Polydiethylsiloxane, Polymethylphenylsiloxane. Bevorzugt sind auch glanzgebende, arylierte Silikone mit einem Brechungsindex von mindestens 1,46, oder mindestens 1,52. Die Silikonverbindungen umfassen insbesondere die Stoffe mit den INCI-Bezeichnungen Cyclomethicone, Dimethicone, Dimethiconol, Dimethicone Copolyol, Phenyl Trimethicone, Amodimethicone, Trimethylsilylamodimethicone, Stearyl Siloxysilicate, Polymethylsilsesquioxane, Dimethicone Crosspolymer. Geeignet sind auch Silikonharze und Silikonelastomere, wobei es sich um hochvemetzte Siloxane handelt.

Bevorzugte Silikone sind cyclische Dimethylsiloxane, lineare Polydimethylsiloxane, Blockpolymere aus Polydimethylsiloxan und Polyethylenoxid und/oder Polypropylenoxid, Polydimethylsiloxane mit end- oder seitenständigen Polyethylenoxid- oder Polypropylenoxidresten, Polydimethylsiloxane mit endständigen Hydroxylgruppen, phenylsubstituierte Polydimethylsiloxane, Silikonemulsionen, Silkonelastomere, Silikonwachse, Silikongums und aminosubstituierte Silikone (CTFA: Amodimethicone).

Haarkonditioniermittel

In einer Ausführungsform enthalten die erfindungsgemäßen Zubereitungen 0,01 bis 20, bevorzugt von 0,05 bis 10, besonders bevorzugt von 0,1 bis 5 Gew.% mindestens eines Konditioniermittels.

Erfindungsgemäß bevorzugte Konditionierungsmittel sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humectant, Skin-Conditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble

conditioning agent" aufgeführten Verbindungen. Weitere vorteilhafte Konditionierungsmittel stellen beispielsweise die nach INCI als Polyquaternium bezeichneten Verbindungen dar (insbesondere Polyquaternium-1 bis Polyquaternium-56).

Zu den geeigneten Konditionierungsmitteln zählen beispielsweise auch polymere quaternäre Ammoniumverbindungen, kationische Cellulosederivate, Chitosanderivate und Polysaccharide.

Das Konditioniermittel ist bevorzugt ausgewählt aus Betain, Panthenol, Panthenylethylether, Sorbitol, Proteinhydrolysaten, Pflanzenextrakten; A-B-Block-Copolymeren aus Alkylacrylaten und Alkylmethacryaten; A-B-Block-Copolymeren aus Alkylmethacrylaten und Acrylnitril; A-B-A-Block-Copolymeren aus Lactid und Ethylenoxid; A-B-A-Block-Copolymeren aus Caprolacton und Ethylenoxid; A-B-C-Block-Copolymeren aus Alkylen- oder Alkadienverbindungen, Styrol und Alkylmethacrylaten; A-B-C-Block-Copolymeren aus Acrylsäure, Styrol und Alkylmethacrylaten, sternförmigen Block-Copolymeren, hyperverzweigten Polymeren, Dendrimeren, intrinsisch elektrisch leitfähigen 3,4-Polyethylendioxythiophenen und intrinsisch elektrisch leitfähigen Polyanilinen.

Weitere erfindungsgemäß vorteilhafte Konditioniermittel stellen Cellulosederivate und quatemisierte Guargum Derivate, insbesondere Guar Hydroxypropylammoniumchlorid (z.B. Jaguar Excel®, Jaguar C 162® (Rhodia), CAS 65497-29-2; CAS 39421-75-5) dar. Auch nichtionische Poly-N-vinylpyrrolidoNPolyvinylaoetat-Copolymere (z.B. Luviskol®VA 64 (BASF)), anionische Acrylat-Copolymere (z.B. Luviflex®Soft (BASF)), und/oder amphotere Amid/Acrylat/Methacrylat Copolymere (z.B. Amphomer® (National Starch)) können erfindungsgemäß vorteilhaft als Konditioniermittel eingesetzt werden.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewichtvon 100 bis 1000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimetylolpropan, Trimetylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Öle, Fette und Wachse

Die erfindungsgemäßen kosmetischen, bevorzugt haarkosmetischen Zubereitungen können auch Öle, Fette oder Wachse enthalten. Solche werde vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat,

Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat Dicaprylyl Carbonat (Cetiol CC) und Cocoglyceride (Myritol 331), Butylen Glycol Dicaprylat/Dicaprat und Dibutyl Adipat sowie synthetische, halbsynthetische und natürliche Gemische solcher Ester, wie z.B. Jojobaöl.

Ferner können eine oder mehrere Ölkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäß vorteilhaft wird die Ölkomponente gewählt aus der Gruppe 2-Ethylhexylisostearat, Octyldodecanol, lsotridecylisononanoat, Isoeicosan, 2-Ethylhexylcocoat, C12-15-Alkylbenzoat, Capryl-Caprinsäure-triglycerid, Dicaprylylether.

Erfindungsgemäß vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Erfindungsgemäß besonders bevorzugt werden als Öle mit einer Polarität von 5 bis 50 mN/m Fettsäuretriglyceride, insbesondere Sojaöl und/oder Mandelöl eingesetzt. Ferner kann die Ölphase vorteilhaft gewählt werden aus der Gruppe der Guerbetalkohole. Diese sind selbst bei niederen Temperaturen flüssig und bewirken praktisch keine Hautreizungen. Vorteilhaft können sie als fettende, überfettende und auch rückfettend wirkende Bestandteile in kosmetischen Zusammensetzungen eingesetzt werden. Die Verwendung von Guerbet-Alkoholen in Kosmetika ist an sich bekannt. Erfindungsgemäß bevorzugte Guerbet-Alkohole sind 2-Butyloctanol (beispielsweise als Isofol®12 (Condea) kommerziell erhältlich) und 2-Hexyldecanol (beispielsweise als Isofol®16 (Condea) kommerziell erhältlich).

Auch Mischungen von erfindungsgemäßen Guerbet-Alkoholen sind erfindungsgemäß vorteilhaft zu verwenden wie beispielsweise Mischungen aus 2-Butyloctanol und 2-Hexyldecanol (beispielsweise als Isofol®14 (Condea) kommerziell erhältlich).

Auch beliebige Abmischungen solcher Öl- und Wachskomponenten sind vorteilhaft im Sinne der vorliegenden Erfindung einzusetzen. Unter den Polyolefinen sind Polydecene die bevorzugten Substanzen.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Vorteilhaft sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise Syncrowax®HRC (Glyceryltribehenat), und Syncrowax®AW 1 C ($C_{18-36}$-Fettsäure) sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Cetyl Ricinoleate wie beispielsweise Tegosoft®CR, Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride wie beispielsweise Hydriertes Soy Glycerid, Trihydroxystearin, Fettsäuren, Fettsäureester und Gly kolester wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan. Erfindungsgemäß können die Fett- und/oder Wachskomponenten sowohl einzeln als auch als Gemisch in den Zusammensetzungen verwendet werden.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C,2-15-Alkylbenzoat, Capryl-Caprin-säure-triglycerid, Dicaprylylether. Besonders vorteilhaft sind Mischungen aus Octyldodecanol, Capryl-Caprinsäure-triglycerid, Dicaprylylether, Dicaprylyl Carbonat, Cocoglyceriden oder Mischungen aus $C_{12-15}$-Alkylbenzoat und 2-Ethylhexylisostearat, Mischungen aus $C_{12-15}$-Alkylbenzoat und Butylen Glycol Dicaprylat/Dicaprat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Cydoparaffin, Squalan, Squalen, hydriertes Polyisobuten bzw. Polydecen vorteilhaft im Sinne der vorliegenden Erfindung zu verwenden.

Die Ölkomponente wird ferner vorteilhaft aus der Gruppe der Phospholipide gewählt. Als erfindungsgemäß vorteilhaftes Paraffinöl kann erfindungsgemäß Merkur®Weissoel Pharma 40 von Merkur Vaseline, Shell Ondina® 917,

Shell Ondina® 927, Shell

Oil 4222, Shell Ondina®933 von Shell & DEA Oil, Pionier® 6301 S, Pionier® 2071 (Hansen & Rosenthal) eingesetzt werden.

Geeignete kosmetisch verträgliche Öl- und Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier in vollem Umfang Bezug genommen wird.

Der Gehalt an Ölen, Fetten und Wachsen beträgt höchstens 30, bevorzugt 20, weiter bevorzugt höchstens 10 Gewichts-%, bezogen auf das Gesamtgewicht der Zusammensetzung.

Pigmente

In einer Ausführungsform enthalten die erfindungsmäßen Zubereitungen mindestens ein Pigment. Hierbei kann es sich um farbige Pigmente handeln, welche der Produktmasse oder dem Haar Farbeffekte verleihen oder es kann sich um Glanzeffektpigmente handeln, welche der Produktmasse oder dem Haar Glanzeffekte verleihen. Die Farb- oder Glanzeffekte am Haar sind vorzugsweise temporär, d.h. sie verbleiben bis zur nächsten Haarwäsche auf dem Haar und können durch Waschen der Haare mit üblichen Shampoos wieder entfernt werden.

Die Pigmente liegen in der Produktmasse in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 μm, insbesondere 3 bis 150 μm, besonders bevorzugt 10 bis 100 μm. Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxid rot, um Glanzpigmente , Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist:

Geeignet sind Metalloxide, -hydroxide und -oxidhydrate, Mischphasenpigmente, schwefelhaltige Silicate, Metallsulfide, komplexe Metallcyanide, Metallsulfate, - Chromate und -molybdate sowie die Metalle selbst (Bronze-Pigmente). Geeignet sind insbesondere Titandioxid (Cl 77891), schwarzes Eisenoxid (Cl 77499), gelbes Eisenoxid (Cl 77492), rotes und braunes Eisenoxid (Cl 77491 ), Manganviolett (Cl 77742), Ultramarine (Natrium-Aluminiumsulfosilikate, Cl T7007, Pigment Blue 29), Chromoxidhydrat (C177289), Eisenblau (Ferric Ferro-Cyanide, C17751 0), Carmine (Cochineal).

Besonders bevorzugt sind Periglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Rona®, Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

In einer Ausführungsform enthalten die erfindungsmäßen Zubereitungen 0,01 bis 10, besonders bevorzugt von 0,05 bis 5 Gew.% mindestens eines partikelförmigen Stoffes. Geeignete Stoffe sind z.B. Stoffe, die bei Raumtemperatur (25°C) fest sind und in Form von Partikeln vorliegen. Geeignet sind etwa Silica, Silikate, Aluminate, Tonerden, Mica, Salze, insbesondere anorganische Metallsalze, Metalloxide, z.B. Titandioxid, Minerale und Polymerpar6kel.

Die Partikel liegen in dem Mittel ungelöster, vorzugsweise stabil dispergierter Form vor und können sich nach Aufbringen auf die Anwendungsobertläche und Verdampfen des Lösungsmittels in fester Form abscheiden.

Bevorzugte partikelförmige Stoffe sind Silica (Kieselgel, Siliciumdioxid) und Metallsalze, insbesondere anorganische Metallsalze, wobei Silica besonders bevorzugt ist. Metallsalze sind z.B. Alkali- oder Erdalkalihalogenide wie Natriumchlorid oder Kaliumchlorid; Alkali- oder Erdalkalisulfate wie Natriumsulfat oder Magnesiumsulfat.

Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc.

Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc..

Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc..

Geeignete Antiphlogistika, die Hautreizungen entgegenwirken, sind z. B. Allantoin, Bisabolol, Dragosantol, Kamillenextrakt, Panthenol, etc..

Darreichungsform

Die erfindungsgemäßen Zubereitungen sind in einer bevorzugten Ausführungsform versprühbar, beispielsweise als Aerosol- oder Pumpspray-Zubereitung.

Die erfindungsgemäßen Zubereitungen können in verschiedenen Applikationsformen Anwendung finden, wie beispielsweise als Lotion, als Non-Aerosol Sprühlotion, welches mittels einer mechanischen Vorrichtung zum Versprühen zum Einsatz kommt; als Aerosol-Spray welches mittels eines Treibmittels versprüht wird, als Aerosol-Schaum oder als Non-Aerosol Schaum, welcher in Kombination mit einer geeigneten mechanischen Vorrichtung zum Verschäumen der Zusammensetzung vorliegt, als Haarcreme, als Haarwachs, als Gel, als Flüssiggel, als versprühbares Gel oder als Schaumgel. Auch ein Einsatz in Form einer mit einem üblichen Verdicker verdickten Lotion ist möglich.

In einer Ausführungsform liegt das erfindungsgemäße Mittel in Form eines Gels, in Form einer viskosen Lotion oder in Form eines Sprühgels, welches mit einer mechanischen Vorrichtung versprüht wird, vor und enthält mindestens eines der oben genannten Verdickungsmittel in einer Menge von vorzugsweise 0,05 bis 10, besonders bevorzugt von 0,1 bis 2 Gew.% und weist eine Viskosität von mindestens 250 mPas auf. Die Viskosität des Gels beträgt vorzugsweise von 500 bis 50.000 mPas, besonders bevorzugt von 1.000 bis 15.000 mPas bei 25°C.

In einer anderen Ausführungsform liegt die erfindungsgemäße Zubereitung in Form einer O/W-Emulsion, einer W/O-Emulsion oder einer Mikroemulsion vor und und enthält mindestens eines der oben genannten, in Wasser emulgierten Öle oder Wachse sowie mindestens ein kosmetisch übliches Tensid.

In einer bevorzugten Ausführungsform liegt die erfindungsgemäße Zubereitung in Form eines Sprühproduktes vor, entweder in Kombination mit einer mechanischen Pumpsprühvorrichtung oder in Kombination mit mindestens einem der vorgenannten Treibmittel. Ein bevorzugten Aerosolspray enthält zusätzlich Treibmittel in einer solchen Menge, dass die Gesamtmenge der flüchtigen organischen Komponenten 80, insbesondere 55 Gew.% der Zubereitung nicht übersteigt und wird in einem Druckbehälter abgefüllt.

Ein Non-Aerosol-Haarspray wird mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem die erfindungsgemäße kosmetische Zubereitung unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

In einer weiteren Ausführungsform liegt die erfindungsgemäße Zubereitung in Form eines verschäumbaren Produktes (Mousse) in Kombination mit einer Vorrichtungen zum Verschäumen vor, enthält mindestens eine übliche, hierfür bekannte schaumgebende Substanz, z.B. mindestens ein schaumbildendes Tensid oder mindestens ein schaumbildendes Polymer. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden. Das Produkt liegt entweder in Kombination mit einer mechanischen Pumpschäumvorrichtung (Pumpschaum) oder in Kombination mit mindestens einem Treibmittel (Aerosol-Schaum) in einer Menge von vorzugsweise 1 bis 20, insbesondere von 2 bis 10 Gew.%, vor. Treibmittel sind z.B. ausgewählt aus Propan, Butan, Dimethylether und fluorierten Kohlenwasserstoffen.

Ein Gegenstand der Erfindung ist somit eine kosmetischen, bevorzugt haarkosmetische Zubereitung in Form eines Sprühproduktes, wobei die Zubereitung entweder in Kombination mit einer mechanischen Pumpsprühvorrichtung oder in Kombination mit mindestens einem Treibmittel ausgewählt aus der Gruppe bestehend aus Propan, Butan, Dimethylether, fluorierten Kohlenwasserstoffen und deren Mischungen vorliegt.

Das Mittel wird ummittelbar vor der Anwendung verschäumt und als Schaum in das Haar eingearbeitet und kann anschließend ausgespült werden oder ohne Ausspülen im Haar belassen werden.

Eine erfindungsgemäß bevorzugte Formulierung für Aerosolhaarschäume enthält

i) 0,1 bis 10 Gew.-% wenigstens eines Polymers A,
ii) 55 bis 99,8 Gew.-% Wasser und Alkohol,
iii) 5 bis 20 Gew.-% eines Treibmittel,
iv) 0,1 bis 5 Gew.-% eines. Emulgators,
v) 0 bis 10 Gew.-% weitere Bestandteile,

wobei die Gesamtmenge von VOC höchstens 80 und bevorzugt 55 Gew.-% beträgt.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Cetheth-1, Polyethylenglykolcetylether; Cetearethe, z. B. Cetheareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).

Anionische Emulgatoren, können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

i) 0,1 bis 10 Gew.-% Polymer A,
ii) 80 bis 99,85 Gew.-% Wasser und Alkohol,
iii) 0 bis 3.Gew.-%, bevorzugt 0,05 bis 2 Gew.-%, eines Gelbildners,
iv) 0 bis 20 Gew.-% weitere Bestandteile.
wobei die Gesamtmenge von VOC höchstens 80 und bevorzugt 55 Gew.-% beträgt.

Bei der Herstellung von Gelen auf Basis der Polymere A können übliche Gelbildner eingesetzt werden, beispielsweise, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquatemium-32 (und) Paraffinum Liquidum (INCI), Natriumacrylat-Copolymere (und) Paraffinum Liquidum (und) PPG-1 Trideceth-6, Acrylamidopropyltrimoniumchlorid/AcrylamidCopolymere, Steareth-10 Allylether Acrylat-Copolymere, Polyquatemium-37 (und) Paraffinum Liquidum (und) PPG-1 Trideceth-6, Polyquatemium 37 (und) Propylenglycoldicapratdicaprylat (und) PPG-1 Trideceth-6, Polyquatemium-7, Polyquatemium-44. Als Gelbildner geeignete vernetzte Homopolymere der Acrylsäure sind beispielsweise kommerziell unter dem Namen Carbopol® (Noveon) erhältlich. Bevorzugt sind auch hydrophob modifizierte vernetzte Polyacrylat Polymere, wie Carbopol®Ultrez 21 (Noveon). Weitere Beispiele für als Gelbildner geeignete anionische Polymere sind Copolymere von Acrylsäure und Acrylamid und deren Salze; Natriumsalze von Polyhydroxycarbonsäuren, wasserlösliches oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem $C_8$-$C_{30}$-Alkylrest terminiert ist Dazu zählen z. B. AcrylatlBeheneth-25-methacrylat-Copolymere, die als Aculyn® (Rohm und Haas) kommerziell erhältlich sind.

In einer weiteren Ausführungsform liegt die erfindungsgemäße Zubereitung in Form eines Haarwachses vor, d.h. sie weist wachsartige Konsistenz auf und enthält mindestens eines der oben genannten Wachse in einer Menge von vorzugsweise 0,5 bis 30 Gew.% sowie gegebenenfalls weitere wasserunlösliche Stoffe. Die wachsartige Konsistenz ist vorzugsweise dadurch gekennzeichnet, dass die Nadelpenetrationszahl (Maßeinheit 0,1 mm, Prüfgewicht 100 g, Prüfdauer 5 s, Prüftemperatur 25°C; nach DIN 51 579) größer oder gleich 10, besonders bevorzugt größer oder gleich 20 ist und dass der Erstarrungspunkt des Produktes vorzugsweise größter oder gleich 30°C und kleiner oder gleich 70°C ist, besonders bevorzugt im Bereich von 40 bis 55°C liegt. Geeignete Wachse und wasserunlösliche Stoffe sind insbesondere Emulgatoren mit einem HLB-Wert unterhalb von 7, Silikonöle, Silikonwachse, Wachse (2.B. Wachsalkohole,

Wachssäuren, Wachsester, sowie insbesondere natürliche Wachse wie Bienenwachs, Carnaubawachs, etc.), Fettalkohole, Fettsäuren, Fettsäureester oder hydrophile Wachse wie z.B. hochmolekulare Polyethylenglykole mit einem Molekulargewicht von 800 bis 20.000, vorzugsweise von 2.000 bis 10.000 g/mol.

Wenn die erfindungsgemäße kosmetische, bevorzugt haarkosmetische Zubereitung in Form einer Haarlotion vorliegt, so liegt sie als im wesentlichen nicht-viskose oder gering viskose, fließfähige Lösung, Dispersion oder Emulsion mit einem Gehalt an mindestens 10 Gew.%, vorzugsweise 20 bis 95 Gew.% eines kosmetisch verträglichen Alkohols vor. Als Alkohole können insbesondere die für kosmetische Zwecke üblicher weise verwendeten niederen Alkohole mit 1 bis 4 Atomen, z.B. Ethanol und Isopropanol verwendet werden.

Wenn die erfindungsgemäße haarkosmetische Zubereitung in Form einer Haarcreme vorliegt, so liegt sie vorzugsweise als Emulsion vor und enthält entweder zusätzlich viskositätsgebende Inhaltsstoffe in einer Menge von 0,1

bis 10 Gew.% oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

Die erfindungsgemäßen Polymere A können in kosmetischen Zubereitungen als Konditioniermittel eingesetzt werden.

Die erfindungsgemäßen Polymere A können bevorzugt in Shampooformulierungen als Festigungs- und/oder Konditioniermittel eingesetzt werden. Bevorzugte Shampooformulierungen enthalten

i) 0,05 bis 10 Gew.-% wenigstens eines Polymers A,
ii) 25 bis 94,95 Gew.-% Wasser,
iii) 5 bis 50 Gew.-% Tenside,
iv) 0 bis 5 Gew.-% eines weiteren Konditioniermittels,
v) 0 bis 10 Gew.-% weitere kosmetische Bestandteile.

In den Shampooformulierungen können alle in Shampoos üblicherweise eingesetzten anionischen, neutralen, amphoteren oder, kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumläurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Polymeren A eingesetzt werden. Hierzu zählen beispielsweise die zuvor genannten kationischen Polymere mit der Bezeichnung Polyquatemium nach INCI, insbesondere Copolymere aus Vinylpyrrolidon/ N-Vinylimidazoliumsalzen (Luviquat®FC, Luviquat®HM, Luviquat®MS, Luviquat®Care, Luviquat®Ultracare), Copolymere aus N-Vinylpyrrolidon/Dimethylaminoethylmethacrylat, quaternisiert mit Diethylsulfat (Luviquat®PQ 11), Copolymere aus N-Vinylcaprolactam/ N-Vinylpyrrolidon/N-Vinylimidazoliumsalzen (Luviquat®Hold); kationische Cellulosederivate (Polyquaternium-4 und -10), Acrylamidcopolymere (Polyquatemium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Messmethoden

Bestimmung des K-Wertes

Die K-Werte werden nach Fikentscher, Cellulosechemie, Bd. 13, S. 58 bis 64 (1932) bei 25°C in Ethanol- oder N-Methylpyrrolidon (NMP)-Lösung gemessen und stellen ein Maß für das Molgewicht dar. Die Ethanol- oder NMP-Lösungen der Polymere enthalten jeweils 1g Polymer A in 100 ml Lösung.

Für den Fall, dass die Polymere in Form von wässrigen Dispersionen vorliegen, werden in Abhängigkeit vom Polymergehalt der Dispersion entsprechende Mengen der Dispersion mit Ethanol auf 100 ml aufgefüllt, so dass die Konzentration 1g in 100 ml beträgt.

Die Messung des K-Wertes erfolgt in einer Micro-Ubbelohde-Kapillare Typ M Ic der Fa. Schott.

Bestimmumg der Tröpfchengrößenverteilung (TGV) mittels Malvern®-Streulichtanalyse Die Bestimmung der

Tröpfchengrößenverteilung wurde mit Partiketgrößenmeßsystem zur Erfassung von Flüssigkeits-Aerosolen Malvern®Master Sizer X" vorgenommen (Malvern Instruments Inc., Southborough MA, USA).

Messprinzip:

Das Messsystem beruht auf der Methode der Laserlicht-Beugung am Partikel, die sich außer zur Spray-Analyse (Aerosole, Pumpsprays) auch zur Größenbestimmung von Feststoffen, Suspensionen und Emulsionen im Größenbereich von 0,1μm bis 2000μm eignet.

Ein Partikelkollektiv (=Tröpfchen) wird von einem Laser beleuchtet. An jedem Tröpfchen wird ein Teil des einfallenden Laserlichtes gestreut. Dieses Licht wird an einem Multielement-Detektor empfangen und die dazugehörige Lichtenergieverteilung bestimmt. Aus diesen Daten wird über die Auswertesoftware die dazugehörige Partikelverteilung berechnet.

Durchführung:

Die Aerosole wurden in einem Abstand von 29,5cm zum Laserstrahl eingesprüht. Der Sprühkegel trat rechtwinklig zum Laserstrahl ein.

Die Aerosoldosen wurden vor jeder Messung an einer fest installierten Haltevorrichtung fixiert, somit wurde erreicht, dass alle zu prüfenden Aerosole im exakt gleichen Abstand vermessen wurden.

Vor der eigentlichen Partikel messung wurde eine "Hintergrundmessung" durchgeführt. Dadurch wurden die Auswirkungen von Staub und anderen Verschmutzungen im Messbereich eliminiert.

Anschließend wurde das Aerosol in den Prüfraum eingesprüht. Das gesamte Partikelvolumen wurde über eine Prüfdauer von 2s erfasst und ausgewertet.

Auswertung:

Die Auswertung enthält eine tabellarische Darstellung über 32 Klassenbreiten von 0,5μm bis 2000μm und zusätzlich eine graphische Darstellung der Partikelgrößenverteilung.

Da es sich bei den Sprühversuchen um eine annähernd gleichmäßige Verteilung handelt, wird der mittlere Durchmesser "Mean Diameter" D(v,0.5) angegeben. Dieser Zahlenwert gibt an, dass 50% des gesamt gemessenen Partikelvolumens unterhalb dieses Wertes liegt.

Bei gut sprühbaren Aerosolsystemen im kosmetischen Bereich liegt dieser Wert, je nach Polymergehalt, Ventil- und Sprühkopfgeometrie, Lösungsmittelverhältnis und Treibgasmengen im Bereich von 30μm bis 80μm.

Bestimmung der Festigung (Biegesteifigkeit):

Die Festigung von polymeren Filmbildnern wurde außer durch subjektive Beurteilung (Handtest) auch physikalisch durch Messung der Biegesteifigkeit von dünnen Haarsträhnen (jeweils ca. 3 g und 24 cm Länge) gemessen. Dazu wurden die gewogenen,

trockenen Haarsträhnen in die 3,0 Gew.-%ige Polymerlösung (Lösungsmittel: Ethanol/Wasser 55:45 w/w) getaucht, wobei durch dreimaliges Eintauchen und Herausnehmen und anschließendes Ausdrücken zwischen Filterpapier eine gleichmäßige Benetzung der Haarsträhne und Verteilung der Polymerlösung sichergestellt wurde.

Die überschüssige Filmbildnerlösung wurde dann zwischen Daumen und Zeigefinger abgestreift und die Haarsträhnen wurden die Strähnen von Hand so geformt, dass sie einen runden Querschnitt erhielten. Bei 20°C und 65 % relativer Feuchte wurde über Nacht im Klimaraum getrocknet. Die Prüfungen wurden im Klimaraum bei 20°C und 65 % relativer Feuchte mittels eines Zug/Druck-Prüfgerätes durchgeführt. Die Haarsträhne wurde symmetrisch an den Enden auf zwei zylindrische Rollen der Probenaufnahme gelegt. Genau in der Mitte wurde die Strähne nun von oben mit einem abgerundetem Stempel ca.40 mm durchgebogen (Brechen des Polymerfilms). Die dafür erforderliche Kraft (Fmax) wurde mit einer Wägezelle (50 N) bestimmt. Dabei stellt ein Messwert das arithmetische Mittel aus den Einzelmessungen an 5 bis 10 gleich behandelten

Hasrstähnen dar. Die so ermittelten Werte wurden zu denen eines handelsüblichen Vergleichspolymers (Amphomer®LV-71) in Relation gesetzt und in % angegeben.

Bestimmung der Auswaschbarkeit:

Eine analog zur Bestimmung der Festigung mit Polymer behandelte Haarsträhne wurde in einer ca. 37°C warmen Texapon®NSO - Lösung (6ml Texapon®NSO (28 %ig) in 1l warmes Wasser) ca. 15 Sekunden durch 5maliges Eintauchen und Ausdrücken gewaschen. Anschließend wurde die Haarsträhne klargespült und nochmals in der gleichen Art behandelt. Danach wurde die Haarsträhne auf Filterpapier gut ausgedrückt und über Nacht trocknen

lassen. Die trockene Haarsträhne wurde eingedreht und auf Rückstände untersucht.

Bestimmung der Curl Retention

Grundrezeptur: (Aerosol-Haarspray)

| | |
|---|---|
| 5 Gew.-% | Wirkstoff zu prüfendes Polymer (100% neutr. mit AMP) |
| 15 Gew.-% | Ethanol |
| 40 Gew.-% | Wasser |
| 40 Gew.-% | Dimethylether. |

Für die Bestimmung der Curl Retention wurden Haarsträhnen von ca. 2 g Gewicht und 15,5 cm Länge und aus mittelbraunem, kaukasischem Menschenhaar verwendet.

Behandlung der Haarsträhnen:

Die Haarsträhnen wurden zweimal mit einer wässrigen Texapon®NSO-Lösung gewaschen. Anschließend wurden die Haarsträhnen mit warmem Wasser ausgespült, bis keine Schaumbildung mehr erkennbar war und mit VE-Wasser nachgespült, gekämmt und auf Filterpapier zum Trocknen gelegt.

Zur Herstellung einer Wasserwelle werden die Haarsträhnen 15 Minuten zum Quellen in einer Lösung aus Ethanol und Wasser (1:1) eingelegt.

Die Haarsträhne wurde vor der Lockenpräparation sorgfältig gekämmt. Mit einem Gummiband wurde die Haarsträhne am Plexiglasstab befestigt. Anschließend wurde gekämmt und spiralförmig gewickelt. Mit einem Baumwolltuch und Gummiband wurde die Locke fest fixiert und über Nacht bei 70°C getrocknet. Die abgekühlten Curl Retention-Strähnen wurden vorsichtig geöffnet und vom Plexiglasstab abgestreift, ohne die Wasserwelle zu deformieren. Aus einer Entfernung von 15 cm wurde wurden aus der wie vorgenannt hergestellten Aerosol-Haarpray 1,8g gleichmäßig auf die Locke ausgesprüht. Die Locke wurde dabei gleichmäßig gedreht. In horizontaler Lage wurden die Locken für 1 h bei Raumtemperatur getrocknet. Nach der Trocknung wurden die Locken in einer Halterung befestigt. Mit Hilfe eines Maßstabs wurde am Anfang die Ausgangslänge der Locken abgelesen und die Längenausdehnung während der Feuchtklimalagerung verfolgt. Nach 5h Lagerung bei 25°C und 90% r.F. in der Klimakammer wurde die erreichte Länge der Locke erneut abgelesen und die Curl Retention nach folgender Gleichung berechnet:

$$\text{Curl Retention in \%} = \frac{L - L_t}{L - L_o} * 100$$

L = Länge der Haare (15,5 cm)
$L_0$ = Länge der Haarlocke nach dem Trocknen
$L_t$ = Länge der Haarlocke nach Klimabehandlung

Als Curl Retention wurde der Mittelwert aus den 5 Einzelmessungen angegeben.

Bestimmung der Klebrigkeit

Es wurde zunächst eine klare, 20 Gew.-%ige ethanolische oder ethanolisch/wässrige Lösung des zu charakterisierenden Polymers hergestellt. Um eine klare Lösung zu erhalten, war es teilweise notwendig, das Polymer zu neutralisieren. Aus der ethanolischen oder ethanolisch/wässrigen Lösung wurde dann mit einem Rakel (120 μm Spaltbreite) ein Film des Polymers auf einer Glasplatte aufgebracht. Diese rechteckige Glasplatte hatte eine Länge von ca. 20 cm und eine Breite von ca. 6,5 m. Der darauf aufgebrachte Polymerfilm hatte jeweils eine Länge von ca. 16 bis 18 cm und eine Breite von ca. 5,5 cm.

Der Film wurde dann ca. 10 Stunden an der Luft getrocknet und anschließend im Klimaschrank bei 20°C und 80% relativer Feuchte weitere 12 Stunden gelagert.

Bei diesen Bedingungen wurde dann im Klimaschrank ein sich auf einem runden Gummistempel (Durchmesser 400 mm, Shore A-Härte 60 ± 5) befindendes Plastic-Carbon-Band (z.B. Pelikan®2060, 50 mm breit) mit einer Kraft von ca. 250 N für 10 Sekunden auf den Polymerfilm gepresst.

Die Menge des nach Entfernen des Stempels auf dem Polymerfilm haftend verbliebenen Schwarz-Pigmentes entspricht der Klebrigkeit des Filmes. Es erfolgte eine visuelle Beurteilung der Schwarzfärbung des Filmes. Die Beurteilungsskala reicht von 0 bis 5, wobei 0 nicht klebrig und 5 sehr stark klebrig bedeuten.

Bestimmung des Aussehens der Aerosol-Formulierung

Zubereitungen aus 5 Gew.-% des jeweiligen Polymers neutralisiert mit AMP, 40 Gew.-% DME, 15 Gew.-% Ethanol und 40 Gew.-% Wasser wurden in einen transparenten Glas-Aerosolbehälter gefüllt. Anschließend wurden die Klarheit des resultierenden Flüssigkeits/Treibgasgemisches visuell beurteilt.

Beispiele

Die folgenden Beispiele erläutern die Erfindung, ohne sie darauf zu beschränken.
Die Prozentangaben bedeuten Gew.-%, sofern nicht anderweitig bezeichnet.

Verwendete Abkürzungen:

| | |
|---|---|
| t-BA | tert.-Butylacrylat |
| MAS | Methacrylsäure |
| AS | Acrylsäure |
| ITS | Itaconsäure |
| EA | Ethylacrylat |
| MMA | Methylmethacrylat |
| EMA | Ethylmethacrylat |
| t-BMA | tert.-Butylmethacrylat |
| i-BMA | Isobutylmethacrylat |
| VE | voll entsalzt |

Herstellung der Polymere

Zur Herstellung der erfindungsgemäßen Polymere A wurden die folgenden Polyesteracrylate als Komponente c) eingesetzt:

Polyetheracrylat 1:

Herstellung gemäß Beispiel 1 der EP-A 0 279 303, S.3
Dispergierung analog DE 2853921, S.17, Beispiel 2.
Polyesteracrylat 1 wurde als Gew.-50% ige Dispersion eingesetzt, im Folgenden verkürzt als Polyesteracrylat 1 bezeichnet.

Polyesteracrylat 2:

Herstellung gemäß Vergleichsbeispiel 1 der EP-A 0 279 303, S.5
Dispergierung analog DE 2853921, S.17, Beispiel 2.
Polyesteracrylat 2 wurde als Gew.-50% ige Dispersion eingesetzt, im Folgenden verkürzt als Polyesteracrylat 2 bezeichnet

Polyetheracrylat 3 :

Herstellung gemäß EP-A 0 279 303, S.3, Beispiel 1.

Beispiel 1: Herstellung von Polymer 1 (Lösungspolymerisation in Ethanol)

Unter Rühren bei 20°C wurden die folgenden Zuläufe hergestellt:

Zulauf 1:

| | |
|---|---|
| 196 g | EMA |
| 72 g | MAS |
| 10 g | Polyetheracrylat 1 |
| 200g | Ethanol |

Zulauf 2 :

| | |
|---|---|
| 7 g | Wako®V 59 |
| 50 g | Ethanol |

Bei 20°C wurden eine Mischung aus 300g Ethanol, 15% der Gesamtmenge von Zulauf 1 sowie 15% der Gesamtmenge von Zulauf 2 hergestellt. Die Mischung wurde unter Normaldruck auf 78°C erhitzt. Nach Erreichen von 78°C wurden Zulauf 1 und Zulauf 2 gleichzeitig gestartet. Zulauf 1 wurde innerhalb von 3h sowie Zulauf 2 innerhalb von 4h mit gleichbleibendem Zulaufstrom zudosiert. Die Reaktionsmischung wurde während des gesamten Zulaufes bei 78 °C gehalten. Nach Ende des Zulaufes 2 wurde die Reaktionsmischung noch weitere 2h bei 78°C gehalten, anschließend wurde auf Raumtemperatur gekühlt.

Vergleichsbeispiel V1

In einem 2 l -Polymerisationsgefäß mit Rührer sowie Heiz- und Kühleinrichtungen wurden bei einer Temperatur von 20 bis 25 °C

| | |
|---|---|
| 400 g | entionisiertes Wasser |
| 0,6 g | einer 15 gew.-%igen wässrigen Lösung von Natriumlaurylsulfat |
| 35g | von Zulauf **II** (siehe unten) |

vorgelegt und unter Rühren und Stickstoffatmosphäre auf 45°C aufgeheizt. Nach Erreichen der Temperatur wurde Zulauf I innerhalb von 5 Minuten zugegeben. Anschließend wurde auf 80°C aufgeheizt und unter Rühren und Beibehaltung der Reaktionstemperatur Zulauf II innerhalb von 2,5 Stunden mit gleichbleibenden Zulaufströmen zudosiert. Nach Ende der Zuläufe wurde das Reaktionsgemisch für eine weitere Stunde bei 80 °C gerührt und dann auf 60°C abgekühlt. Unter Beibehaltung der Temperatur von 60°C wurde Zulauf III zugegeben. Anschließend wird auf 35°C abgekühlt und unter Beibehaltung der Temperatur wurde Zulauf IV zugegeben.

Zulauf I:

| | |
|---|---|
| 6 g | 7 gew.%ige wässrige Lösung von Natriumpersulfat in entionisiertem Wasser |

Zulauf II: wässrige Monomerenemulsion hergestellt aus

| | |
|---|---|
| 204g | entionisiertem Wasser |
| 8g | einer 15 gew.-%igen wässrigen Lösung von Natriumlaurylsulfat, |
| 40g | einer 25 Gew% igen Lösung von Tween™ 80 in VE Wasser, |
| 297g | Methylmethacrylat, |
| 99g | Methacrylsäure, |

(fortgesetzt)

2,4g n-Dodecylmercaptan.

Zu dem vorgelegten, entionisierten Wasser wurde unter Rühren die Gesamtmenge der 15 Gew.-%igen wässrigen Lösung von Natriumlaurylsulfat zugegeben. Zu der homogenen Lösung, die weiterhin gerührt wurde, wurden in der angegebenen Reihenfolge die entsprechenden Mengen
Methylmethacrylat,
25 Gew.-%ige Lösung von Tween™ 80 in VE-Wasser,
Methacrylsäure und
n-Dodecylmercaptan zugegeben.

Zulauf III:

4g 30 Gew%-ige Lösung von Wasserstoffperoxid in entionisiertem Wasser 0,3

Zulauf IV:

40g 10 Gew.%ige Lösung von Ammoniumhydrogencarbonat in entionisiertem Wasser

Die Herstellung der Beispiele 2, 3, 4, 10, 11, 12, 13 und Vergleichsbeispiel 3 (V3) in der folgenden Tabelle erfolgte analog Beispiel 1. Polymer V1 wurde durch Emulsionspolymerisation analog dem beschriebenen Beispiel hergestellt.

| Polymer | Monomere | Gew.-Verhältnis |
|---|---|---|
| V1 | MMA/MAS | 75/25 |
| V3 | MMA/MAS | 75/25 |
| | | |
| 1 | EMA/MAS/Polyetheraaylat 1 | 70,5/26/3,5 |
| 2 | MMA/MAS/ Polyetheracrylat 1 | 71/25/4 |
| 3 | t-BA/MAS/ Polyetheracylat 1 | 71,5/25/3,5 |
| 4 | t-BA/MAS/ Polyesteracrylat 2 | 71,5/25/3,5 |
| 10 | MMA/ITS/ Polyetheracrylat 1 | 71/25/4 |
| 11 | MMA/AS/ITS/ Polyetheracrylat 1 | 71/10/15/5 |
| 12 | MMA/MAS/ Polyetheracrylat 1 | 67/26/7 |
| 13 | EMA/MAS/AS/ Polyetheracrylat 1 | 67/14/12/7 |

Analog zu den obigen Beispielen können beispielsweise auch Polymere folgender Zusammensetzungen hergestellt werden:

| | |
|---|---|
| t-BA/MAS/ Polyetheracrylat 3 | 71,5/2513,5 |
| i-BMA/MAS/ Polyetheracrylat 1 | 71.512513,5 |
| t-BA/HEMA/MAS/Polyetheracrylat 1 | 42, 5/28/25/3, 5 |
| MMA/AS/MAS/ Polyetheracrylat 1 | 71/13/12/4 |

Anwendungstechnische Eigenschaften der erfindungsgemäßen Polymere A

| Beispiel | Klarheit als Aerosol | Auswaschbarkeit | Festigung [% im Vergleich mit Amphomer®LV71][a)] | TGV Malvem [μm] |
|---|---|---|---|---|
| V1 | fast klar | schlecht | 90 | 70 |
| V3 | angetrübt | - | - | - |
| 1 | klar | noch gut | 75 | 50 |
| 2 | fast klar | gut | 115 | 50 |
| 3 | klar | gut | 85 | 70 |
| 4 | klar | gut | 90 | 70 |
| 10 | klar | gut | 75 | 33 |
| 11 | fast klar | gut | 91 | 65 |
| 12 | klar | gut | 80 | 33 |
| 13 | klar | gut | 65 | 40 |
| [a)] VOC 55 Aerosol: 5% des jeweiligen Polymers, vollständig neutralisiert mit AMP,<br>40% DME,<br>15% Ethanol,<br>40% Wasser; | | | | |

Sprühvorrichtung:

Sprühkopf: Kosmos .020D Wirbel .018$^n$ 21-6443-20 (Fa. Precision Valve),
Ventil: DPV 33876 (Fa. Precision Valve)

II) Anwendungstechnische Beispiele

Falls nicht anders angegeben, werden alle eingesetzten Säuregruppen enthaltenden Polymere zu 100% neutralisiert mit 2-Amino-2-Methyl-propanol (AMP) eingesetzt. "Wasser ad 100" bedeutet, dass zu der jeweiligen Zirbereitung die zum Erreichen einer Gesamtmenge von 100 Gew.-% notwendige Restmenge an Wasser zugegeben wird.
Die Mengenangaben % sind Gew.-% sofern nicht anderweitig bestimmt.
Die Abkürzung "q.s." bedeutet "quantum satis", d.h. so viel eines Inhaltsstoffes zugeben, wie notwendig ist, um einen gewünschten Effekt zu erreichen.
Die Angabe (fest) bdeutet, dass die Menge des verwendeten Polymers auf Basis des Feststoffanteils berechnet wird, sofern das Polymer in Lösung vorliegt.

Beispiel 1a:

| VOC 55-Aerosol-Haarspray | [%] |
|---|---|
| Polymer aus Beispiel Nr. 2 (fest) | 5,00 |
| Dimethylether | 40,00 |
| Ethanol | 15,00 |
| Wasser | ad 100 |

weitere Zusätze: Silikon, Parfüm, Entschäumer, UV-Absorber
Das Beispiel lässt sich jeweils mit den erfindungsgemäßen Polymeren 1 bzw. 3, 4, 10 bis 13 wiederholen. Es wird jeweils ein VOC 55-Aerosol-Haarspray mit guten Eigenschaften erhalten.

Beispiel 1b:

| VOC 55-Aerosol-Haarspray | [%] |
|---|---|
| Polymer aus Beispiel Nr. 2 (fest) | 3,00 |
| Balance® 0/55 (Fa. National Starch) | 2,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4, 10 bis 13 wiederholen. Es wird jeweils ein VOC 55-Aerosol-Haarspray mit guten Eigenschaften erhalten.

Beispiel 1c:

| VOC 55-Aerosol-Haarspray | [%] |
|---|---|
| Polymer aus Beispiel Nr. 2 (fest) | 3,00 |
| Acudyne® 180 (Fa. Rohm&Haas) | 1,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1d:

| VOC 55-Aerosol-Haarspray | [%] |
|---|---|
| Polymer aus Beispiel Nr. 2 (fest) | 3,00 |
| Amphomer® LV 71 (Fa. National Starch) | 2,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1e:

| VOC 55-Aerosol-Haarspray | [%] |
|---|---|
| Polymer aus Beispiel Nr. 2 (fest) | 3,00 |
| Acudyne® DHR (Fa. Rohm&Haas) | 1,00 |
| Dimethylether | 40,00 |
| Ethanol | 15,00 |

(fortgesetzt)

| | |
|---|---|
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1e:

| VOC 55-Aerosol-Haarspray | [%] |
|---|---|
| Polymer aus Beispiel Nr. 2 (fest) | 3,00 |
| Eastman® AQ 48 (Fa. Eastman-Kodak) | 2,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1f:

| VOC 55-Aerosol-Haarspray | [%] |
|---|---|
| Polymer aus Beispiel Nr. 2 (fest) | 3,00 |
| Resyn® 28-2930 (Fa. National Starch) | 2,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1g:

| VOC 55-Aerosol-Haarspray | [%] |
|---|---|
| Polymer aus Beispiel Nr. 2 (fest) | 3,00 |
| Balance® 47 (Fa. National Starch) | 2,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1 h:

| VOC 55-Aerosol-Haarspray | [%] |
|---|---|
| Polymer aus Beispiel Nr. 2 (fest) | 3,00 |
| Aquaflex® SF-40 (Fa. ISP) | 1,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 1i:

| VOC 55-Aerosol-Haarspray | [%] |
|---|---|
| Polymer aus Beispiel Nr. 2 (fest) | 3,00 |
| DynamX® (Fa. ISP) | 1,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2a:

| VOC 55-Aerosol-Haarspray | [%] |
|---|---|
| Polymer aus Beispiel Nr. 2 (fest) | 5,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2b:

| VOC 55-Aerosal-Haarspray | [%] |
|---|---|
| Polymer aus Beispiel Nr. 2 (fest) | 3,00 |
| Balance® 0/55 (Fa. National Starch) | 2,00 |
| Dimethylether | 35,00 |
| Propan/Butan | 5,00 |

(fortgesetzt)

| | |
|---|---|
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2c:

| VOC 55-Aerosol-Haarspray | [%] |
|---|---|
| Polymer aus Beispiel Nr. 2 (fest) | 3,00 |
| Acudyne® 180 (Fa. Rohm&Haas) | 1,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2d:

| VOC 55-Aerosol-Haarspray | [%] |
|---|---|
| Polymer aus Beispiel Nr. 2 (fest) | 3,00 |
| Amphomer® LV 71 (Fa. National Starch) | 2,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2e:

| VOC 55-Aerosol-Haarspray | [%] |
|---|---|
| Polymer aus Beispiel Nr. 2 (fest) | 3,00 |
| Acudyne® DHR (Fa. Rohm&Haas) | 1,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen.

Es wird jeweils ein VOC 55-Aerosol-Haarspray mit guten Eigenschaften erhalten.

Beispiel 2e:

| VOC 55-Aerosol-Haarspray | [%] |
|---|---|
| Polymer aus Beispiel Nr. 2 (fest) | 3,00 |
| Eastman® AQ 48 (Fa. Eastman-Kodak) | 2,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2f:

| VOC 55-Aerosol-Haarspray | [%] |
|---|---|
| Polymer aus Beispiel Nr. 2 (fest) | 3,00 |
| Resyn® 28-2930 (Fa. National Starch) | 2,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2g:

| VOC 55-Aerosol-Haarspray | [%] |
|---|---|
| Polymer aus Beispiel Nr. 2 (fest) | 3,00 |
| Balance® 47 (Fa. National Starch) | 2,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2h:

| VOC 55-Aerosol-Haarspray | [%] |
|---|---|
| Polymer aus Beispiel Nr. 2 (fest) | 3,00 |
| Aquaflex® SF-40 (Fa. ISP) | 1,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 2i:

| VOC 55-Aerosol-Haarspray | [%] |
|---|---|
| Polymer aus Beispiel Nr. 2 (fest) | 3,00 |
| DynamX® (Fa. ISP) | 1,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 3a:

| Aerosol-Haarspray mit Fluorcarbon-Treibmitteln | [%] |
|---|---|
| Polymer aus Beispiel Nr. 2 (fest) | 5,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3b:

| VOC 55-Aerosol-Haarspray | [%] |
|---|---|
| Polymer aus Beispiel Nr. 2 (fest) | 3,00 |
| Balance® 0/55 (Fa. National Starch) | 2,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen.

Es wird jeweils ein Aerosol-Haarspray mit guten Eigenschaften erhalten.

Beispiel 3c:

| VOC 55-Aerosol-Haarspray | [%] |
|---|---|
| Polymers aus Beispiel Nr. 2 (fest) | 3,00 |
| Aardyne® 180 (Fa. Rohm&Haas) | 1,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3d:

| VOC 55-Aerosol-Haarspray | [%] |
|---|---|
| Polymer aus Beispiel Nr. 2 (fest) | 3,00 |
| Amphomer® LV 71 (Fa. National Starch) | 2,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3e:

| VOC 55-Aerosol-Haarspray | [%] |
|---|---|
| Polymer aus Beispiel Nr. 2 (fest) | 3,00 |
| Acudyne® DHR (Fa. Rohm&Haas) | 1,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3e:

| VOC 55-Aerosol-Haarspray | [%] |
|---|---|
| Polymer aus Beispiel Nr. 2 (fest) | 3,00 |
| Eastman® AQ 48 (Fa. Eastman-Kodak) | 2,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3f:

| VOC 55-Aerosol-Haarspray | [%] |
|---|---|
| Polymer aus Beispiel Nr. 2 (fest) | 3,00 |
| Resyn® 28-2930 (Fa. National Starch) | 2,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3g:

| VOC 55-Aerosol-Haarspray | [%] |
|---|---|
| Polymer aus Beispiel Nr. 2 (fest) | 3,00 |
| Balance® 47 (Fa. National Starch) | 2,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3h:

| VOC 55-Aerosol-Haarspray | [%] |
|---|---|
| Polymer aus Beispiel Nr. 2 (fest) | 3,00 |
| Aquaflex® SF-40 (Fa. ISP) | 1,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 3i:

| VOC 55-Aerosol-Haarspray | [%] |
|---|---|
| Polymer aus Beispiel Nr. 2 (fest) | 3,00 |
| DynainX® (Fa. ISP) | 1,00 |
| Ethanol abs. | ad 100 |

(fortgesetzt)

| | |
|---|---|
| HFC 152A | 40,00 |

weitere Zusätze: Silikon, Parfüm, Entschäumer, UV-Absorber

Das Beispiel lässt sich jeweils mit den erfindungsgemäßen Polymeren 1 bzw. 3, 4 und 10 bis 13 wiederholen. Es wird jeweils ein Aerosol-Haarspray mit guten Eigenschaften erhalten.

Beispiel 4:

| Aerosol-Haarspray mit Fluorcarbon-Treibmitteln | [%] |
|---|---|
| Polymer aus Beispiel Nr. 2 (fest) | 5,00 |
| Dest. Wasser | ad 100 |
| HFC 152A | 10,00 |
| Dimethylether | 30,00 |
| Ethanol abs. | 30,00 |

weitere Zusätze: Silikon, Parfüm, Entschäumer, UV-Absorber

Das Beispiel lässt sich jeweils mit den Polymeren 1-11 bzw. 13-15 wiederholen. Es wird jeweils ein Aerosol-Haarspray mit guten Eigenschaften erhalten.

Beispiel 5:

| VOC 55-Aerosol-Haarspray | [%] |
|---|---|
| Polymer aus Beispiel Nr. 2 (fest) | 3,00 |
| Ultrahold® Strong (fest; Fa. BASF) | 1,00 |
| Dimethylether | 40,00 |
| Ethanol | 15,00 |
| + AMP | auf pH 8,3 |
| Wasser | ad 100 |

weiterer Zusatz: Silikon, Parfüm, Entschäumer

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 6:

| VOC 55-Aerosol-Haarspray | [%] |
|---|---|
| Polymer aus Beispiel Nr. 2 (fest) | 3,00 |
| Luvimer® Pro55 (fest, Fa. BASF) | 1,00 |
| Dimethylether | 40,00 |
| Ethanol | 15,00 |
| Wasser | ad 100 |

weiterer Zusatz: Silikon, Parfüm, Entschäumer .

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 7:

| VOC 55-Aerosol-Haarspray | [%] |
|---|---|
| Polymer aus Beispiel Nr. 2 (fest) | 3,00 |
| Luvimer® P.U.R (fest, Fa. BASF) | 1,00 |
| Dimethylether | 40,00 |
| Ethanol | 15,00 |
| Wasser | ad 100 |

weiterer Zusatz: Silikon, Parfüm, Entschäumer

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 8:

| VOC 55-Aerosol-Haarspray | [%] |
|---|---|
| Polymer aus Beispiel Nr. 2 (fest) | 3,00 |
| Resyn® 28-2930 (fest, Fa. National Starch) | 1,00 |
| Dimethylether | 40,00 |
| Ethanol | 15,00 |
| Wasser | ad 100 |

weiterer Zusatz: Silikon, Parfüm, Entschäumer

Das Beispiel lässt sich jeweils mit den erfindungsgemäßen Polymeren 1 bzw. 3, 4 und 10 bis 13 wiederholen. Es wird ebenfalls ein VOC 55-Aerosol-Haarspray mit guten Eigenschaften erhalten.

Beispiel 9:

| VOC 55-Aerosol-Haarspray | [%] |
|---|---|
| Polymer aus Beispiel Nr. 2 (fest) | 2,00 |
| Stepanhold®R-1*) (Fa. Stepan Chemical Co.) | 1,00 |
| Dimethylether | 40,00 |
| Ethanol | 15,00 |
| + AMP | auf pH 8,3 |
| Wasser | ad 100 |

weiterer Zusatz: Silikon, Parfüm, Entschäumer

*) Stepanhold®R-1 = Poly(Vinylpyrrolidon/Ethylmethacrylat/Methacrylsäure)

Das Beispiel lässt sich jeweils mit den erfindungsgemäßen Polymeren 1 bzw. 3, 4 und 10 bis 13 wiederholen. Es wird jeweils ein VOC 55-Aerosol-Haarspray mit guten Eigenschaften erhalten.

Beispiel 10:

| VOC 55-Handpumpenspray | [%] |
|---|---|
| Polymer aus Beispiel Nr. 2 (fest) | 7,00 |
| Ethanol | 55,00 |

(fortgesetzt)

| | |
|---|---|
| Wasser | ad 100 |

weiterer Zusatz: Silikon, Parfüm, Entschäumer

Das Beispiel lässt sich jeweils mit den erfindungsgemäßen Polymeren 1 bzw. 3, 4 und 10 bis 13 wiederholen. Es wird jeweils ein VOC 55-Handpumpenspray mit guten Eigenschaften erhalten.

Beispiel 11:

| VOC 80-Aerosol-Haarspray | [%] |
|---|---|
| Polymer aus Beispiel Nr. 2 (fest) | 12,00 |
| Dimethylether | 40,00 |
| Ethanol | 40,00 |
| Wasser | ad 100 |

weiterer Zusatz: Silikon, Parfüm, Entschäumer

Das Beispiel lässt sich jeweils mit den erfindungsgemäßen Polymeren 1 bzw. 3, 4 und 10 bis 13 wiederholen. Es wird jeweils ein VOC 80-Aerosol-Haarspray mit guten Eigenschaften erhalten.

Beispiel 11:

| Wässriges Handpumpenspray | [%] |
|---|---|
| Polymer aus Beispiel Nr. 2 (fest) | 4,00 |
| Luviset®Clear *) (fest) | 1,00 |
| Wasser | ad 100 |

weiterer Zusatz: wasserlösliches Silikon, Parfüm, Entschäumer.

*) Luviset®Clear: Poly(Vinylpyrrolidon/Methacrylsäureamid/Vinylimidazol), Fa. BASF

Das Beispiel lässt sich jeweils mit den erfindungsgemäßen Polymeren 1 bzw. 3, 4 und 10 bis 13 wiederholen. Es wird jeweils ein wässriges Handpumpenspray mit guten Eigenschaften erhalten.

Beispiel 12:

| Wässrig/ethanolische Festiger-Lösung | [%] |
|---|---|
| Polymer aus Beispiel Nr. 2 (fest) | 7,00 |
| dest. Wasser | ad 100 |
| Ethanol | 52,00 |

weiterer Zusatz: Silikon, Parfüm, Entschäumer

Das Beispiel lässt sich jeweils mit den erfindungsgemäßen Polymeren 1 bzw. 3, 4 und 10 bis 13 wiederholen. Es wird jeweils eine Festiger-Lösung mit guten Eigenschaften erhalten.

Beispiel 13:

| Ethanolische Festiger-Lösung | [%] |
|---|---|
| Polymer aus Beispiel Nr. 2 (fest) | 7,0 |

(fortgesetzt)

| | |
|---|---|
| Ethanol | ad 100 |

weiterer Zusatz: Silikon, Parfüm, Entschäumer ...

Das Beispiel lässt sich jeweils mit den erfindungsgemäßen Polymeren 1 bzw. 3, 4 und 10 bis 13 wiederholen. Es wird jeweils eine Festiger-Lösung mit guten Eigenschaften erhalten.

Beispiel 14:

| Haargel mit Aculyn 28: | [%] |
|---|---|
| Phase 1: | |
| Polymer aus Beispiel Nr. 2 (fest) | 6,00 |
| Aminomethylpropanol (38 %ige Lösung) | 1,0 |
| Wasser, dest. | ad 50 |

weiterer Zusatz: Konservierungsmittel, lösliches ethoxiliertes Silikon, Parfüm ...

Phase 2:

| | |
|---|---|
| Aculyn 28 (1 %ige wässrige Suspension) | 50,00 |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich jeweils mit den erfindungsgemäßen Polymeren 1 bzw. 3, 4 und 10 bis 13 wiederholen. Es wird jeweils ein Haargel mit Aculyn 28 mit guten Eigenschaften erhalten.

Beispiel 15:

| Haargel mit Hydroxyethylcellulose: | [%] |
|---|---|
| Phase 1: | |
| Polymer aus Beispiel Nr. 2 (fest) | 6,00 |
| Wasser, dest. | ad 50 |

weiterer Zusatz: Konservierungsmittel, lösliches ethoxiliertes Silikon, Parfüm

Phase 2:

| | |
|---|---|
| Natrosol HR 250 (5 %ige Lösung) Hydroxyethylcellulose (Fa. Hercules) | 50,00 |

Herstellung:

Die Phasen 1 und 2 werden getrennt eingewogen und homogenisiert. Dann wird Phase 2 langsam in Phase 1

eingerührt. Es bildet sich ein im Wesentlichen klares, stabiles Gel.

Das Beispiel lässt sich jeweils mit den erfindungsgemäßen Polymeren 1 bzw. 3, 4 und 10 bis 13 wiederholen. Es wird jeweils ein Haargel mit Hydroxyethylcellulose mit guten Eigenschaften erhalten.

Beispiel 16:

| Schaumconditioner | [%] |
|---|---|
| Polymer aus Beispiel Nr. 2 (fest) | 0,50 |
| Cremophor®A 25 (Ceteareth 25/Fa. BASF) | 0,20 |
| Comperlan®KD (Coamide DEA/Fa. Henkel) | 0,10 |
| Propan/Butan | 10,00 |

weiterer Zusatz: Parfüm, Konservierungsmittel

Wasser ad 100

Herstellung: Einwiegen und unter Rühren lösen. Abfüllen und Treibgas zusetzen.
Das Beispiel lässt sich jeweils mit den erfindungsgemäßen Polymeren 1 bzw. 3, 4 und 10 bis 13 wiederholen. Es wird jeweils ein Schauniooditioner mit guten Eigenschaften erhalten.

Beispiel 17:

| Conditionershampoo: | [%] |
|---|---|
| A) Texapon®NSO 28 %ig (Natriumlaurethsulfat/Fa. Henkel) | 50,00 |
| Comperian®KS (Coamid DEA/Fa. Henkel) | 1,00 |
| Polymer aus Beispiel Nr. 2 (fest) q. s. Parfümöl | 3,00 |
| | |
| B) Wasser | 44,5 |
| Natriumchlorid q. s. Konservierungsmittel | 1,5 |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich jeweils mit den erfindungsgemäßen Polymeren 1 bzw. 3, 4 und 10 bis 13 wiederholen. Es wird jeweils ein Conditionershampoo mit guten Eigenschaften erhalten.

Beispiel 18:

Standard O/W-Creme:

| Ölphase: | [%] | CTFA-Name |
|---|---|---|
| Cremophor®A6 | 3,5 | Ceteareth-6 (and) Stearyl Alcohol |
| Cremophor®A25 | 3,5 | Ceteareth-25 |
| Glycerinmonostearat s.e. | 2,5 | Glycerylstearat |

(fortgesetzt)

| | | |
|---|---|---|
| Paraffinöl | | 7,5 Paraffin Oil |
| Cetylalkohol | | 2,5 Cetyl Alcohol |
| Luvitol®EHO | | 3,2 Cetearyl Octanoate |
| Vitamin-E-Acetat | 1,0 | Tocopheryl Acetate |
| Nip-Nip | | 0,1 Methyl- and Propyl-4-hydroxybenzoate (7:3) |

Wasserphase:

| | | | |
|---|---|---|---|
| Polymer aus Beispiel Nr. 2 (fest) | | | 0,6 |
| Wasser | | 77,0 | |
| 1,2-Propylenglykol | 1,5 | Propylenglykol | |
| Germall II | | 0,1 | Imidazolidinyl-Harnstoff |

Herstellung:

Die Öl- und Wasserphasen werden getrennt eingewogen und bei einer Temperatur von ca. 80°C homogenisiert. Dann wird die Wasserphase langsam in die Ölphase eingerührt und unter Rühren langsam auf Raumtemperatur abgekühlt.

Das Beispiel lässt sich jeweils mit den erfindungsgemäßen Polymeren 1 bzw. 3, 4 und 10 bis 13 wiederholen. Es wird jeweils eine Standard O/W-Creme mit guten Eigenschaften erhalten.

Beispiel 19: Flüssiges Makeup

| | |
|---|---|
| A | |
| 1,70 | Glycerylstearat |
| 1,70 | Cetylalkohol |
| 1,70 | Ceteareth-6 |
| 1,70 | Ceteareth-25 |
| 5,20 | Capryl/Caprin-Triglycerid |
| 5,20 | Mineralöl |
| B | |
| q.s. | Konservierungsmittel |
| 4,30 | Propylenglykol |
| 2,50 | Polymer aus Beispiel 2 (fest) |
| ad 100 | dest. Wasser |
| C | |
| q.s. | Parfumöl |
| D | |
| 2,00 | Eisenoxid |
| 12,00 | Titandioxid |

Herstellung:

Phase A und Phase B getrennt voneinander auf 80°C erwärmen. Dann Phase B in Phase A mit einem Rührer mischen. Alles auf 40°C abkühlen lassen und Phase C und Phase D zugeben. Wiederholt homogenisieren.

Das Beispiel lässt sich jeweils mit den erfindungsgemäßen Polymeren 1 bzw. 3, 4 und 10 bis 13 wiederholen. Es wird jeweils ein flüssiges Makeup mit guten Eigenschaften erhalten.

Beispiel 20: Ölfreies Makeup

| | |
|---|---|
| A | |
| 0,35 | Veegum |
| 5,00 | Butylenglykol |
| 0,15 | Xanthangummi |
| B | |
| 34,0 | dest. Wasser |
| q.s. | Konservierungsmittel |
| 0,2 | Polysorbate-20 |
| 1,6 | Tetrahydroxypropylethylenediamin |
| C | |
| 1,0 | Siliciumdioxid |
| 2,0 | Nylon-12 |
| 4,15 | Glimmer (mica) |
| 6,0 | Titandioxid |
| 1,85 | Eisenoxid |
| D | |
| 4,0 | Stearinsäure |
| 1,5 | Glycerylstearat |
| 7,0 | Benryllaurat |
| 5,0 | Isoeicosan |
| q.s. | Konservierungsmittel |
| E | |
| 0,5 | Panthenol |
| 0,1 | Imidazolidinyl-Harnstoff |
| 5,0 | Polymer aus Beispiel 2 (fest) |

Herstellung:

Phase A mit Butylenglykol benetzen, in Phase B hineingeben und gut mischen. Phase AB auf 75°C erwärmen. Phase C Einsatzstoffe pulverisieren, in Phase AB hineingeben und gut homogenisieren. Einsatzstoffe von Phase D mischen, auf 80°C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

Das Beispiel lässt sich jeweils mit den erfindungsgemäßen Polymeren 1 bzw. 3, 4 und 10 bis 13 wiederholen. Es wird jeweils ein ölfreies Makeup mit guten Eigenschaften erhalten.

Beispiel 21: Schimmerndes Gel

| | |
|---|---|
| A | |
| 32,6 | dest. Wasser |
| 0,1 | Dinatrium-EDTA |
| 25,0 | Natrosol (4 %ige wässrige Lösung) |
| 0,3 | Konservienrngsmittel |
| B | |
| 0,5 | dest. Wasser |
| 0,5 | Triethanolamin |
| C | |
| 2,0 | Polymer aus Beispiel 2 (fest) |
| ad 100 | dest. Wasser |
| 1,0 | Polyquaternium-46 (20 %ige wässrige Lösung) |
| 5,0 | Eisenoxid |
| D | |
| 15,0 | dest. Wasser |
| 1,0 | D-Panthenol 50 P (Panthenol und Propylenglykol) |

Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

Das Beispiel lässt sich jeweils mit den erfindungsgemäßen Polymeren 1 bzw. 3, 4 und 10 bis 13 wiederholen. Es wird jeweils ein schimmerndes Gel mit guten Eigenschaften erhalten.

Beispiel 22: Sonnenschutz-Gel

| | |
|---|---|
| Phase A | |
| 1,00 | hydriertes Ricinusöl-PEG-40 |
| 8,00 | Octyl Methoxycinnamate (Uvinul®MC 80) |
| 5,00 | Octoaylene (Uvinul®N 539) |
| 0,80 | Octyl Triazone (Uvinul®T 150) |
| 2,00 | Butyl Methoxydibenzoylmethane (Uvinul®BMBM) |
| 2,00 | Tocopherylacetat |
| q.s. | Parfümöl |
| Phase B | |
| 2,50 | Polymer aus Beispiel 2 (fest) |
| ad 100 | dest. Wasser |

(fortgesetzt)

| | |
|---|---|
| 0,30 | Acrylat/$C_{10-30}$Alkylacrylat-Copolymer |
| 0,20 | Carbomer |
| 5,00 | Glycerin |
| 0,20 | Dinatrium-EDTA |
| q.s. | Konservierungsmittel |
| 62,80 | dest. Wasser |
| Phase C | |
| 0,20 | Natriumhydroxid |

Herstellung:

Die Komponenten der Phase A mischen. Phase B quellen lassen und unter Homogenisieren in Phase A einrühren. Mit Phase C neutralisieren und erneut homogenisieren.

Das Beispiel lässt sich jeweils mit den erfindungsgemäßen Polymeren 1 bzw. 3, 4 und 10 bis 13 wiederholen. Es wird jeweils ein Sonnenschutz-Gel mit guten Eigenschaften erhalten.

Beispiel 23: Sonnenschutzemulsion mit $TiO_2$ und $ZnO_2$

| | |
|---|---|
| Phase A | |
| 6,00 | hydriertes Ricinusöl-PEG-7 |
| 2,00 | PEG-45/Dodecyl Glycol Copolymer |
| 3,00 | Isopropylmyristat |
| 8,00 | Jojobaöl (Buxus Chinensis) |
| 4,00 | Octyl Methoxycinnamate (Uvinul®MC 80) |
| 2,00 | 4-Methylbenzylidene Camphor (Uvinul®MBC 95) |
| 3,00 | Titandioxid, Dimethicon |
| 1,00 | Dimethicon |
| 5,00 | Zinkoxid, Dimethicon |
| Phase B | |
| 2,0 | Polymer aus Beispiel 2 (fest) |
| ad 100 | dest. Wasser |
| 0,20 | Dinatrium-EDTA |
| 5,00 | Glycerin |
| q.s. | Konservierungsmittel |
| 50,80 | dest. Wasser |
| Phase C | |
| q.s. | Parfümöl |

Herstellung:

Die Phasen A und B getrennt auf ca. 85°C erwärmen. Phase B in Phase A einrühren und homogenisieren. Abkühlen auf ca. 40°C, Phase C hinzugeben und nochmals kurz homogenisieren.

Das Beispiel lässt sich jeweils mit den erfindungsgemäßen Polymeren 1 bzw. 3, 4 und 10 bis 13 wiederholen. Es wird jeweils eine Sonnenschutzemulsion mit $TiO_2$ und $ZnO_2$ mit guten Eigenschaften erhalten.

Beispiel 24: Sonnenschutz-Lotion

| | |
|---|---|
| Phase A | |
| 6,00 | Octyl Methoxycinnamate (Uvinul®MC 80) |
| 2,50 | 4-Methylbenzylidene Camphor (Uvinul®MBC 95) |
| 1,00 | Octyl Triazone (Uvinul®T 150) |
| 2,00 | Butyl Methoxydibenzoylmethane (Uvinul®BMBM) |
| 2,00 | PVP/Hexadecen-Copolymer |
| 5,00 | PPG-3 Myristyl Ether |
| 0,50 | Dimethicon |
| 0,10 | BHT, Ascorbylpalmitat, Citronensäure, Glycerylstearat Propylenglykol |
| 2,00 | Cetylalkohol |
| 2,00 | Kaliumcetylphosphat |
| Phase B | |
| 0,50 | Polymer aus Beispiel 2 (fest) |
| ad 100 | dest. Wasser |
| 5,00 | Propylenglykol |
| 0,20 | Dinatrium-EDTA |
| q.s. | Konservierungsmittel |
| 63,92 | dest. Wasser |
| Phase C | |
| 5,00 | Mineralöl |
| 0,20 | Carbomer |
| Phase D | |
| 0,08 | Natriumhydroxid |
| Phase E | |
| q.s. | Parfümöl |

Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

Das Beispiel lässt sich jeweils mit den erfindungsgemäßen Polymeren 1 bzw. 3, 4 und 10 bis 13 wiederholen. Es wird jeweils eine Sonnenschutz-Lotion mit guten Eigenschaften erhalten.

Beispiel 25: Abziehbare Gesichtsmaske

| | |
|---|---|
| Phase A | |
| 57,10 | dest. Wasser |

(fortgesetzt)

| | |
|---|---|
| 6,00 | Polyvinylalkohol |
| 5,00 | Propylenglykol |
| Phase B | |
| 20,00 | Alkohol |
| 4,00 | PEG-32 |
| q.s | Parfümöl |
| Phase C | |
| 5,00 | Polyquaternium-44 |
| 0,50 | Polymer aus Beispiel 2 (fest) |
| ad 100 | dest. Wasser |
| 0,20 | Allantoin |

Herstellung:

Phase A auf mind. 90°C erwärmen und rühren bis gelöst. Phase B bei 50°C lösen und in Phase A einrühren. Bei ca. 35°C den Ethanolverlust ausgleichen. Phase C zugeben und unterrühren.

Das Beispiel lässt sich jeweils mit den erfindungsgemäßen Polymeren 1 bzw. 3, 4 und 10 bis 13 wiederholen. Es wird jeweils eine abziehbare Gesichtsmaske mit guten Eigenschaften erhalten.

Beispiel 26: Gesichtsmaske

| | |
|---|---|
| Phase A | |
| 3,00 | Ceteareth-6 |
| 1,50 | Ceteareth-25 |
| 5,00 | Cetearylalkohol |
| 6,00 | Cetearyloctanoat |
| 6,00 | Mineralöl |
| 0,20 | Bisabolol |
| 3,00 | Glycerylstearat |
| Phase B | |
| 2,00 | Propylenglykol |
| 5,00 | Panthenol |
| 2,50 | Polymer aus Beispiel 2 (fest) |
| ad 100 | dest. Wasser |
| q.s. | Konservierungsmittel |
| 53,80 | dest. Wasser |
| Phase C | |
| q.s. | Parfümöl |
| 0,50 | Tocopherylacetat |

Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

Das Beispiel lässt sich jeweils mit den erfindungsgemäßen Polymeren 1 bzw. 3, 4 und 10 bis 13 wiederholen. Es wird jeweils eine Gesichtsmaske mit guten Eigenschaften erhalten.

Beispiel 27: Körperlotion-Schaum

| | |
|---|---|
| Phase A | |
| 1,50 | Ceteareth-25 |
| 1,50 | Ceteareth-6 |
| 4,00 | Cetearylalkohol |
| 10,00 | Cetearyloctanoat |
| 1,00 | Dimethicon |
| Phase B | |
| 0,50 | Polymer aus Beispiel 2 (fest) |
| ad 100 | dest. Wasser |
| 2,00 | Panthenol |
| 2,50 | Propylenglykol |
| q.s. | Konservierungsmittel |
| 74,50 | dest. Wasser |
| Phase C | |
| q.s. | Parfümöl |

Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Abfüllung: 90 % Wirkstoff und 10 % Propan/Butan bei 3,5 bar (20°C).

Das Beispiel lässt sich jeweils mit den erfindungsgemäßen Polymeren 1 bzw. 3, 4 und 10 bis 13 wiederholen. Es wird jeweils ein Körperlotion-Schaum mit guten Eigenschaften erhalten.

Beispiel 28: Gesichtswasser für trockene und empfindliche Haut

| | |
|---|---|
| Phase A | |
| 2,50 | hydriertes Rizinusöl-PEG-40 |
| q.s. | Parfümöl |
| 0,40 | Bisabolol |
| Phase B | |
| 3,00 | Glycerin |
| 1,00 | Hydroxyethylcetyldimoniumphosphat |
| 5,00 | Zaubemuss (Hamamelis Virginiana) Destillat |
| 0,50 | Panthenol |
| 0,1 | Polymer aus Beispiel 2 (fest) |

(fortgesetzt)

| | |
|---|---|
| ad 100 | dest. Wasser |
| q.s. | Konservierungsmittel |
| 87,60 | dest. Wasser |

Herstellung:

Phase A klar lösen. Phase B in Phase A einrühren.

Das Beispiel lässt sich jeweils mit den erfindungsgemäßen Polymeren 1 bzw. 3, 4 und 10 bis 13 wiederholen. Es wird jeweils ein Gesichtswasser für trockene und empfindliche Haut mit guten Eigenschaften erhalten.

Beispiel 29: Gesichtswaschpaste mit Peelingeffekt

| | |
|---|---|
| Phase A | |
| 58,00 | dest. Wasser |
| 2,50 | Polymer aus Beispiel 2 (fest) |
| ad 100 | dest. Wasser |
| 1,50 | Carbomer |
| q.s. | Konservierungsmittel |
| Phase B | |
| q.s. | Parfümöl |
| 7,00 | Potassium Cocoyl Hydrolyzed Protein |
| 4,00 | Cocamidpropylbetain |
| Phase C | |
| 1,50 | Triethanolamin |
| Phase D | |
| 13,00 | Polyethylen (Luwax®A) |

Herstellung:

Phase A quellen lassen. Phase B klar lösen. Phase B in Phase A einrühren. Mit Phase C neutralisieren. Danach Phase **D** einrühren.

Das Beispiel lässt sich jeweils mit den erfindungsgemäßen Polymeren 1 bzw. 3, 4 und 10 bis 13 wiederholen. Es wird jeweils eine Gesichtswaschpaste mit Peelingeffekt mit guten Eigenschaften erhalten.

Beispiel 30: Gesichtsseife

| | |
|---|---|
| Phase A | |
| 25,0 | Kaliumcocoat |
| 20,0 | Disodium Cocoamphodiacetate |
| 2.0 | Lauramide DEA |

(fortgesetzt)

| | |
|---|---|
| 1,0 | Glykolstearat |
| 0,50 | Polymer aus Beispiel 2 (fest) |
| ad 100 | dest. Wasser |
| 50,0 | dest. Wasser |
| q.s. | Citronensäure |
| Phase B | |
| q.s. | Konservierungsmittel |
| q.s. | Parfumöl |

Herstellung:

Phase A unter Rühren auf 70°C erwärmen, bis alles homogen ist, pH-Wert auf 7,0 - 7,5 mit Zitronensäure einstellen, alles auf 50°C abkühlen lassen und Phase B zugeben.

Das Beispiel lässt sich jeweils mit den erfindungsgemäßen Polymeren 1 bzw. 3, 4 und 10 bis 13 wiederholen. Es wird jeweils eine Gesichtsseife mit guten Eigenschaften erhalten.

Beispiel 31: Gesichtsreinigungsmilch, Typ O/W

| | |
|---|---|
| Phase A | |
| 1,50 | Ceteareth-6 |
| 1,50 | Ceteareth-25 |
| 2,00 | Glycerylstearat |
| 2,00 | Cetylalkohol |
| 10,00 | Mineralöl |
| Phase B | |
| 5,00 | Propylenglykol |
| q.s. | Konservierungsmittel |
| 1,0 | Polymer aus Beispiel 2 (fest) |
| ad 100 | dest. Wasser |
| 62,30 | dest. Wasser |
| Phase C | |
| 0,20 | Carbomer |
| 10,00 | Cetearyloctanoat |
| Phase D | |
| 0,40 | Tetrahydroxypropylethylendiamin |
| Phase E | |
| q.s. | Parfümöl |
| 0,10 | Bisabolol |

Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

Das Beispiel lässt sich jeweils mit den erfindungsgemäßen Polymeren 1 bzw. 3, 4 und 10 bis 13 wiederholen. Es wird jeweils eine Gesichtsreinigungsmilch, Typ O/W mit guten Eigenschaften erhalten.

Beispiel 32: Peeling-Creme, Typ O/W

| | |
|---|---|
| Phase A | |
| 3,00 | Ceteareth-6 |
| 1,50 | Ceteareth-25 |
| 3,00 | Glycerylstearat |
| 5,00 | Cetearylalkohol, Sodium Cetearyl Sulfate |
| 6,00 | Cetearyloctanoat |
| 6,00 | Mineralöl |
| 0,20 | Bisabolol |
| Phase B | |
| 2,00 | Propylenglykol |
| 0,10 | Dinatrium-EDTA |
| 0,50 | Polymer aus Beispiel 2 (fest) |
| ad 100 | dest. Wasser |
| q.s. | Konservierungsmittel |
| 59,70 | dest. Wasser |
| Phase C | |
| 0,50 | Tocopherylacetat |
| q.s. | Parfümöl |
| Phase D | |
| 10,00 | Polyethylen |

Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Anschließend Phase D unterrühren.

Das Beispiel lässt sich jeweils mit den erfindungsgemäßen Polymeren 1 bzw. 3, 4 und 10 bis 13 wiederholen. Es wird jeweils eine Peeling-Creme, Typ O/W mit guten Eigenschaften erhalten.

Beispiel 33: Rasierschaum

| | |
|---|---|
| 6,00 | Ceteareth-25 |
| 5,00 | Poloxamer 407 |
| 52,00 | dest. Wasser |
| 1,00 | Triethanolamin |
| 5,00 | Propylenglykol |

(fortgesetzt)

| | |
|---|---|
| 1,00 | Lanolinöl-PEG-75 |
| 1,0 | Polymer aus Beispiel 2 (fest) |
| ad 100 | dest. Wasser |
| q.s. | Konservierungsmittel |
| q.s. | Parfümöl |
| 25,00 | Sodium Laureth Sulfate |

Herstellung:

Alles zusammen wiegen, danach rühren bis gelöst. Abfüllung: 90 Teile Wirksubstanz und 10 Teile Propan/Butan-Mischung 25:75.

Das Beispiel lässt sich jeweils mit den erfindungsgemäßen Polymeren 1 bzw. 3, 4 und 10 bis 13 wiederholen. Es wird jeweils ein Rasierschaum mit guten Eigenschaften erhalten.

Beispiel 34: After Shave Balsam

| | |
|---|---|
| Phase A | |
| 0,25 | Acrylat/$C_{10-30}$ Alkylacrylat-Copolymer |
| 1,50 | Tocopherylacetat |
| 0,20 | Bisabolol |
| 10,00 | Capryl/Caprin-Triglycerid |
| q.s. | Parfümöl |
| 1,00 | hydriertes Rizinusöl-PEG-40 |
| Phase B | |
| 1,00 | Panthenol |
| 15,00 | Alkohol |
| 5,00 | Glycerin |
| 0,05 | Hydroxyethylcellulose |
| 0,50 | Polymer aus Beispiel 2 (fest) |
| ad 100 | dest. Wasser |
| 64,00 | dest. Wasser |
| Phase C | |
| 0,08 | Natriumhydroxid |

Herstellung:

Die Komponenten der Phase A mischen. Phase B unter Homogenisieren in Phase A einrühren, kurz nachhomogenisieren. Mit Phase C neutralisieren und erneut homogenisieren.

Das Beispiel lässt sich jeweils mit den erfindungsgemäßen Polymeren 1 bzw. 3, 4 und 10 bis 13 wiederholen. Es wird jeweils ein After Shave Balsam mit guten Eigenschaften erhalten.

Beispiel 35: Körperpflegecreme

| | |
|---|---|
| Phase A | |
| 2,00 | Ceteareth-6 |
| 2,00 | Ceteareth-25 |
| 2,00 | Cetearylalkohol |
| 3,00 | Glycerylstearat SE |
| 5,00 | Mineralöl |
| 4,00 | Jojobaöl (Buxus Chinensis) |
| 3,00 | Cetearyloctanoat |
| 1,00 | Dimethicon |
| 3,00 | Mineralöl, Lanolinalkohol |
| Phase B | |
| 5,00 | Propylenglykol |
| 0,50 | Veegum |
| 1,00 | Panthenol |
| 1,70 | Polymer aus Beispiel1 (fest) |
| ad 100 | dest. Wasser |
| 6,00 | Polyquaternium-44 (10 %ige wässrige Lösung) |
| q.s. | Konservierungsmittel |
| 54,00 | dest. Wasser |
| Phase C | |
| q.s. | Parfümöl |

Herstellung:

Die Phasen A und B getrennt auf ca. 80°C erwärmen. Phase B homogenisieren. Phase B unter Homogenisieren in Phase A einrühren, kurz nachhomogenisieren. Abkühlen auf ca. 40°C, Phase C hinzugeben und nochmals kurz homogenisieren.

Das Beispiel lässt sich jeweils mit den erfindungsgemäßen Polymeren 1 bzw. 3, 4 und 10 bis 13 wiederholen. Es wird jeweils eine Körperpflegecreme mit guten Eigenschaften erhalten.

Beispiel 36: Zahnpasta

| | |
|---|---|
| Phase A | |
| 34,79 | dest. Wasser |
| 0,50 | Polymer aus Beispiel 2 (fest) |
| ad 100 | dest. Wasser |
| 0,30 | Konservierungsmittel |
| 20,00 | Glycerin |
| 0,76 | Natriummonofluorphosphat |
| Phase B | |
| 1,20 | Natriumcarboxymethylcellulose |

(fortgesetzt)

| | |
|---|---|
| Phase C | |
| 0,80 | Aromaöl |
| 0,06 | Saccharin |
| 0,10 | Konservierungsmittel |
| 0,05 | Bisabolol |
| 1,00 | Panthenol |
| 0,50 | Tocopherylacetat |
| 2,80 | Siliciumdioxid |
| 1,00 | Natriumlaurylsulfat |
| 7,90 | Dicalciumphosphat, wasserfrei |
| 25,29 | Dicalciumphosphat-Dihydrat |
| 0,45 | Titandioxid |

Herstellung:

Phase A lösen. Phase B in Phase A einstreuen und lösen. Phase C zugeben und unter Vakuum bei RT ca. 45 Min. rühren lassen.

Das Beispiel lässt sich jeweils mit den erfindungsgemäßen Polymeren 1 bzw. 3, 4 und 10 bis 13 wiederholen. Es wird jeweils eine Zahnpasta mit guten Eigenschaften erhalten.

Beispiel 37: Mundwasser

| | |
|---|---|
| Phase A | |
| 2,00 | Aromaöl |
| 4,00 | hydriertes Rizinusöl-PEG-40 |
| 1,00 | Bisabolol |
| 30,00 | Alkohol |
| Phase B | |
| 0,20 | Saccharin |
| 5,00 | Glycerin |
| q.s. | Konservierungsmittel |
| 5,00 | Poloxamer 407 |
| 0,50 | Polymer aus Beispiel 2 (fest) |
| ad 100 | dest. Wasser |

Herstellung:

Phase A und Phase B getrennt klar lösen. Phase B in Phase A einrühren.

Das Beispiel lässt sich jeweils mit den erfindungsgemäßen Polymeren 1 bzw. 3, 4 und 10 bis 13 wiederholen. Es wird jeweils ein Mundwasser mit guten Eigenschaften erhalten.

Beispiel 38: Prothesenhaftmittel

| | |
|---|---|
| Phase A | |
| 0,20 | Bisabolol |
| 1,00 | Betacarotin |
| q.s. | Aromaöl |
| 20,00 | Cetearyloctanoat |
| 5,00 | Siliciumdioxid |
| 33,80 | Mineralöl |
| Phase B | |
| 1,0 | Polymer aus Beispiel 2 (fest) |
| ad 100 | dest. Wasser |
| 35,00 | PVP (20 %ige Lösung in Wasser) |

Herstellung:

Phase A gut mischen. Phase B in Phase A einrühren.

Das Beispiel lässt sich jeweils mit den erfindungsgemäßen Polymeren 1 bzw. 3, 4 und 10 bis 13 wiederholen. Es wird jeweils ein Prothesenhaftmittel mit guten Eigenschaften erhalten.

Beispiel 39: Hautpflegecreme, Typ O/W

| | |
|---|---|
| Phase A | |
| 8,00 | Cetearylalkohol |
| 2,00 | Ceteareth-6 |
| 2,00 | Ceteareth-25 |
| 10,00 | Mineralöl |
| 5,00 | Cetearyloctanoat |
| 5,00 | Dimethicon |
| Phase B | |
| 0,50 | Polymer aus Beispiel 2 (fest) |
| ad 100 | dest. Wasser |
| 2,00 | Panthenol, Propylenglykol |
| q.s. | Konservierungsmittel |
| Phase C | |
| q.s. | Parfümöl |

Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

Das Beispiel lässt sich jeweils mit den erfindungsgemäßen Polymeren 1 bzw. 3, 4 und 10 bis 13 wiederholen. Es wird jeweils eine Hautpflegecreme, Typ O/W mit guten Eigenschaften erhalten.

Beispiel 40: Hautpflegecreme, Typ W/O

| | |
|---|---|
| Phase A | |
| 6,00 | hydriertes Rizinusöl-PEG-7 |
| 8,00 | Cetearyloctanoat |
| 5,00 | Isopropylmyristat |
| 15,00 | Mineralöl |
| 2,00 | PEG-45/Dodecyl Glycol Copolymer |
| 0,50 | Magnesiumstearat |
| 0,50 | Aluminumstearat |
| Phase B | |
| 3,00 | Glycerin |
| 0,60 | Polymer aus Beispiel 2 (fest) |
| ad 100 | dest. Wasser |
| 0,70 | Magnesiumsulfat |
| 2,00 | Panthenol |
| q.s. | Konservierungsmittel |
| Phase C | |
| 1,00 | Tocopherol |
| 5,00 | Tocopherylacetat |
| q.s. | Parfümöl |

Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren.

Das Beispiel lässt sich jeweils mit den erfindungsgemäßen Polymeren 1 bzw. 3, 4 und 10 bis 13 wiederholen. Es wird jeweils eine Hautpflegecreme, Typ W/O mit guten Eigenschaften erhalten.

Beispiel 41: Lippenpflegecreme

| | |
|---|---|
| Phase A | |
| 10,00 | Cetearyloctanoat |
| 5,00 | Polybuten |
| Phase B | |
| 0,10 | Carbomer |
| Phase C | |
| 2,00 | Ceteareth-6 |
| 2,00 | Ceteareth-25 |
| 2,00 | Glycerylstearat |
| 2,00 | Cetylalkohol |
| 1,00 | Dimethicon |
| 1,00 | Benzophenone-3 |

(fortgesetzt)

| | |
|---|---|
| 0,20 | Bisabolol |
| 6,00 | Mineralöl |
| Phase D | |
| 1,50 | Polymer aus Beispiel 2 (fest) |
| ad 100 | dest. Wasser |
| 3,00 | Panthenol |
| 3,00 | Propylenglykol |
| q.s. | Konservierungsmittel |
| Phase E | |
| 0,10 | Triethanolamin |
| Phase F | |
| 0,50 | Tocopherylacetat |
| 0,10 | Tocopherol |
| q.s. | Parfümöl |

Herstellung:

Phase A klar lösen. Phase B dazugeben und homogenisieren. Phase C addieren und schmelzen bei 80°C. Phase D auf 80°C erwärmen. Phase D zu Phase ABC geben und homogenisieren. Abkühlen auf ca. 40°C, Phase E und Phase F zugeben, nochmals homogenisieren.

Das Beispiel lässt sich jeweils mit den erfindungsgemäßen Polymeren 1 bzw. 3, 4 und 10 bis 13 wiederholen. Es wird jeweils eine Lippenpflegecreme mit guten Eigenschaften erhalten.

Beispiel 42: Duschgel

| | |
|---|---|
| 50,00 | Sodium Laureth Sulfate, Magnesium Laureth Sulfate, Sodium Laureth-8 Sulfate, Magnesium Laureth-8 |
| 1,00 | Cocoamide DEA |
| 0,8 | Polymer aus Beispiel 2 (fest) |
| ad 100 | dest. Wasser |
| 2,00 | Sodium Laureth Sulfate, Glycol Distearate, Cocamide MEA, Laureth-10 |
| q.s. | Konservierungsmittel |
| q.s. | Parfümöl |
| 2,00 | Natriumchlorid |

Herstellung:

Alle Komponenten gemeinsam einwiegen und bis zur Lösung rühren.

Das Beispiel lässt sich jeweils mit den erfindungsgemäßen Polymeren 1 bzw. 3, 4 und 10 bis 13 wiederholen. Es wird jeweils ein Duschgel mit guten Eigenschaften erhalten.

Beispiel 43: Duschgel

| | |
|---|---|
| 30,00 | Sodium Laureth Sulfate |
| 6,00 | Sodium Cocoamphodiacetate |
| 6,00 | Cocamidopropylbetain |
| 3,00 | Sodium Laureth Sulfate, Glycol Distearate, Cocamide MEA, Laureth-10 |
| 7,70 | Polyquaternium-44 |
| 0,2 | Polymer aus Beispiel 2 (fest) |
| ad 100 | dest. Wasser |
| 1,00 | Panthenol |
| q.s. | Konservienrngsmittel |
| q.s. | Parfümöl |
| q.s. | Citronensäure |
| 0,50 | Natriumchlorid |

Herstellung:

Die Komponenten der Phase A einwiegen und lösen. Den pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Duschgel mit guten Eigenschaften erhalten.

Beispiel 44: Klares Duschgel

| | |
|---|---|
| 40,00 | Sodium Laureth Sulfate |
| 5,00 | Decylglukosid |
| 5,00 | Cocamidopropylbetain |
| 0,50 | Polyquaternium-10 |
| 2,00 | Polymer aus Beispiel 2 (fest) |
| ad 100 | dest. Wasser |
| 1,00 | Panthenol |
| q.s. | Parfümöl |
| q.s. | Konservierungsmittel |
| q.s. | Citronensäure |
| 2,00 | Natriumchlorid |

Herstellung:

Die Komponenten der Phase A einwiegen und klar lösen.

Das Beispiel lässt sich jeweils mit den erfindungsgemäßen Polymeren 1 bzw. 3, 4 und 10 bis 13 wiederholen. Es wird jeweils ein klares Duschgel mit guten Eigenschaften erhalten.

Beispiel 45: Duschbad

| | |
|---|---|
| A | |
| 40,00 | Sodium Laureth Sulfate |
| 5,00 | Sodium $C_{12-15}$ Pareth-15 Sulfonate |
| 5,00 | Decylglukosid |
| q.s. | Parfümöl |
| 0,10 | Phytantriol |
| B | |
| 0,1 | Guarhydroxypropyltrimoniumchlorid |
| 2,00 | Polymer aus Beispiel 2 (fest) |
| ad 100 | dest. Wasser |
| 1,00 | Panthenol |
| q.s. | Konservierungsmittel |
| 1,00 | Laureth-3 |
| q.s. | Citronensäure |
| 2,00 | Natriumchlorid |

Herstellung:

Die Komponenten der Phase A mischen. Die Komponenten der Phase B nacheinander zugeben und mischen. Den pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Duschbad mit guten Eigenschaften erhalten.

Beispiel 46: Flüssigseife

| | |
|---|---|
| A | |
| 43,26 | dest. Wasser |
| 0,34 | Aminomethylpropanol |
| 3,40 | Poly(Ethylacrylat/Methacrylsäure) (Luviflex®Soft, Fa. BASF) |
| B | |
| 40,00 | Sodium Laureth Sulfate |
| 10,00 | Cocamidopropylbetain |
| 0,2 | Polymer aus Beispiel 2 (fest) |
| ad 100 | dest. Wasser |
| q.s. | Parfümöl |
| q.s. | Konservierungsmittel |
| 2,00 | Natriumchlorid |

Herstellung:

Die Komponenten der Phase A einwiegen und klar lösen. Die Komponenten der Phase B nacheinander zugeben und mischen.

Das Beispiel lässt sich jeweils mit den erfindungsgemäßen Polymeren 1 bzw. 3, 4 und 10 bis 13 wiederholen. Es wird jeweils eine Flüssigseife mit guten Eigenschaften erhalten.

Beispiel 47: Flüssiges Fußbad

| | |
|---|---|
| A | |
| 1,00 | Nonoxynol-14 |
| 0,10 | Bisabolol |
| 1,00 | Pinienöl (Pinus Sylvestris) |
| B | |
| 5,00 | PEG-8 |
| 1,50 | Polymer aus Beispiel 2 (fest) |
| ad 100 | dest. Wasser |
| 0,50 | Triclosan |
| 30,00 | Sodium Laureth Sulfate |
| 3,00 | Polyquaternium-16 |
| q.s. | C. I. 19 140 + C. I. 42 051 |

Herstellung:

Phase A solubilisieren. Phase B mischen.

Das Beispiel lässt sich jeweils mit den erfindungsgemäßen Polymeren 1 bzw. 3, 4 und 10 bis 13 wiederholen. Es wird jeweils ein flüssiges Fußbad mit guten Eigenschaften erhalten.

Beispiel 48: Erfrischungsgel

| | |
|---|---|
| A | |
| 0,60 | Carbomer |
| 45,40 | dest. Wasser |
| B | |
| 0,50 | Bisabolol |
| 0,50 | Famesol |
| q.s. | Parfümöl |
| 5,00 | PEG-40 Hydrogenated Castor Oil |
| 0,50 | Polymer aus Beispiel 2 (fest) |
| ad 100 | dest. Wasser |
| 1,00 | Tetrahydroxypropylethylendiamin |
| 1,50 | Menthol |
| 43,00 | Alkohol |
| q.s. | C. I. 74 180, Direct Blue 86 |

Herstellung:

Phase A quellen lassen. Phase B lösen. Phase B in Phase A einrühren.

Das Beispiel lässt sich jeweils mit den erfindungsgemäßen Polymeren 1 bzw. 3, 4 und 10 bis 13 wiederholen.

Es wird jeweils ein Erfrischungsgel mit guten Eigenschaften erhalten.

Beispiel 49: Roll-on Antiperspirant

| | |
|---|---|
| A | |
| 0,40 | Hydroxyethylcellulose |
| 50,00 | dest. Wasser |
| B | |
| 25,00 | Alkohol |
| 0,10 | Bisabolol |
| 0,30 | Farnesol |
| 2,00 | PEG-40 Hydrogenated Castor Oil |
| q.s. | Parfümöl |
| C | |
| 5,00 | Aluminumchlorhydrat |
| 3,00 | Propylenglykol |
| 3,00 | Dimethicon-Copolyol |
| 3,00 | Polyquaternium-16 |
| 1,50 | Polymer aus Beispiel 2 (fest) |
| ad 100 | dest. Wasser |

Herstellung:

Phase A quellen lassen. Phase B und C getrennt lösen. Phase A und B in Phase C einrühren.

Das Beispiel lässt sich jeweils mit den erfindungsgemäßen Polymeren 1 bzw. 3, 4 und 10 bis 13 wiederholen. Es wird jeweils ein Roll-on Antiperspirant mit guten Eigenschaften erhalten.

Beispiel 50: Transparenter Deostift

| | |
|---|---|
| 5,00 | Natriumstearat |
| 0,50 | Triclosan |
| 3,00 | Ceteareth-25 |
| 20,00 | Glycerin |
| 0,50 | Polymer aus Beispiel 2 (fest) |
| ad 100 | dest. Wasser |
| q.s. | Parfümöl |
| 60,00 | Propylenglykol |
| 0,20 | Bisabolol |

Herstellung:

Phase A zusammenwiegen, schmelzen und homogenisieren. Anschließend in die Form gießen.

Das Beispiel lässt sich jeweils mit den erfindungsgemäßen Polymeren 1 bzw. 3, 4 und 10 bis 13 wiederholen. Es wird jeweils ein transparenter Deostift mit guten Eigenschaften erhalten.

Beispiel 51: Wasserlösliches Badeöl

| | |
|---|---|
| 15,00 | Cetearyloctanoat |
| 15,00 | Capryl/Caprin-Triglycerid |
| 1,00 | Panthenol, Propylenglykol |
| 0,10 | Bisabolol |
| 2,00 | Tocopherylacetat |
| 2,00 | Retinylpalmitat |
| 0,10 | Tocopherol |
| 37,00 | PEG-7-Glyceryl-Cocoate |
| 0,4 | Polymer aus Beispiel 2 (fest) |
| ad 100 | dest. Wasser |
| q.s. | Parfümöl |
| 23,60 | PEG-40 Hydrogenated Castor Oil |

Herstellung:

Mischen und rühren bis alles klar gelöst ist.

Das Beispiel lässt sich jeweils mit den erfindungsgemäßen Polymeren 1 bzw. 3, 4 und 10 bis 13 wiederholen. Es wird jeweils ein wasserlösliches Badeöl mit guten Eigenschaften erhalten.

Beispiel 52: Tagespflege-Aerosol

| | |
|---|---|
| A | |
| 4,00 | Ethylhexyl Methoxycinnamate |
| 1,50 | Octocrylen |
| 9,00 | Capryl/Caprin-Triglycerid |
| 5,00 | Simmondsia Chinensis (Jojoba) Seed Oil |
| 1,50 | Cyclomethicon |
| 3,00 | Hydrogenated Coco-Glycerides |
| 1,00 | PVP/Hexadecen-Copolymer |
| 1,00 | Ceteareth-6, Stearylalkohol |
| B | |
| 5,00 | Zinkoxid |
| C | |
| 2,00 | Ceteareth-25 |
| 1,20 | Panthenol |
| 0,20 | Sodium Ascorbyl Phosphate |
| 0,30 | Imidazolidinyl-Harnstoff |
| 0,10 | Disodium EDTA |
| 1,50 | Polymer aus Beispiel 2 (fest) |
| ad 100 | dest. Wasser |

(fortgesetzt)

| | |
|---|---|
| D | |
| 0,50 | Tocopherylacetat |
| 0,20 | Bisabolol |
| 0,33 | Capryl/Caprin-Triglycerid, Retinol |
| q.s. | Parfümöl |

Herstellung:

Phase A auf 80°C erwärmen. Phase A klar lösen. Phase B einarbeiten und homogenisieren. Phase C zugeben, erhitzen auf 80°C, aufschmelzen und homogenisieren. Unter Rühren auf ca. 40°C abkühlen, Phase D hinzugeben und kurz homogenisieren. 90 % Wirkstofflösung: 10 % Propan/Butan mit 3,5 bar (20°C) abfüllen.

Das Beispiel lässt sich jeweils mit den erfindungsgemäßen Polymeren 1 bzw. 3, 4 und 10 bis 13 wiederholen. Es wird jeweils ein Tagespflege-Aerosol mit guten Eigenschaften erhalten.

Beispiel 53: Feuchtigkeitscreme

| | |
|---|---|
| A | |
| 3,00 | Vitis Vinifera (Grape) Seed Oil |
| 1,00 | Cyclopentasiloxan, Cyclohexasiloxan |
| 1,50 | Cyclomethicon |
| 2,00 | Soybean (Glycine Soja) Oil |
| 2,00 | Ethylhexyl Methoxycinnamate |
| 1,00 | Uvinul®A Plus |
| 1,00 | Hydrogenated Lecithin |
| 1,00 | Cholesterol |
| 2,00 | PEG-40 Hydrogenated Castor Oil |
| 5,00 | Cetearyloctanoat |
| 5,00 | Capryl/Caprin-Triglycerid |
| B | |
| 3,00 | Capryl/Caprin-Triglycerid, Acrylat-Copolymer |
| C | |
| 2,50 | Polymer aus Beispiel 2 (fest) |
| ad 100 | dest. Wasser |
| 0,50 | Cocotrimoniummethsulfat |
| 2,00 | Panthenol, Propylenglykol |
| 3,00 | Glycerin |
| 0,10 | Dinatrium-EDTA |
| D | |
| 0,30 | Parfümöl |
| 0,30 | DMDM Hydantoin |
| 1,00 | Tocopherylacetat |
| 2,00 | Tocopherol |

Herstellung:

Phase A auf 80°C erwärmen. Phase B in Phase A einrühren. Phase C auf ca. 80°C erwärmen und unter Homogenisieren in Phase A+B einrühren. Unter Rühren auf ca. 40°C abkühlen, Phase D hinzugeben und kurz homogenisieren.

Das Beispiel lässt sich jeweils mit den erfindungsgemäßen Polymeren 1 bzw. 3, 4 und 10 bis 13 wiederholen. Es wird jeweils eine Feuchtigkeitscreme mit guten Eigenschaften erhalten.

Beispiel 54: Aerosol-Haarschaum

| | |
|---|---|
| A | |
| 2,00 | Cocotrimoniummethsulfat |
| 0,20 | Parfümöl |
| B | |
| 1,60 | Polymer aus Beispiel 2 (fest) |
| ad 100 | dest. Wasser |
| 0,50 | Poly(Ethylacrylat/Methacrylsäure) (Luviflex®Soft) |
| 0,10 | Aminomethylpropanol |
| 0,20 | Ceteareth-25 |
| 0,20 | Trimethylsilylamodimethicon, Trideceth-10, Cetrimonium Chloride |
| 0,10 | PEG-25 PABA |
| 0,20 | Hydroxyethylcellulose |
| 0,20 | PEG-8 |
| 0,20 | Panthenol |
| 15,00 | Alkohol |
| C | |
| 10,00 | Propan/Butan 3,5 bar (20°C) |

Herstellung:

Phasen A und B mischen und mit Treibgas abfüllen.

Das Beispiel lässt sich jeweils mit den erfindungsgemäßen Polymeren 1 bzw. 3, 4 und 10 bis 13 wiederholen. Es wird jeweils ein Aerosol-Haarschaum mit guten Eigenschaften erhalten.

Beispiel 55: Pumpmousse

| | |
|---|---|
| A | |
| 2,00 | Cocotrimoniummethosulfat |
| q.s. | Parfümöl |
| C | |
| 7,00 | Polyquaternium-46 (10 %ige wässrige Lösung) |

(fortgesetzt)

| | |
|---|---|
| 2,50 | Polymer aus Beispiel 2 (fest) |
| ad 100 | dest. Wasser |
| 0,50 | PEG-8 |
| 1,00 | Panthenol |
| q.s. | Konservierungsmittel |
| 0,20 | PEG-25 PABA (ethoxylierte p-Aminobenzoesäure) |

Herstellung:

Die Komponenten der Phase A mischen. Die Komponenten der Phase B nacheinander zugeben und klar lösen.

Das Beispiel lässt sich jeweils mit den erfindungsgemäßen Polymeren 1 bzw. 3, 4 und 10 bis 13 wiederholen. Es wird jeweils ein Pumpmousse mit guten Eigenschaften erhalten.

Beispiel 56: Aerosolschaum

| | |
|---|---|
| 3,0 | Polymer aus Beispiel 2 (fest) |
| ad 100 | dest. Wasser |
| 5,00 | PVP/VA-Copolymer (40 %ige wässrige Lösung) |
| 0,50 | Hydroxyethylcetyldimoniumphosphat |
| 0,20 | Ceteareth-25 |
| 0,40 | Parfümöl PC 910.781/Cremophor |
| q.s. | Konservierungsmittel |
| 10,00 | Propan/Butan 3,5 bar (20°C) |

Herstellung:

Alles zusammen wiegen, rühren bis gelöst, dann abfüllen.

Das Beispiel lässt sich jeweils mit den erfindungsgemäßen Polymeren 1 bzw. 3, 4 und 10 bis 13 wiederholen. Es wird jeweils ein Aerosolschaum mit guten Eigenschaften erhalten.

Beispiel 57: Farbstyling-Mousse

| | |
|---|---|
| A | |
| 2,00 | Cocotrimoniummethosulfat |
| q.s. | Parfümöl |
| B | |
| 6,50 | Polymer aus Beispiel 2 (fest) |
| ad 100 | dest. Wasser |
| 0,50 | Acrylatcopolymer (Luvimer®100 P, Fa. BASF) |
| 0,10 | Aminomethylpropanol |
| 0,20 | Ceteareth-25 |
| 0,20 | Panthenol |

(fortgesetzt)

| | |
|---|---|
| 0,20 | Hydroxyethylcellulose |
| 10,00 | Alkohol |
| 0,08 | C.I. 12245, Basic Red 76 |
| 0,05 | C.I. 42510, Basic. Violet 14 |
| C | |
| 10,00 | Propan/Butan 3,5 bar (20°C) |

Herstellung:

Alles zusammen wiegen, rühren bis gelöst, dann abfüllen. Nur für dunkelblondes und braunes Haar geeignet!

Das Beispiel lässt sich jeweils mit den erfindungsgemäßen Polymeren 1 bzw. 3, 4 und 10 bis 13 wiederholen. Es wird jeweils ein Farbstyling-Mousse mit guten Eigenschaften erhalten.

Beispiel 58: Pumphaarschaum

| | |
|---|---|
| A | |
| 1,50 | Cocotrimoniummethosulfat |
| q.s. | Parfümöl |
| B | |
| 2,00 | Polymer aus Beispiel 2 (fest) |
| ad 100 | dest. Wasser |
| C | |
| 0,46 | Aminomethylpropanol |
| 4,00 | PEG/PPG-25/25 Dimethicon/Acrylat-Copolymer |
| q.s. | Konservierungsmittel |

Herstellung:

Phase A mischen. Phase B in Phase A einrühren. Phase C zugeben und rühren bis gelöst.

Das Beispiel lässt sich jeweils mit den erfindungsgemäßen Polymeren 1 bzw. 3, 4 und 10 bis 13 wiederholen. Es wird jeweils ein Pumphaarschaum mit guten Eigenschaften erhalten.

Beispiel 59: Aquawax

| | |
|---|---|
| 10 | Polymer aus Beispiel 2 (fest) |
| ad 100 | dest. Wasser |
| q.s. | Parfümöl |
| q.s. | hydriertes Rizinusöl-PEG-40 |
| 0,10 | Diethylphthalat |
| 0,10 | Cetearylethylhexanoat |

(fortgesetzt)

| | |
|---|---|
| 0,10 | PEG-7 Glyceryl Cocoate |
| 0,10 | Konservierungsmittel |
| 2,00 | Capryl/Caprin-Triglycerid, Acrylat-Copolymer |

Herstellung:

Alles mischen und homogenisierten. 15 Minuten nachrühren.

Das Beispiel lässt sich jeweils mit den erfindungsgemäßen Polymeren 1 bzw. 3, 4 und 10 bis 13 wiederholen. Es wird jeweils ein Aquawax mit guten Eigenschaften erhalten.

Beispiel 60: Rinse-off Conditioner und Repair Treatment

| | |
|---|---|
| A | |
| 0,20 | Cetearyloctanoat |
| 0,10 | Phytantriol |
| 2,00 | hydriertes Rizinusöl-PEG-40 |
| B | |
| q.s. | Parfümöl |
| 2,00 | Cocotrimoniummethosulfat |
| C | |
| ad 100 | dest. Wasser |
| D | |
| 2,00 | Polyquaternium-16 (20 %ige wässrige Lösung) |
| 1,0 | Polymer aus Beispiel 2 (fest) |
| 1,00 | Dimethicon-Copolyol |
| q.s. | Konservierungsmittel |
| 10,00 | Alkohol |
| q.s. | Citronensäure |

Herstellung:

Die Phasen A und B getrennt mischen. Phase C in Phase B einrühren.

Das Beispiel lässt sich jeweils mit den erfindungsgemäßen Polymeren 1 bzw. 3, 4 und 10 bis 13 wiederholen. Es wird jeweils ein Rinse-off Conditioner und Repair Treatment mit guten Eigenschaften erhalten.

Beispiel 61: Haarkur

| | |
|---|---|
| A | |
| 2,00 | Ceteareth-6, Stearylalkohol |
| 1,00 | Ceteareth-25 |

(fortgesetzt)

| | |
|---|---|
| 6,00 | Cetearylalkohol |
| 6,00 | Cetearyloctanoat |
| 0,30 | Phytantriol |
| | |
| B | |
| 1,0 | Polymer aus Beispiel 2 (fest) |
| ad 100 | dest. Wasser |
| 0,70 | Guar Hydroxypropytrimonoium Chloride |
| 5,00 | Propylenglykol |
| 2,00 | Panthenol |
| 0,30 | Imidazolidinyl-Harnstoff |
| | |
| C | |
| 2,00 | Cosi Silk Soluble |
| 0,20 | Parfum |
| 0,50 | Phenoxyethanol |

Herstellung:

Die Phasen A und B getrennt auf ca. 80°C erwärmen. Phase B homogenisieren.

Das Beispiel lässt sich jeweils mit den erfindungsgemäßen Polymeren 1 bzw. 3, 4 und 10 bis 13 wiederholen. Es wird jeweils eine Haarkur mit guten Eigenschaften erhalten.

Beispiel 62: Haar-Cocktail

| | |
|---|---|
| A | |
| 0,40 | Aaylate/$C_{10-30}$ Alkylacrylat-Crosspolymer |
| 2,00 | Dimethicon |
| 3,00 | Cyclomethicon, Dimethiconol |
| 2,00 | Phenyltrimethicon |
| 2,00 | Amodimethicone, Cetrimonium Chloride, Trideceth-10 |
| 0,50 | Dimethicon-Copolyol |
| 1,00 | Macadamia-Nussöl (Ternifolia) |
| 0,50 | Tocopherylacetat |
| 1,00 | PEG-40 Hydrogenated Castor Oil |
| q.s. | Parfümöl |
| | |
| B | |
| 0,3 | Polymer aus Beispiel 2 (fest) |
| ad 100 | dest. Wasser |
| 0,46 | Aminomethylpropanol |
| 4,00 | PEG/PPG-25/25 Dimethicon/Acrylat-Copolymer |

Herstellung:

:

Die Komponenten der Phase A mischen. Phase B lösen. Phase B unter Homogenisieren in Phase A einrühren.

Das Beispiel lässt sich jeweils mit den erfindungsgemäßen Polymeren 1 bzw. 3, 4 und 10 bis 13 wiederholen. Es wird jeweils ein Haar-Cocktail mit guten Eigenschaften erhalten.

Beispiel 63: Dauerwelle

Well-Lösung

| | |
|---|---|
| A | |
| 0,20 | Cocamidopropylbetain |
| 0,20 | Polysorbate 20 |
| 1,55 | Polymer aus Beispiel 2 (fest) |
| ad 100 | dest. Wasser |
| 0,20 | Dinatrium-EDTA |
| 0,20 | Hydroxyethylcellulose |
| B | |
| 8,00 | Thioglykolsäure |
| C | |
| 11,00 | Ammoniumhydroxid |
| D | |
| 5,00 | Ammoniumcarbonat |

Herstellung:

Die Komponenten der Phase A einwiegen und klar lösen. Phase B in Phase A einrühren.

Das Beispiel lässt sich jeweils mit den erfindungsgemäßen Polymeren 1 bzw. 3, 4 und 10 bis 13 wiederholen. Es wird jeweils eine Well-Lösung mit guten Eigenschaften erhalten.

Beispiel 64: Fixierung:

| | |
|---|---|
| A | |
| 1,00 | PEG-40 Hydrogenated Castor Oil |
| 0,20 | Parfümöl |
| ad 100 | dest. Wasser |
| B | |
| 0,20 | Cocamidopropylbetain |
| 0,20 | Ceteareth-25 |
| 2,5 | Polymer aus Beispiel 2 (fest) |
| q.s. | Konservierungsmittel |
| C | |
| 2,30 | Wasserstoffperoxid |

(fortgesetzt)

| | |
|---|---|
| D | |
| q.s. | Phosphorsäure |

Herstellung:

Phase A solubilisieren. Die Komponenten der Phase B nacheinander zugeben und klar lösen.

Das Beispiel lässt sich jeweils mit den erfindungsgemäßen Polymeren 1 bzw. 3, 4 und 10 bis 13 wiederholen. Es wird jeweils eine Fixierung mit guten Eigenschaften erhalten.

Beispiel 65: dunkelbraune Permanenthaarfarbe (Oxidationshaarfarbe)

| | |
|---|---|
| A | |
| 0,20 | Natriumsulfit |
| 0,05 | Dinatrium-EDTA |
| 0,20 | p-Phenylendiamin |
| 0,30 | Resorcinol |
| 0,20 | 4-Amino-2-hydroxytoluol |
| 0,10 | m-Aminophenol |
| 1,50 | Oleylalkohol |
| 4,50 | Propylenglykol |
| 2,30 | Sodium $C_{12-15}$ Pareth-15 Sulfonate |
| 20,00 | Ölsäure |
| ad 100 | dest. Wasser |
| B | |
| 1,0 | Polymer aus Beispiel 2 (fest) |
| 13,70 | Ammoniumhydroxid |
| 6,00 | i-Propanol |
| q.s. | Parfum |

Herstellung:

Phase A solubilisieren. Die Komponenten der Phase B nacheinander zugeben und mischen.

Das Beispiel lässt sich jeweils mit den erfindungsgemäßen Polymeren 1 bzw. 3, 4 und 10 bis 13 wiederholen. Es wird jeweils eine dunkelbraune Permanenthaarfarbe (Oxidationshaarfarbe) mit guten Eigenschaften erhalten.

Beispiel 66: Entwickleremulsion (pH 3 -4)

| | |
|---|---|
| 3,00 | Hexadecylalkohol |
| 1,0 | Polymer aus Beispiel 2 (fest) |
| ad 100 | dest. Wasser |
| 1,00 | Ceteareth-20 |
| 1,00 | Sodium $C_{12-15}$ Pareth-15 Sulfonate |

(fortgesetzt)

| | |
|---|---|
| 6,00 | Wasserstoffperoxid |
| 0,50 | Phosphorsäure |
| 0,01 | Acetanilid |

Herstellung:

Die Komponenten nacheinander zugeben und mischen.

Das Beispiel lässt sich jeweils mit den erfindungsgemäßen Polymeren 1 bzw. 3, 4 und 10 bis 13 wiederholen. Es wird jeweils eine Entwickleremulsion (pH 3 - 4) mit guten Eigenschaften erhalten.

Beispiel 67: hellbraune Semi-Permanenthaarfarbe

| | |
|---|---|
| 10,00 | Cocodiethanolamid |
| 4,00 | Natriumdodecylbenzylsulfonat, 50 %ig |
| 1,0 | Polymer aus Beispiel 2 (fest) |
| ad 100 | dest. Wasser |
| 6,00 | $C_{9-11}$ Pareth-3 |
| 2,50 | Natriumlaurylsulfat |
| 0,40 | 2-Nitro-p-phenylendiamin |
| 0,20 | HC Red No.3 |
| 0,20 | HC Yellow No.2 |

Herstellung:

Die Komponenten nacheinander zugeben und mischen.

Das Beispiel lässt sich jeweils mit den erfindungsgemäßen Polymeren 1 bzw. 3, 4 und 10 bis 13 wiederholen. Es wird jeweils eine hellbraune Semi-Permanenthaarfarbe mit guten Eigenschaften erhalten.

Beispiel 68: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glycol Distearate, Cocamide MEA, Laureth-10 |
| 1,0 | Polymer aus Beispiel 2 (fest) |
| 2,00 | Dimethicon |
| q.s. | Parfum |
| q.s. | Konservierungsstoff |
| q.s. | Citronensäure |
| 1,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

Beispiel 69: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glykol Distearate, Cocamide MEA, Laureth-10 |
| 1,0 | Polymer aus Beispiel 2 (fest) |
| 2,00 | Amodimethicon |
| q.s. | Parfum |
| q.s. | Konservierungsstoff |
| q.s. | Citronensäure |
| 1,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

Beispiel 70: Shampoo

| | |
|---|---|
| 40,00 | Sodium Laureth Sulfate |
| 10,00 | Cocamidopropylbetain |
| 3,00 | Sodium Laureth Sulfate, Glykol Distearate, Cocamide MEA, Laureth-10 |
| 1,0 | Polymer aus Beispiel 2 (fest) |
| 2,00 | Dow Coming 3052 |
| q.s. | Parfum |
| q.s. | Konservierungsstoff |
| q.s. | Citronensäure |
| 2,00 | Cocamido DEA |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

Beispiel 71: Antischuppen-Shampoo

| | |
|---|---|
| 40,00 | Sodium Laureth Sulfate |
| 10,00 | Cocamidopropylbetain |
| 10,00 | Disodium Laureth Sulfosuccinate |
| 2,50 | Sodium Laureth Sulfate, Glycol Distearate, Cocamide MEA, Laureth-10 |
| 1,0 | Polymer aus Beispiel 2 (fest) |
| 0,50 | Climbazol |
| q.s. | Parfum |
| q.s. | Konservierungsstoff |
| 0,50 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Antischuppen-Shampoo mit guten Eigenschaften erhalten.

Beispiel 72: Shampoo

| | |
|---|---|
| 25,00 | Sodium Laureth Sulfate |
| 5,00 | Cocamidopropylbetain |
| 2,50 | Sodium Laureth Sulfate, Glycol Distearate, Cocamide MEA, Laureth-10 |
| 1,0 | Polymer aus Beispiel 2 (fest) |
| q.s. | Parfum |
| q.s. | Konservierungsstoff |
| 2,00 | Cocamido DEA |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

Beispiel 73: Shampoo

| | |
|---|---|
| 20,00 | Ammonium Laureth Sulfate |
| 15,00 | Ammonium Lauryl Sulfate |

(fortgesetzt)

| | |
|---|---|
| 5,00 | Cocamidopropylbetain |
| 2,50 | Sodium Laureth Sulfate, Glycol Distearate,Cocamide MEA, Laureth-10 |
| 1,0 | Polymer aus Beispiel 2 (fest) |
| q.s. | Parfum |
| q.s. | Konservierungsstoff |
| 0,50 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

Beispiel 74: Klares Duschgel

| | |
|---|---|
| 40,00 | Sodium Laureth Sulfate |
| 5,00 | Decylglukosid |
| 5,00 | Cocamidopropylbetain |
| 1,0 | Polymer aus Beispiel 2 (fest) |
| 1,00 | Panthenol |
| q.s. | Parfum |
| q.s. | Konservierungsstoff |
| q.s. | Citronensäure |
| 2,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein klares Duschgel mit guten Eigenschaften erhalten.

Beispiel 75: Shampoo

| | |
|---|---|
| 12,00 | Sodium Laureth Sulfate |
| 1,50 | Decylglukosid |
| 2,50 | Cocamidopropylbetain |
| 5,00 | Coco-Glucoside Glyceryl Oleate |
| 2,00 | Sodium Laureth Sulfate, Glycol Distearate, Cocamide MEA,Laureth-10 |
| 1,0 | Polymer aus Beispiel 2 (fest) |
| q.s. | Konservierungsstoff |
| q.s. | Sunset Yellow C.I. 15 985 |

(fortgesetzt)

| | |
|---|---|
| q.s. | Parfum |
| 1,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

Beispiel 76: Shampoo

| | |
|---|---|
| A | |
| 40,00 | Sodium Laureth Sulfate |
| 5,00 | Sodium $C_{12-15}$ Pareth-15 Sulfonate |
| 5,00 | Decylglukosid |
| q.s. | Parfum |
| 0,10 | Phytantriol |
| B | |
| 1,0 | Polymer aus Beispiel 2 (fest) |
| ad 100 | dest. Wasser |
| 1,00 | Panthenol |
| q.s. | Konservierungsstoff |
| 1,00 | Laureth-3 |
| q.s. | Citronensäure |
| 2,00 | Natriumchlorid |

Herstellung:

Komponenten der Phase A einwiegen und lösen. pH-Wert auf 6 bis 7 einstellen. Phase B zugeben und mischen.

Das Beispiel lässt sich jeweils mit den erfindungsgemäßen Polymeren 1 bzw. 3, 4 und 10 bis 13 wiederholen. Es wird jeweils ein Shampoo mit guten Eigenschaften erhalten.

## Patentansprüche

**1.** Kosmetische wässrige Zubereitung enthaltend wenigstens einen kosmetisch akzeptablen Träger B) der ausgewählt ist unter

i) wassermischbaren organischen Lösungsmitteln, bevorzugt C2-C4-Alkanolen, besonders bevorzugt Ethanol,
ii) Ölen, Fetten, Wachsen,
iii) von ii) verschiedenen Estern von C6-C30-Monocarbonsäuren mit ein-, zwei- oder dreiwertigen Alkoholen,
iv) gesättigten acyclischen und cyclischen Kohlenwasserstoffen,
v) Fettsäuren,

vi) Fettalkoholen,
vii) Treibmitteln (Treibgasen) und
viii) Mischungen davon

und
wenigstens ein Polymer A, welches

a) 40-89,5 Gew.-% wenigstens eines Esters der (Meth)acrylsäure,
b) 10-49 Gew%.-% wenigstens einer olefinisch ungesättigten, anionogenen oder anionischen Verbindung,
c) 0,5-10 Gew%.-% wenigstens einer Verbindung ausgewählt aus
c1) Polyestern enthaltend wenigstens zwei radikalisch polymeri sierbare, olefinisch ungesättigte Doppelbindungen und
c2) Polyethern enthaltend wenigstens zwei radikalisch polymeri
sierbare, olefinisch ungesättigte Doppelbindungen,
d) 0-30 Gew.-% gegebenenfalls weiterer olefinisch ungesättigter Ver bindungen einpolymerisiert enthält mit der Massgabe, dass sich die Mengen der Komponenten a) bis d) zu 100 Gew%.-% addieren, wobei die Polyether c2) Polyetheracrylate sind, erhältlich durch Reaktion von

A) 1 Äquivalent eines 2-bis 6-wertigen oxalkylierten C2-bis C10-Alkohols mit
B) 0,05 bis 1 Äquivalent einer 2-bis 4-wertigen C2-bis C10-Carbonsäure oder deren Anhydride und
C) 0,1 bis 1,5 Äquivalenten Acrylsäure und/oder Methacrylsäure sowie Umsetzung der überschüssigen Carboxylgruppen mit der äquivalenten Menge einer Epoxidverbindung.

2. Kosmetische Zubereitung nach Anspruch 1 , wobei Komponente a) ausgewählt ist aus der Gruppe bestehend aus Methyl(meth)acrylat, Ethyl(meth)acrylat, n-Propyl(meth)acrylat, i-Propyl(meth)acrylat, n-Butyl(meth)acrylat, tert.-Butyl(meth)acrylat, i-Butyl(meth)acrylat, sec-Butyl(meth)acrylat, 2-Pentyl(meth)acrylat, 3-Pentyl(meth)acrylat, Isopentylacrylat, Neopentylacrylat, n-Octyl(meth)acrylat, 1,1,3,3-Tetramethylbutyl(meth)acrylat, Ethylhexyl(meth) acrylat, n-Nonyl(meth)acrylat, n-Decyl(meth)acrylat, n-Undecyl(meth)acrylat, Tridecyl(meth)acrylat, Myristyl(meth) acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, Phenoxyethyl(meth)acrylat, t-Butylcyclohexyl(meth)acrylat, Cyclohexyl(meth)acrylat, Ureido (meth)acrylat, Tetrahydrofurfuryl(meth)acrylat und deren Mischungen.

3. Kosmetische Zubereitung nach Anspruch 2, wobei Komponente a) ausgewählt ist aus der Gruppe bestehend aus tert.-Butylacrylat, Methylmethacrylat, Ethylmethacrylat, n-Propylmethacrylat, i-Propylmethacrylat, n-Butylmethacrylat, tert:-Butymethacrylat, i-Butylmethacrylat, sec-Butylmethacrylat und deren Mischungen.

4. Kosmetische Zubereitung nach einem der Ansprüche 1 bis 3, wobei Komponente b) ausgewählt ist aus monoolefinisch ungesättigten Carbonsäuren und deren Salzen.

5. Kosmetische Zubereitung nach Anspruch 4, wobei Komponente b) ausgewählt ist aus der Gruppe bestehend aus Acrylsäure, Methacrylsäure, Methacrylsäure, alpha-Chloracrylsäure, Crotonsäure, Maleinsäure, Maleinsäureanhydrid, Itaconsäure, Citraconsäure, Mesaconsäure, Glutaconsäure, Aconitsäure, Fumarsäure, Halbestern von monoethylenisch ungesättigten Dicarbonsäuren mit 4 bis 10, vorzugsweise 4 bis 6 C-Atomen.

6. Kosmetische Zubereitung nach Anspruch 5, wobei Komponente b) umfasst oder besteht aus Verbindungen ausgewählt aus der Gruppe bestehend aus Acrylsäure, Methacrylsäure und deren Mischungen.

7. Kosmetische Zubereitung nach Anspruch 5, wobei Komponente b) umfasst oder besteht aus Verbindungen ausgewählt aus der Gruppe bestehend aus Acrylsäure, Itaconsäure und deren Mischungen.

8. Kosmetische Zubereitung nach einem der Ansprüche 1 bis 7, wobei als Komponente c) Verbindungen c1) und/oder c2) oder Gemische von Verbindungen c1) und/oder c2) verwendet werden, wobei die durchschnittliche Anzahl von olefinischen, radikalisch polymerisierbaren Doppelbindungen pro Molekül mehr
als 2 beträgt.

9. Kosmetische Zubereitung nach einem der Ansprüche 1 bis 8, wobei Komponente c) ein Molekulargewicht Mw von

wenigstens 400 g/mol, bevorzugt mehr als 700 g/mol besitzt.

**10.** Kosmetische Zubereitung nach einem der Ansprüche 1 bis 9, wobei das wenigstens eine Polymer A

a) 60-80 Gew.-% der Komponente a),
b) 12-39 Gew.-% der Komponente b),
c) 1-8 Gew.-% der Komponente c) und
d) 0-30 Gew.-% der Komponente d)
einpolymerisiert enthält, mit der Massgabe, dass sich die Mengen der Komponenten a) bis d) zu 100 Gew.-% addieren.

**11.** Kosmetische Zubereitung nach einem der Ansprüche 1 bis 10 in Form eines Sprühproduktes, wobei die Zubereitung entweder in Kombination mit einer mechanischen Pumpsprühvorrichtung oder in Kombination mit mindestens einem Treibmittel ausgewählt aus der Gruppe bestehend aus Propan, Butan, Dimethylether, fluorierten Kohlenwasserstoffen und deren Mischungen vorliegt.

**12.** Polymer A wie in einem der Ansprüche 1 bis 10 definiert.

**13.** Verwendung von Polymer A nach Anspruch 12 in kosmetischen Zubereitungen

## Claims

**1.** A cosmetic aqueous preparation comprising
at least one cosmetically acceptable carrier B) which is chosen from

i) water-miscible organic solvents, preferably C2-C4-alkanols, particularly preferably ethanol,
ii) oils, fats, waxes,
iii) esters of C6-C30-monocarboxylic acids with mono-, di- or trihydric alcohols which are different from ii),
iv) saturated acyclic and cyclic hydrocarbons,
v) fatty acids,
vi) fatty alcohols,
vii) propellants (propellant gases) and
viii) mixtures thereof

and
at least one polymer A which comprises, in copolymerized form,

a) 40-89.5% by weight of at least one ester of (meth)acrylic acid,
b) 10-49% by weight of at least one olefinically unsaturated, anionogenic or anionic compound,
c) 0.5-10% by weight of at least one compound chosen from
c1) polyesters comprising at least two free-radically polymerizable, olefinically unsaturated double bonds and
c2) polyethers comprising at least two free-radically
polymerizable, olefinically unsaturated double bonds,
d) 0-30% by weight of optionally further olefinically unsaturated compounds with the proviso that the amounts of components a) to d) add up to 100% by weight,

the polyethers c2) being polyether acrylates, obtainable by reacting

A) one equivalent of a 2- to 6-hydric oxalkylated C2- to C10-alcohol with
B) 0.05 to 1 equivalent of a 2- to 4-basic C2- to C10-carboxylic acid or anhydrides thereof and
C) 0.1 to 1.5 equivalents of acrylic acid and/or methacrylic acid, and reacting the excess carboxyl groups with the equivalent amount of an epoxide compound.

**2.** The cosmetic preparation according to claim 1, where component a) is chosen from the group consisting of methyl (meth)acrylate, ethyl (meth)acrylate, n-propyl (meth)acrylate, isopropyl (meth)acrylate, n-butyl (meth)acrylate, tert-butyl (meth)acrylate, isobutyl (meth)acrylate, sec-butyl (meth)acrylate, 2-pentyl (meth)acrylate, 3-pentyl (meth)acrylate, isopentyl acrylate, neopentyl acrylate, n-octyl (meth)acrylate, 1,1,3,3-tetramethylbutyl (meth)acrylate, ethylhexyl (meth)acrylate, n-nonyl (meth)acrylate, n-decyl (meth)acrylate, n-undecyl (meth)acrylate, tridecyl (meth)acrylate, myristyl (meth)acrylate, pentadecyl (meth)acrylate, palmityl (meth)acrylate, heptadecyl (meth)acrylate, nonadecyl (meth)acrylate, arrachinyl (meth)acrylate, behenyl (meth)acrylate, lignocerenyl (meth)acrylate, cerotinyl (meth)acrylate, melissinyl (meth)acrylate, palmitoleinyl (meth)acrylate, oleyl (meth)acrylate, linolyl (meth)acrylate, linolenyl (meth)acrylate, stearyl (meth)acrylate, lauryl (meth)acrylate, phenoxyethyl (meth)acrylate, t-butylcyclohexyl (meth)acrylate, cyclohexyl (meth)acrylate, ureido (meth)acrylate, tetrahydrofurfuryl (meth)acrylate and mixtures thereof.

**3.** The cosmetic preparation according to claim 2, where component a) is chosen from the group consisting of tert-butyl acrylate, methyl methacrylate, ethyl methacrylate, n-propyl methacrylate, isopropyl methacrylate, n-butyl methacrylate, tert-butyl methacrylate, isobutyl methacrylate, sec-butyl methacrylate and mixtures thereof.

**4.** The cosmetic preparation according to one of claims 1 to 3, where component b) is chosen from monoolefinically unsaturatated carboxylic acids and salts thereof.

**5.** The cosmetic preparation according to claim 4, where component b) is chosen from the group consisting of acrylic acid, methacrylic acid, ethacrylic acid, alpha-chloroacrylic acid, crotonic acid, maleic acid, maleic anhydride, itaconic acid, citraconic acid, mesaconic acid, glutaconic acid, aconitic acid, fumaric acid, half-esters of monoethylenically unsaturated dicarboxylic acids having 4 to 10, preferably 4 to 6, carbon atoms.

**6.** The cosmetic preparation according to claim 5, where component b) comprises or consists of compounds chosen from the group consisting of acrylic acid, methacrylic acid and mixtures thereof.

**7.** The cosmetic preparation according to claim 5, where component b) comprises or consists of compounds chosen from the group consisting of acrylic acid, itaconic acid and mixtures thereof.

**8.** The cosmetic preparation according to one of claims 1 to 7, where compounds c1) and/or c2) or mixtures of compounds c1) and/or c2) are used as component c), where the average number of olefinic, free-radically polymerizable double bonds per molecule is more than 2.

**9.** The cosmetic preparation according to one of claims 1 to 8, where component c) has a molecular weight Mw of at least 400 g/mol, preferably more than 700 g/mol.

**10.** The cosmetic preparation according to one of claims 1 to 9, where the at least one polymer A comprises, in copolymerized form,

a) 60-80% by weight of component a),
b) 12-39% by weight of component b),
c) 1-8% by weight of component c) and
d) 0-30% by weight of component d)
with the proviso that the amounts of components a) to d) add up to 100% by weight.

**11.** The cosmetic preparation according to one of claims 1 to 10 in the form of a spray product, where the preparation is present either in combination with a mechanical pump spray device or in combination with at least one propellant chosen from the group consisting of propane, butane, dimethyl ether, fluorinated hydrocarbons and mixtures thereof.

**12.** A polymer A as defined in one of claims 1 to 10.

**13.** The use of polymer A according to claim 12 in cosmetic preparations.

## Revendications

**1.** Préparation cosmétique aqueuse contenant au moins un véhicule B) cosmétiquement acceptable qui est choisi parmi

i) des solvants organiques miscibles à l'eau, de préférence des alcanols en $C_2$-$C_4$, de façon particulièrement préférée l'éthanol,
ii) des huiles, des graisses, des cires,

iii) des esters d'acides monocarboxyliques en $C_6$-$C_{30}$ avec des alcools mono-, di- ou trihydriques, différents de ii),
iv) des hydrocarbures acycliques et cycliques saturés,
v) des acides gras,
vi) des alcools gras,
vii) des propulseurs (gaz propulseurs) et
viii) des mélanges de ceux-ci

et
au moins un polymère A, qui contient

a) 40-89,5 % en poids d'au moins un ester de l'acide (méth)acrylique,
b) 10-49 % en poids d'au moins un composé anionique ou anionogène à insaturation oléfinique,
c) 0,5-10 % en poids d'au moins un composé choisi parmi
c1) des polyesters comportant au moins deux doubles liaisons à insaturation oléfinique, aptes à la polymérisation radicalaire et
c2) des polyéthers comportant au moins deux doubles liaisons à insaturation oléfinique, aptes à la polymérisation radicalaire,
d) 0-30 % en poids éventuellement d'autres composés à insaturation oléfinique, incorporés par polymérisation,

étant entendu que la somme des quantités des composants a) à d) est égale à 100 % en poids,
les polyéthers c2) étant des polyétheracrylates pouvant être obtenus par réaction de

A) 1 équivalent d'un alcool en $C_2$-$C_{10}$ oxyalkylé di- à hexahydrique avec
B) 0,05 à 1 équivalent d'un acide di- à tetracarboxylique en $C_2$-$C_{10}$ ou de son anhydride et
C) 0,1 à 1,5 équivalent d'acide acrylique et/ou méthacrylique

ainsi que réaction des groupes carboxy en excès avec la quantité équivalente d'un composé époxyde.

**2.** Préparation cosmétique selon la revendication 1, dans laquelle le composant a) est choisi dans le groupe constitué par le (méth)acrylate de méthyle, le (méth)acrylate d'éthyle, le (méth)acrylate de n-propyle, le (méth)acrylate d'isopropyle, le (méth)acrylate de n-butyle, le (méth)acrylate de tert-butyle, le (méth)acrylate d'isobutyle, le (méth)acrylate de sec-butyle, le (méth)acrylate de 2-pentyle, le (méth)acrylate de 3-pentyle, l'acrylate d'isopentyle, l'acrylate de néopentyle, le (méth)acrylate de n-octyle, le (méth)acrylate de 1,1,3,3-tétraméthylbutyle, le (méth)acrylate d'éthylhexyle, le (méth)acrylate de n-nonyle, le (méth)acrylate de n-décyle, le (méth)acrylate de n-undécyle, le (méth) acrylate de tridécyle, le (méth)acrylate de myristyle, le (méth)acrylate de pentadécyle, le (méth)acrylate de palmityle, le (méth)acrylate d'heptadécyle, le (méth)acrylate de nonadécyle, le (méth)acrylate d'arrachinyle, le (méth)acrylate de béhényle, le (méth)acrylate de lignocérényle, le (méth)acrylate de cérotinyle, le (méth)acrylate de mélissinyle, le (méth)acrylate de palmitoléyle, le (méth)acrylate d'oléyle, le (méth)acrylate de linolyle, le (méth)acrylate de linolényle, le (méth)acrylate de stéaryle, le (méth)acrylate de lauryle, le (méth)acrylate de phénoxyéthyle, le (méth) acrylate de tert-butylcyclohexyle, le (méth)acrylate de cyclohexyle, l'uréido(méth)acrylate, le (méth)aCrylate de tétrahydrofurfuryle et des mélanges de ceux-ci.

**3.** Préparation cosmétique selon la revendication 2, dans laquelle le composant a) est choisi dans le groupe constitué par l'acrylate de tert-butyle, le méthacrylate de méthyle, le méthacrylate d'éthyle, le méthacrylate de n-propyle, le méthacrylate d'isopropyle, le méthacrylate de n-butyle, le méthacrylate de tert-butyle, le méthacrylate d'isobutyle, le méthacrylate de sec-butyle et des mélanges de ceux-ci.

**4.** Préparation cosmétique selon l'une quelconque des revendications 1 à 3, dans laquelle le composant b) est choisi parmi des acides carboxyliques à insaturation mono-oléfinique et leurs sels.

**5.** Préparation cosmétique selon la revendication 4, dans laquelle le composant b) est choisi dans le groupe constitué par l'acide acrylique, l'acide méthacrylique, l'acide méthacrylique, l'acide alpha-chloracrylique, l'acide crotonique, l'acide maléique, l'anhydride maléique, l'acide itaconique, l'acide citraconique, l'acide mésaconique, l'acide glutaconique, l'acide aconitique, l'acide fumarique, les hémiesters d'acides dicarboxyliques à insaturation monoéthylénique ayant de 4 à 10, de préférence de 4 à 6 atomes de carbone.

**6.** Préparation cosmétique selon la revendication 5, dans laquelle le composant b) comprend ou consiste en des

composés choisis dans le groupe constitué par l'acide acrylique, l'acide méthacrylique et des mélanges de ceux-ci.

**7.** Préparation cosmétique selon la revendication 5, dans laquelle le composant b) comprend ou consiste en des composés choisis dans le groupe constitué par l'acide acrylique, l'acide itaconique et des mélanges de ceux-ci.

**8.** Préparation cosmétique selon l'une quelconque des revendications 1 à 7, dans laquelle on utilise en tant que composant c) des composés c1) et/ou c2) ou des mélanges de composés c1) et/ou c2), dans lesquels le nombre moyen des doubles liaisons oléfiniques, aptes à la polymérisation radicalaire, par molécule, est supérieur à 2.

**9.** Préparation cosmétique selon l'une quelconque des revendications 1 à 8, dans laquelle le composant c) a une masse moléculaire Mw d'au moins 400 g/mole, de préférence de plus de 700 g/mole.

**10.** Préparation cosmétique selon l'une quelconque des revendications 1 à 9, dans laquelle ledit au moins un polymère A contient incorporés par polymérisation

a) 60-80 % en poids du composant a) ;
b) 12-39 % du composant b),
c) 1-8 % en poids du composant c) et
d) 0-30 % en poids du composant d), étant entendu que la somme des quantités des composants a) à d) est égale à 100 % en poids.

**11.** Préparation cosmétique selon l'une quelconque des revendications 1 à 10, sous forme d'un produit à pulvériser, la préparation se trouvant soit en association avec un dispositif de pulvérisation à pompe soit en association avec au moins un propulseur choisi dans le groupe constitué par le propane, le butane, l'éther diméthylique, des hydrocarbures fluorés et des mélanges de ceux-ci.

**12.** Polymère A tel que défini dans l'une quelconque des revendications 1 à 10.

**13.** Utilisation du polymère A selon la revendication 12 dans des préparations cosmétiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03062288 A
- WO 03061615 A
- EP 0184785 A
- WO 9505402 A
- DE 2330957
- US 3940351 A
- EP 0279303 A
- DE 2853921
- EP 0686621 A
- WO 9732917 A
- EP 1035144 A
- DE 4333238 A
- DE 3929973
- DE 2150557
- DE 2817369
- DE 3708451
- DE 3314742 A
- EP 1084696 A
- DE PS1165574 C
- DE PS2024051 C
- EP 934956 A


### In der Beschreibung aufgeführte Nicht-Patentliteratur


- **Houben-Weyl.** *Makromolekulare Stoffe II,* 1963, vol. XIV, 2
- Macromolecules. Plenum Press, 1984, vol. 2
- **S. P. Pappas.** *J. Rad. Cur.,* Juli 1987, 6
- **Fikentscher.** *Cellulosechemie,* 1932, vol. 13, 58-64
- Kosmetik und Hygiene von Kopf bis Fuß. Wiley-VCH, 2004, 235-236
- International Cosmetic Ingredient Dictionary and Handbook. The Cosmetic, Toiletry, and Fragrance Association, 2002, vol. 4
- Grundlagen und Rezepturen der Kosmetika. Verlag Hüthig, 319-355